(19) Europäisches Patentamt
European Patent Office
Office européen des brevets

(11) EP 3 039 135 B1

(12) EUROPÄISCHE PATENTSCHRIFT

(45) Veröffentlichungstag und Bekanntmachung des Hinweises auf die Patenterteilung:
**04.04.2018 Patentblatt 2018/14**

(21) Anmeldenummer: **14783532.6**

(22) Anmeldetag: **27.08.2014**

(51) Int Cl.:
***C12N 9/14*** *(2006.01)*

(86) Internationale Anmeldenummer:
**PCT/AT2014/000165**

(87) Internationale Veröffentlichungsnummer:
**WO 2015/027259 (05.03.2015 Gazette 2015/09)**

(54) **POLYPEPTID ZUR HYDROLYTISCHEN SPALTUNG VON ZEARALENON UND/ODER ZEARALENON-DERIVATEN, ISOLIERTES POLYNUKLEOTID DAVON SOWIE EINEN ZUSATZSTOFF ENTHALTENDES POLYPEPTID, VERWENDUNG DESSELBEN SOWIE VERFAHREN**

POLYPEPTIDE FOR THE HYDROLYTIC CLEAVAGE OF ZEARALENONE AND/OR ZEARALENONE DERIVATIVES, ISOLATED POLYNUCLEOTIDE THEREOF, AND ADDITIVE CONTAINING POLYPEPTIDE, USE OF SAID POLYPEPTIDE AND METHOD

POLYPEPTIDE POUR LA DÉCOMPOSITION HYDROLYTIQUE DU ZÉARALÉNONE OU DE DÉRIVÉS DU ZÉARALENONE, POLYNUCLÉOTIDE ISOLÉ DE CELUI-CI, AINSI QU’ADDITIF CONTENANT CE POLYPEPTIDE, UTILISATION DE CELUI-CI, ET PROCÉDÉ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **28.08.2013 AT 6672013**

(43) Veröffentlichungstag der Anmeldung:
**06.07.2016 Patentblatt 2016/27**

(73) Patentinhaber: **Erber Aktiengesellschaft**
**3131 Getzersdorf bei Traismauer (AT)**

(72) Erfinder:
- **FRUHAUF, Sebastian**
**A-3040 Neulengbach (AT)**
- **THAMHESL, Michaela**
**A-1180 Wien (AT)**
- **PFEFFER, Martin**
**A-3430 Tulln (AT)**
- **MOLL, Dieter**
**A-2000 Stockerau (AT)**
- **SCHATZMAYR, Gerd**
**A-3430 Tulln (AT)**
- **BINDER, Eva Maria**
**A-3430 Tulln (AT)**

(74) Vertreter: **Cunow, Gerda**
**Cunow Patentanwalts KG**
**Teschnergasse 33/1/3**
**1180 Wien (AT)**

(56) Entgegenhaltungen:
**WO-A1-03/053161 WO-A1-2012/113827**

- **DATABASE UniProt [Online] 16. April 2014 (2014-04-16), "SubName: Full=Hydrolase {ECO:0000313|EMBL:AHH94730.1};", XP002732829, gefunden im EBI accession no. UNIPROT:W5W1S2 Database accession no. W5W1S2**
- **I. Rodrigues ET AL: "Microorganisms and their enzymes for detoxifying mycotoxins posing a risk to livestock animals" In: "Cellulose Solvents: For Analysis, Shaping and Chemical Modification", 20. Dezember 2009 (2009-12-20), American Chemical Society, Washington, DC, XP055103712, ISSN: 0097-6156 ISBN: 978-0-84-120007-4 Bd. 1031, Seiten 107-117, DOI: 10.1021/bk-2009-1031.ch008, das ganze Dokument**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- **DATABASE UniProt [Online] 16. November 2011 (2011-11-16), "SubName: Full=Alpha/beta hydrolase fold containing protein {ECO:0000313|EMBL:AEM80235.1};", XP002732830, gefunden im EBI accession no. UNIPROT:G2P9U4 Database accession no. G2P9U4**
- **TAKAHASHI-ANDO N ET AL: "A novel lactonohydrolase responsible for the detoxification of zearalenone: enzyme purification and gene cloning", BIOCHEMICAL JOURNAL, PORTLAND PRESS LTD, GB, Bd. 365, 1. Juli 2002 (2002-07-01), Seiten 1-6, XP002967677, ISSN: 0264-6021, DOI: 10.1042/BJ20020450**
- **YUANSHAN YU ET AL: "Oxidation of zearalenone by extracellular enzymes fromSM04 into smaller estrogenic products", WORLD JOURNAL OF MICROBIOLOGY AND BIOTECHNOLOGY, KLUWER ACADEMIC PUBLISHERS, DO, Bd. 27, Nr. 11, 26. April 2011 (2011-04-26), Seiten 2675-2681, XP019959940, ISSN: 1573-0972, DOI: 10.1007/S11274-011-0741-3**

SEQUENCE LISTING

<110> Erber Aktiengesellschaft

<120> Polypeptid zur hydrolytischen Spaltung von zearalenon und / oder zearalenon Derivaten

<130> P05342PCT

<160> 67

<170> PatentIn version 3.5

<210> 1
<211> 328
<212> PRT
<213> Rhodococcus erythropolis

<400> 1

```
Met Ala Glu Glu Gly Thr Arg Ser Glu Ala Ala Asp Ala Ala Thr Gln
1               5                   10                  15

Ala Arg Gln Leu Pro Asp Ser Arg Asn Ile Phe Val Ser His Arg Phe
            20                  25                  30

Pro Glu Arg Gln Val Asp Leu Gly Glu Val Val Met Asn Phe Ala Glu
        35                  40                  45

Ala Gly Ser Pro Asp Asn Pro Ala Leu Leu Leu Leu Pro Glu Gln Thr
    50                  55                  60

Gly Ser Trp Trp Ser Tyr Glu Pro Val Met Gly Leu Leu Ala Glu Asn
65                  70                  75                  80

Phe His Val Phe Ala Val Asp Ile Arg Gly Gln Gly Arg Ser Thr Trp
                85                  90                  95

Thr Pro Arg Arg Tyr Ser Leu Asp Asn Phe Gly Asn Asp Leu Val Arg
            100                 105                 110

Phe Ile Ala Leu Val Ile Lys Arg Pro Val Val Val Ala Gly Asn Ser
        115                 120                 125

Ser Gly Gly Leu Leu Ala Ala Trp Leu Ser Ala Tyr Ala Met Pro Gly
    130                 135                 140

Gln Ile Arg Ala Ala Leu Cys Glu Asp Ala Pro Phe Phe Ala Ser Glu
145                 150                 155                 160

Leu Val Pro Ala Tyr Gly His Ser Val Leu Gln Ala Ala Gly Pro Ala
                165                 170                 175

Phe Glu Leu Tyr Arg Asp Phe Leu Gly Asp Gln Trp Ser Ile Gly Asp
            180                 185                 190
```

```
Trp Lys Gly Phe Val Glu Ala Ala Lys Ala Ser Pro Ala Lys Ala Met
        195                 200                 205

Gln Leu Phe Pro Thr Pro Asp Glu Ala Pro Gln Asn Leu Lys Glu Tyr
    210                 215                 220

Asp Pro Glu Trp Gly Arg Ala Phe Phe Glu Gly Thr Val Ala Leu His
225                 230                 235                 240

Cys Pro His Asp Arg Met Leu Ser Gln Val Lys Thr Pro Ile Leu Ile
                245                 250                 255

Thr His His Ala Arg Thr Ile Asp Pro Glu Thr Gly Glu Leu Leu Gly
            260                 265                 270

Ala Leu Ser Asp Leu Gln Ala Glu His Ala Gln Asp Ile Ile Arg Ser
        275                 280                 285

Ala Gly Val Arg Val Asp Tyr Gln Ser His Pro Asp Ala Leu His Met
    290                 295                 300

Met His Leu Phe Asp Pro Ala Arg Tyr Ala Glu Ile Leu Thr Ser Trp
305                 310                 315                 320

Ser Ala Thr Leu Pro Ala Asn Asp
                325
```

<210> 2
<211> 308
<212> PRT
<213> Streptomyces violaceusniger

<400> 2

```
Met Ala Asp Pro Ala Gln Arg Asp Val Tyr Val Pro His Ala Tyr Pro
1               5                   10                  15

Glu Lys Gln Ala Asp Leu Gly Glu Ile Thr Met Asn Tyr Ala Glu Ala
            20                  25                  30

Gly Glu Pro Asp Met Pro Ala Val Leu Leu Ile Pro Glu Gln Thr Gly
        35                  40                  45

Ser Trp Trp Gly Tyr Glu Glu Ala Met Gly Leu Leu Ala Glu Asn Phe
    50                  55                  60

His Val Tyr Ala Val Asp Leu Arg Gly Gln Gly Arg Ser Ser Trp Ala
65                  70                  75                  80

Pro Lys Arg Tyr Ser Leu Asp Asn Phe Gly Asn Asp Leu Val Arg Phe
                85                  90                  95

Ile Ala Leu Val Val Lys Arg Pro Val Ile Val Ala Gly Asn Ser Ser
```

100 105 110

Gly Gly Val Leu Ala Ala Trp Leu Ser Ala Tyr Ser Met Pro Gly Gln
115 120 125

Val Arg Gly Ala Leu Cys Glu Asp Ala Pro Phe Phe Ala Ser Glu Leu
130 135 140

Val Thr Thr Cys Gly His Ser Ile Arg Gln Ala Ala Gly Pro Met Phe
145 150 155 160

Glu Leu Phe Arg Thr Tyr Leu Gly Asp Gln Trp Ser Val Gly Asp Trp
165 170 175

Thr Gly Tyr Cys Arg Ala Ala Asp Ala Ser Ser Ser Pro Met Ala Arg
180 185 190

Tyr Phe Val Ala Asp Glu Ile Pro Gln His Met Arg Glu Tyr Asp Pro
195 200 205

Glu Trp Ala Arg Ala Phe Trp Glu Gly Thr Val Ala Leu His Cys Pro
210 215 220

His Glu Gln Leu Leu Thr Gln Val Lys Thr Pro Val Leu Leu Thr His
225 230 235 240

His Met Arg Asp Ile Asp Pro Asp Thr Gly His Leu Val Gly Ala Leu
245 250 255

Ser Asp Glu Gln Ala Ala Arg Ala Arg Leu Leu Met Glu Ser Ala Gly
260 265 270

Val Lys Val Asp Tyr Ala Ser Val Pro Asp Ala Leu His Met Met His
275 280 285

Gln Phe Asp Pro Pro Arg Tyr Val Glu Ile Phe Thr Gln Trp Ala Ala
290 295 300

Thr Leu Ala Ala
305

<210> 3
<211> 309
<212> PRT
<213> Streptomyces coelicolor

<400> 3

Met Val Thr Ser Pro Ala Leu Arg Asp Val His Val Pro His Ala Tyr
1               5                   10                  15

Pro Glu Gln Gln Val Asp Leu Gly Glu Ile Thr Met Asn Tyr Ala Glu
            20                  25                  30

```
Ala Gly Asp Pro Gly Arg Pro Ala Val Leu Leu Ile Pro Glu Gln Thr
        35                  40                  45

Gly Ser Trp Trp Ser Tyr Glu Glu Ala Met Gly Leu Leu Ala Glu His
    50                  55                  60

Phe His Val Tyr Ala Val Asp Leu Arg Gly Gln Gly Arg Ser Ser Trp
65                  70                  75                  80

Thr Pro Lys Arg Tyr Ser Leu Asp Asn Phe Gly Asn Asp Leu Val Arg
                85                  90                  95

Phe Ile Ala Leu Val Val Arg Arg Pro Val Val Val Ala Gly Asn Ser
            100                 105                 110

Ser Gly Gly Val Leu Ala Ala Trp Leu Ser Ala Tyr Ser Met Pro Gly
        115                 120                 125

Gln Ile Arg Gly Val Leu Cys Glu Asp Pro Pro Phe Phe Ala Ser Glu
    130                 135                 140

Leu Val Pro Ala His Gly His Ser Val Arg Gln Gly Ala Gly Pro Val
145                 150                 155                 160

Phe Glu Leu Phe Arg Thr Tyr Leu Gly Asp Gln Trp Ser Val Gly Asp
                165                 170                 175

Trp Glu Gly Phe Arg Ser Ala Ala Asp Ala Ser Ala Ser Pro Met Ala
            180                 185                 190

Arg Ser Phe Val Ala Asp Thr Ile Pro Gln His Leu Lys Glu Tyr Asp
        195                 200                 205

Pro Glu Trp Ala Arg Ala Phe Tyr Glu Gly Thr Val Gly Leu Asn Cys
    210                 215                 220

Pro His Glu Arg Met Leu Asn Arg Val Asn Thr Pro Val Leu Leu Thr
225                 230                 235                 240

His His Met Arg Gly Thr Asp Pro Glu Thr Gly Asn Leu Leu Gly Ala
                245                 250                 255

Leu Ser Asp Glu Gln Ala Ala Gln Val Arg Arg Leu Met Glu Ser Ala
            260                 265                 270

Gly Val Lys Val Asp Tyr Glu Ser Val Pro Asp Ala Ser His Met Met
        275                 280                 285

His Gln Ser Asp Pro Ala Arg Tyr Ala Glu Ile Leu Thr Pro Trp Thr
    290                 295                 300
```

Ala Ala Leu Ala Pro
305

<210> 4
<211> 309
<212> PRT
<213> Streptomyces rapamycinicus

<400> 4

```
Met Val Thr Ser Pro Ala Leu Arg Asp Val His Val Pro His Ala Tyr
1               5                   10                  15

Pro Glu Gln Gln Val Asp Leu Gly Glu Ile Thr Met Asn Tyr Ala Glu
            20                  25                  30

Ala Gly Asp Pro Asp Arg Pro Ala Val Leu Leu Ile Pro Glu Gln Thr
        35                  40                  45

Gly Ser Trp Trp Ser Tyr Glu Glu Ala Met Gly Leu Leu Ala Glu His
    50                  55                  60

Phe His Val Tyr Ala Val Asp Leu Arg Gly Gln Gly Arg Ser Ser Trp
65                  70                  75                  80

Thr Pro Lys Arg Tyr Ser Leu Asp Asn Phe Gly Asn Asp Leu Val Arg
                85                  90                  95

Phe Ile Ala Leu Val Val Lys Arg Pro Val Val Val Ala Gly Asn Ser
            100                 105                 110

Ser Gly Gly Val Leu Ala Ala Trp Leu Ser Ala Tyr Ser Met Pro Gly
        115                 120                 125

Gln Leu Arg Gly Val Leu Cys Glu Asp Pro Pro Phe Phe Ala Ser Glu
    130                 135                 140

Leu Val Pro Ala His Gly His Ser Val Arg Gln Gly Ala Gly Pro Val
145                 150                 155                 160

Phe Glu Leu Phe Arg Thr Tyr Leu Gly Asp Gln Trp Ser Val Ser Asp
                165                 170                 175

Trp Glu Gly Phe Cys Arg Ala Ala Gly Ala Ser Ala Ser Pro Met Ala
            180                 185                 190

Arg Ser Phe Val Ala Asp Gly Ile Pro Gln His Leu Lys Glu Tyr Asp
        195                 200                 205

Pro Glu Trp Ala Arg Ala Phe His Glu Gly Thr Val Gly Leu Asn Cys
    210                 215                 220
```

Pro His Glu Arg Met Leu Gly Arg Val Asn Thr Pro Val Leu Leu Thr
225 230 235 240

His His Met Arg Gly Thr Asp Pro Glu Thr Gly Asn Leu Leu Gly Ala
245 250 255

Leu Ser Asp Glu Gln Ala Ala Gln Ala Arg Leu Leu Met Glu Ser Ala
260 265 270

Gly Val Arg Val Asp Tyr Glu Ser Val Pro Asp Ala Ser His Met Met
275 280 285

His Gln Ser Asp Pro Ala Arg Tyr Ala Glu Ile Phe Thr Arg Trp Ala
290 295 300

Ala Ala Leu Ala Pro
305

<210> 5
<211> 309
<212> PRT
<213> Streptomyces lividans

<400> 5

Met Val Thr Ser Pro Ala Leu Arg Asp Val His Val Pro His Ala Tyr
1 5 10 15

Pro Glu Gln Gln Val Asp Leu Gly Glu Ile Thr Met Asn Tyr Ala Glu
20 25 30

Ala Gly Asp Pro Gly Arg Pro Ala Val Leu Leu Ile Pro Glu Gln Thr
35 40 45

Gly Ser Trp Trp Ser Tyr Glu Glu Ala Met Gly Leu Leu Ala Glu His
50 55 60

Phe His Val Tyr Ala Val Asp Leu Arg Gly Gln Gly Arg Ser Ser Trp
65 70 75 80

Thr Pro Lys Arg Tyr Ser Leu Asp Asn Phe Gly Asn Asp Leu Val Arg
85 90 95

Phe Met Ala Leu Val Val Arg Arg Pro Val Val Val Ala Gly Asn Ser
100 105 110

Ser Gly Gly Val Leu Ala Ala Trp Leu Ser Ala Tyr Ser Met Pro Gly
115 120 125

Gln Ile Arg Gly Val Leu Cys Glu Asp Pro Pro Phe Phe Ala Ser Glu
130 135 140

```
Leu Val Pro Ala His Gly His Ser Val Arg Gln Gly Ala Gly Pro Val
145                 150                 155                 160

Phe Glu Leu Phe Arg Thr Tyr Leu Gly Asp Gln Trp Ser Val Gly Asp
                165                 170                 175

Trp Glu Gly Phe Arg Ser Ala Ala Gly Ala Ser Ala Ser Pro Met Ala
            180                 185                 190

Arg Ser Phe Val Ala Asp Thr Ile Pro Gln His Leu Lys Glu Tyr Asp
        195                 200                 205

Pro Glu Trp Ala Arg Ala Phe Tyr Glu Gly Thr Val Gly Leu Asn Cys
    210                 215                 220

Pro His Glu Arg Met Leu Asn Arg Val Asn Thr Pro Val Leu Leu Thr
225                 230                 235                 240

His His Met Arg Gly Thr Asp Pro Glu Thr Gly Asn Leu Leu Gly Ala
                245                 250                 255

Leu Ser Asp Glu Gln Ala Ala Gln Ala Arg Arg Leu Met Glu Ser Ala
            260                 265                 270

Gly Val Lys Val Asp Tyr Glu Ser Val Pro Asp Ala Ser His Met Met
        275                 280                 285

His Gln Ser Asp Pro Ala Arg Tyr Ala Glu Ile Leu Thr Pro Trp Ala
    290                 295                 300

Ala Ala Leu Ala Pro
305
```

<210> 6
<211> 309
<212> PRT
<213> streptomyces coelicoflavus

<400> 6

```
Met Val Thr Ser Pro Ala Leu Arg Asp Val His Val Pro His Ala Tyr
1               5                   10                  15

Pro Glu Gln Gln Val Asp Leu Gly Glu Ile Thr Met Asn Tyr Ala Glu
            20                  25                  30

Ala Gly Asp Pro Asp Arg Pro Ala Val Leu Leu Ile Pro Glu Gln Thr
        35                  40                  45

Gly Ser Trp Trp Ser Tyr Glu Glu Ala Met Gly Leu Leu Ser Glu His
    50                  55                  60

Phe His Val Tyr Ala Val Asp Leu Arg Gly Gln Gly Arg Ser Ser Trp
```

65 70 75 80

Thr Pro Lys Arg Tyr Ser Leu Asp Asn Phe Gly Asn Asp Leu Val Arg
85 90 95

Phe Ile Ala Leu Val Val Lys Arg Pro Val Val Val Ala Gly Asn Ser
100 105 110

Ser Gly Gly Val Leu Ala Ala Trp Leu Ser Ala Tyr Ser Met Pro Gly
115 120 125

Gln Leu Arg Gly Val Leu Cys Glu Asp Pro Pro Phe Phe Ala Ser Glu
130 135 140

Leu Val Pro Ala His Gly His Ser Val Arg Gln Gly Ala Gly Pro Val
145 150 155 160

Phe Glu Leu Phe Arg Thr Tyr Leu Gly Asp Gln Trp Ser Val Gly Asp
165 170 175

Trp Glu Gly Phe Cys Arg Ala Ala Gly Ala Ser Ala Ser Pro Met Ala
180 185 190

Arg Ser Phe Val Ala Asp Gly Ile Pro Gln His Leu Gln Glu Tyr Asp
195 200 205

Pro Glu Trp Ala Arg Val Phe Tyr Glu Gly Thr Val Gly Leu Ser Cys
210 215 220

Pro His Glu Arg Met Leu Gly Gln Val Lys Thr Pro Val Leu Leu Thr
225 230 235 240

His His Met Arg Gly Ile Asp Pro Glu Thr Gly Asn Leu Leu Gly Ala
245 250 255

Leu Ser Asp Glu Gln Ala Leu Arg Ala Arg Arg Leu Met Asp Ser Ala
260 265 270

Gly Val Thr Val Asp Tyr Glu Ser Val Pro Asp Ala Ser His Met Met
275 280 285

His Gln Ser Ala Pro Ala Arg Tyr Val Glu Ile Phe Thr Arg Trp Ala
290 295 300

Ala Ala Leu Ala Pro
305

<210> 7
<211> 300
<212> PRT
<213> Rhodococcus triatome

<400> 7

Met Pro His Asp Tyr Glu Glu Lys Leu Val Asp Leu Gly Glu Ile Asp
1               5                   10                  15

Leu Asn Tyr Ala Glu Ala Gly Ser Pro Asp Lys Pro Ala Leu Leu Leu
            20                  25                  30

Ile Pro Ser Gln Ser Glu Ser Trp Trp Gly Tyr Glu Glu Ala Met Gly
        35                  40                  45

Leu Leu Ala Glu Asp Tyr His Val Phe Ala Val Asp Met Arg Gly Gln
    50                  55                  60

Gly Arg Ser Thr Trp Thr Pro Gly Arg Tyr Ser Leu Asp Asn Phe Gly
65                  70                  75                  80

Asn Asp Leu Val Arg Phe Ile Asp Leu Val Ile Gly Arg Thr Val Ile
                85                  90                  95

Val Ser Gly Asn Ser Ser Gly Gly Val Val Ala Ala Trp Leu Ala Ala
            100                 105                 110

Phe Ser Leu Pro Gly Gln Val Arg Ala Ala Leu Ala Glu Asp Ala Pro
        115                 120                 125

Phe Phe Ala Ser Glu Leu Asp Pro Lys Val Gly His Thr Ile Arg Gln
    130                 135                 140

Ala Ala Gly His Ile Phe Val Asn Trp Arg Asp Tyr Leu Gly Asp Gln
145                 150                 155                 160

Trp Ser Val Gly Asp Tyr Ala Gly Phe Leu Lys Ala Met Lys Ser Ser
                165                 170                 175

Glu Val Pro Met Leu Arg Gln Val Pro Leu Pro Glu Thr Ala Pro Gln
            180                 185                 190

Asn Leu Leu Glu Tyr Asp Pro Glu Trp Ala Arg Ala Phe Tyr Glu Gly
        195                 200                 205

Thr Val Ala Gln Thr Cys Pro His Asp Tyr Met Leu Ser Gln Val Lys
    210                 215                 220

Val Pro Met Leu Val Thr His His Ala Arg Met Ile Asp Glu Ala Thr
225                 230                 235                 240

Ser Gly Leu Val Gly Ala Met Ser Asp Leu Gln Val Gln Lys Ala Ala
                245                 250                 255

Glu Ile Ile Arg Gly Thr Gly Val Gln Val Asp Val Val Asp Leu Pro
            260                 265                 270

Glu Ala Pro His Ile Leu His Gln Leu Ala Pro Lys Glu Tyr Val Glu
275 280 285

Ile Leu Asn Asn Trp Val Glu Lys Leu Pro Pro Val
290 295 300

<210> 8
<211> 307
<212> PRT
<213> Hirschia baltica

<400> 8

Met Ile Gln Asn Asn Lys Thr Ala Pro Tyr Lys Tyr Lys Glu Lys Leu
1 5 10 15

Val Asp Leu Gly Glu Ile Lys Met Asn Tyr Ile Val Ala Gly Ala Asp
20 25 30

Val Ser Pro Ala Leu Leu Leu Ile Pro Gly Gln Thr Glu Ser Trp Trp
35 40 45

Gly Phe Glu Ala Ala Ile Glu Lys Leu Glu Ser Asn Phe Gln Val Phe
50 55 60

Ala Ile Asp Leu Arg Gly Gln Gly Lys Ser Thr Gln Thr Pro Gly Arg
65 70 75 80

Tyr Ser Leu Asn Leu Met Gly Asn Asp Leu Val Arg Phe Ile Ser Leu
85 90 95

Val Ile Lys Arg Pro Val Ile Val Ser Gly Asn Ser Ser Gly Gly Leu
100 105 110

Leu Ala Ala Trp Leu Ser Ala Tyr Ala Met Pro Asn Gln Ile Arg Ala
115 120 125

Ile His Cys Glu Asp Ala Pro Phe Phe Thr Ala Glu Lys Ala Pro Leu
130 135 140

Tyr Gly His Ala Ile Gln Gln Ala Ala Gly Pro Ile Phe Ser Leu Met
145 150 155 160

Ser Lys Phe Leu Gly Asp Gln Trp Ser Ile Asn Asn Trp Glu Gly Leu
165 170 175

Lys Ala Ala Gln Ala Lys Asp Thr His Pro Ala Asn Lys Met Ile Ser
180 185 190

Gln Val Glu Gln Pro Pro Gln His Leu Lys Glu Tyr Asp Pro Glu Trp
195 200 205

```
Gly Arg Ala Phe Ile Glu Gly Lys Phe Asn Leu Asn Ser Pro His His
    210                 215                 220

Thr Leu Leu Ser Asp Ile Lys Thr Pro Met Leu Tyr Thr His His Met
225                 230                 235                 240

Arg Phe Glu Asp Pro Gln Thr Gly Leu Leu Ile Gly Ala Thr Ser Asp
                245                 250                 255

Phe Gln Ala Ser Lys Ile Lys Glu Ile Ala Leu Lys Thr Gly Asn Ser
            260                 265                 270

Phe Glu Leu Ile Asp Ala Pro Asp Ala Phe His Ser Met His Glu Ala
        275                 280                 285

Asp Pro Gln Arg Phe Val Asp Ile Leu Thr Ser Trp Ile Glu Arg Leu
    290                 295                 300

Asn Leu Gln
305
```

<210> 9
<211> 321
<212> PRT
<213> Nocardia brasiliensis

<400> 9

```
Met Gly Ile Ser Glu Ala Ala Asp Arg Ala Asp Thr Phe Val Ala His
1               5                   10                  15

Lys Phe Glu Glu Gln Leu Val Asp Leu Gly Glu Ile Arg Met Asn Tyr
            20                  25                  30

Val Ala Ala Gly Asp Pro Thr Ser Pro Ala Leu Leu Leu Ile Pro Ala
        35                  40                  45

Gln Gly Glu Ser Trp Trp Gly Tyr Glu Asn Ala Ile Thr Leu Leu Ala
    50                  55                  60

Asn Asp Phe Arg Val Phe Ala Ile Asp Leu Arg Gly Gln Gly Arg Ser
65                  70                  75                  80

Thr Trp Thr Pro Gly Arg Tyr Asn Leu Asn Thr Trp Gly Asn Asp Val
                85                  90                  95

Glu Arg Phe Ile Asp Leu Val Ile Gly Arg Pro Thr Leu Val Ala Gly
            100                 105                 110

Asn Ser Ser Gly Gly Val Ile Ala Ala Trp Leu Ala Ala Tyr Ala Lys
        115                 120                 125
```

Pro Gly Gln Ile Arg Gly Ala Met Leu Glu Asp Pro Pro Leu Phe Ala
130 135 140

Ser Gln Ala Ala Pro Pro Tyr Gly Pro Gly Ile Met Gln Thr Leu Gly
145 150 155 160

Pro Ile Phe Val Leu Trp Ala Lys Trp Leu Gly Pro Gln Trp Ser Val
165 170 175

Gly Asp Trp Asp Gly Met Val Ala Ala Ala Pro Arg Glu Leu Pro Glu
180 185 190

Phe Leu His Pro Gly Ile Ala Phe Leu Phe Gly Asp Gly Thr Gly Glu
195 200 205

Gly Ala Ala Ala Thr Pro Pro Gln His Leu Lys Glu Tyr Asp Pro Glu
210 215 220

Trp Ala Gln Ala Trp Ala Thr Asp Val Ala Asn Ala Gly Cys Asp His
225 230 235 240

Ala Thr Met Leu Ala Gln Asn Arg Val Pro Val Leu Leu Thr His His
245 250 255

Phe His Leu Thr Asp Pro Asp Thr Gly Gln Leu Met Gly Ala Met Thr
260 265 270

Asp Ile Gln Ala Gln Gln Ala Arg Arg Leu Leu Ala Ala Thr Gly Gln
275 280 285

Pro Val Thr Phe Thr Ala Leu Asp Ala Pro His Thr Met His Asp Pro
290 295 300

Glu Pro Glu Arg Tyr Phe Glu Val Leu Thr Glu Trp Ala Ser Ala Leu
305 310 315 320

Asp

<210> 10
<211> 319
<212> PRT
<213> Mycobacterium vaccae

<400> 10

Met Gly Arg Tyr Ala Gly Val Phe Gly Pro His Ala Pro Glu Ser Thr
1               5                   10                  15

Tyr Val Gly His Ala Tyr Pro Glu Gln Leu Phe Asp Thr Gly Glu Val
            20                  25                  30

Arg Leu Asn Tyr Ala Val Ala Gly Asp Ala Ser Ala Ser Pro Leu Leu

35 40 45

Leu Ile Pro Gly Gln Thr Glu Ser Trp Trp Gly Tyr Glu Pro Ala Met
50 55 60

Gly Leu Leu Ala Glu His Phe His Val His Ala Val Asp Leu Arg Gly
65 70 75 80

Gln Gly Arg Ser Thr Arg Thr Pro Arg Arg Tyr Thr Leu Asp Asn Ile
85 90 95

Gly Asn Asp Leu Val Arg Phe Leu Asp Gly Val Ile Gly Arg Pro Ala
100 105 110

Phe Val Ser Gly Leu Ser Ser Gly Gly Leu Leu Ser Ala Trp Leu Ser
115 120 125

Ala Phe Ala Glu Pro Gly Gln Val Leu Ala Ala Cys Tyr Glu Asp Pro
130 135 140

Pro Phe Phe Ser Ser Glu Leu Asp Pro Val Ile Gly Pro Gly Leu Met
145 150 155 160

Ser Thr Val Gly Pro Leu Phe Ala Leu Tyr Val Lys Tyr Leu Gly Asp
165 170 175

Gln Trp Ser Ile Gly Asp Trp Asp Gly Phe Val Ala Gly Ala Pro Gln
180 185 190

Glu Leu Ala Gly Trp Gln Ala His Val Ala Leu Ala Gly Gly Thr Ala
195 200 205

Glu Pro Pro Gln His Leu Lys Glu Tyr Asp Pro Glu Trp Gly Arg Ala
210 215 220

Phe Val Gly Gly Thr Phe Thr Thr Gly Cys Pro His Gln Val Met Leu
225 230 235 240

Ser Gln Val Lys Val Pro Val Leu Phe Thr His His Phe Arg Met Leu
245 250 255

Asp Asp Glu Ser Gly Ser Leu Ile Gly Ala Ala Thr Asp Asp Gln Ala
260 265 270

Ala Arg Val Val Glu Leu Val Glu Asn Ser Gly Ala Pro Leu Thr Tyr
275 280 285

Arg Ser Phe Pro Met Met Gly His Ser Met His Ala Gln Asp Pro Ala
290 295 300

Leu Phe Ala Gly Thr Leu Val Asp Trp Phe Thr Ala Ala Arg Ser

305 310 315

<210> 11
<211> 319
<212> PRT
<213> Mycobacterium gilvum

<400> 11

```
Met Gly Arg Tyr Ala Gly Val Phe Gly Pro His Ala Pro Glu Ala Thr
1               5                   10                  15

Tyr Val Glu His Gly Tyr Pro Glu Arg Leu Phe Asp Thr Gly Glu Val
            20                  25                  30

Gln Leu Asn Tyr Val Val Ala Gly Asp Ala Ala Ala Pro Pro Leu Leu
        35                  40                  45

Leu Ile Pro Gly Gln Ser Glu Ser Trp Trp Gly Tyr Glu Ala Ala Ile
    50                  55                  60

Pro Leu Leu Ala Arg His Phe His Val His Ala Val Asp Leu Arg Gly
65                  70                  75                  80

Gln Gly Arg Ser Thr Arg Thr Pro Gly Arg Tyr Thr Leu Asp Asn Val
                85                  90                  95

Gly Asn Asp Leu Val Arg Phe Leu Asp Gly Val Ile Gly Arg Pro Ala
            100                 105                 110

Phe Val Ser Gly Leu Ser Ser Gly Gly Leu Ala Ser Ala Trp Leu Ser
        115                 120                 125

Ala Phe Ala Lys Pro Gly Gln Val Val Ala Ala Cys Trp Glu Asp Pro
    130                 135                 140

Pro Phe Phe Ser Ser Glu Thr Ala Pro Ile Val Gly Pro Pro Ile Thr
145                 150                 155                 160

Asp Ser Ile Gly Pro Leu Phe Gly Met Trp Ala Arg Tyr Leu Gly Asp
                165                 170                 175

Gln Trp Ser Val Gly Asp Trp Asp Gly Phe Val Ala Ala Val Pro Thr
            180                 185                 190

Glu Leu Ala Asp Trp Gln Ala His Val Ala Leu Val Val Gly Thr Ala
        195                 200                 205

Asp Pro Pro Gln Asn Leu Arg Glu Tyr Asp Pro Glu Trp Gly Lys Ala
    210                 215                 220

Phe Ile Thr Gly Thr Phe Ala Ala Ser Cys Pro His His Val Met Leu
225                 230                 235                 240
```

```
Ser Lys Val Lys Val Pro Val Leu Tyr Thr His His Phe Arg Met Ile
                245                 250                 255

Asp Glu Gly Ser Gly Gly Leu Ile Gly Ala Cys Ser Asp Ile Gln Ala
            260                 265                 270

Gly Arg Val Thr Gln Leu Ala Lys Ser Gly Gly Arg Ser Val Thr Tyr
        275                 280                 285

Arg Ser Phe Pro Met Met Ala His Ser Met His Gly Gln Asp Pro Ala
    290                 295                 300

Leu Phe Ser Glu Thr Leu Val Glu Trp Phe Ser Arg Phe Thr Gly
305                 310                 315
```

<210> 12
<211> 322
<212> PRT
<213> Gordonia effusa

<400> 12

```
Met Pro Lys Ser Glu Ala Ala Asp Arg Ala Asp Ser Phe Val Ser His
1               5                   10                  15

Asp Phe Lys Glu Asn Ile Val Asp Leu Gly Glu Ile Arg Met Asn Tyr
            20                  25                  30

Val Val Gln Gly Asn Lys Lys Ser Pro Ala Leu Leu Leu Ile Pro Ala
        35                  40                  45

Gln Gly Glu Ser Trp Trp Gly Tyr Glu Ala Ala Ile Pro Leu Leu Ala
    50                  55                  60

Lys His Phe Gln Val Phe Ala Ile Asp Leu Arg Gly Gln Gly Arg Thr
65                  70                  75                  80

Thr Trp Thr Pro Gly Arg Tyr Thr Leu Asp Ile Phe Gly Asn Asp Val
                85                  90                  95

Val Arg Phe Ile Asp Leu Val Ile Gly Arg Glu Thr Leu Ile Ala Gly
            100                 105                 110

Asn Ser Ser Gly Gly Leu Ile Gly Ala Trp Leu Ala Ala Phe Ala Lys
        115                 120                 125

Pro Gly Gln Val Arg Ala Val Met Leu Glu Asp Pro Pro Leu Phe Ala
    130                 135                 140

Ser Glu Ile Arg Pro Pro Tyr Gly Pro Gly Ile Trp Gln Gly Leu Gly
145                 150                 155                 160
```

Pro Met Phe Ala Ala Trp Ala Lys Trp Leu Gly Pro Gln Trp Ser Ile
165 170 175

Gly Asp Trp Asp Gly Met Val Lys Ala Leu Pro Asp Glu Leu Pro Glu
180 185 190

Asp Leu Leu Pro Gly Ile Gly Phe Met Leu Gly Asp Gly Glu Ser Asp
195 200 205

Gly Ala Ala Pro Thr Pro Pro Gln His Leu Lys Glu Tyr Asp Pro Glu
210 215 220

Trp Gly Ala Ser Trp Ala Ser Gly Phe Ala Asn Thr Gly Cys Glu His
225 230 235 240

Glu Ala Val Ile Ser Gln Val Arg Val Pro Val Leu Leu Thr His His
245 250 255

Phe Arg Gln Ile Asn Glu Glu Thr Gly His Leu Met Gly Ala Leu Ser
260 265 270

Asp Leu Gln Ala Ala Gln Val Arg His Ile Ile Glu Glu Val Ala Gly
275 280 285

Gln Glu Val Thr Tyr Val Ser Leu Asp Ala Pro His Thr Met His Glu
290 295 300

Pro Gln Pro Glu Arg Tyr Thr Asp Val Leu Leu Asp Trp Val Lys Lys
305 310 315 320

Leu Gly

<210> 13
<211> 328
<212> PRT
<213> Togninia minima

<400> 13

Met Asn Tyr Ala Thr Ala Gly Ser Ser Asp Lys Pro Ala Leu Leu Leu
1 5 10 15

Val Pro Gly Gln Ser Glu Ser Trp Trp Gly Tyr Glu Met Ala Met Trp
20 25 30

Leu Leu Lys Asp Asp Tyr Gln Val Phe Ala Val Asp Met Arg Gly Gln
35 40 45

Gly Gln Ser Thr Trp Thr Pro Gly Arg Tyr Ser Leu Asp Thr Phe Gly
50 55 60

```
Asn Asp Leu Val Lys Phe Ile Asp Ile Val Ile Lys Arg Pro Val Val
65                  70                  75                  80

Val Ser Gly Leu Ser Ser Gly Gly Val Val Ser Ala Trp Leu Ser Ala
                85                  90                  95

Phe Ala Lys Pro Gly Gln Ile Arg Ala Ala Val Tyr Glu Asp Pro Pro
            100                 105                 110

Leu Phe Ala Ser Gln Ser Lys Pro Ala Ile Gly Gln Ser Val Met Gln
        115                 120                 125

Thr Val Ala Gly Pro Phe Phe Asn Leu Trp Tyr Lys Trp Leu Gly Ala
    130                 135                 140

Gln Trp Thr Ile Gly Asp Gln Ala Gly Met Val Ala Ala Met Pro Lys
145                 150                 155                 160

Glu Ile Pro Ala Trp Ile Leu Gln Tyr Leu Gly Asn Thr Thr Ser Gly
                165                 170                 175

Pro Thr Gly Leu Asp Leu Thr Leu Asn Glu Tyr Asp Pro Glu Trp Gly
            180                 185                 190

His Gly Phe Val Ser Gly Thr Val Asp Ala Thr Cys Asp His Glu Ala
        195                 200                 205

Met Leu Thr His Val Lys Val Pro Val Leu Phe Thr His His Ser Arg
    210                 215                 220

Ala Ile Asp Pro Tyr Thr Gly Asn Leu Ile Gly Ser Val Ser Asp Thr
225                 230                 235                 240

Gln Val Ser Tyr Ala Gln Gly Leu Ile Thr Thr Asn Gly Asn Gln Ser
                245                 250                 255

Phe Thr Leu Lys Asn Phe Pro Leu Ala Ser His Asp Met His Asn Ser
            260                 265                 270

Asp Pro Ala Thr Tyr Val Ser Ala Ile Thr Thr Trp Met Ala Ser Leu
        275                 280                 285

Gly Ile Gly Ser Ala Val Ile Pro Gly Pro Val Lys Val Ala Ser Ala
    290                 295                 300

Ser Ala Gln Val Ser Ala Ala Ser Thr Ala Pro Pro Ser Cys Thr Ser
305                 310                 315                 320

Thr Ser Ala Pro Ser Thr Gly His
                325
```

<210> 14

<211> 280
<212> PRT
<213> Actinosynnema mirum

<400> 14

```
Met Thr Val Val Asp Pro Pro Ala Pro Arg Asp Phe Pro Glu Leu Leu
1               5                   10                  15

Val Asp Leu Gly Glu Val Val Leu Asn His Ala Glu Ala Gly Ser Pro
            20                  25                  30

Asp Arg Pro Ala Leu Val Pro Val Pro Glu Gln Gly Gly Ser Trp Trp
        35                  40                  45

Ser Tyr Glu Arg Val Met Pro Leu Pro Ala Arg Asp Phe His Val Phe
    50                  55                  60

Ala Val Asp Leu Arg Gly Arg Gly Arg Ser Thr Arg Thr Pro Arg Arg
65                  70                  75                  80

Tyr Ser Leu Asp Asp Phe Gly Asn Asp Leu Val Arg Phe Leu Ala Leu
                85                  90                  95

Val Val Arg Arg Pro Ala Val Val Ala Gly Asn Ser Ser Gly Gly Val
            100                 105                 110

Leu Ala Ala Trp Ser Ser Ala Tyr Ala Met Pro Gly Gln Val Arg Ala
        115                 120                 125

Val Leu Leu Glu Asp Pro Pro Leu Phe Ser Ser Glu Leu Thr Pro Val
    130                 135                 140

Cys Gly Pro Gly Val Arg Gln Ala Ala Gly Pro Leu Phe Glu Leu Leu
145                 150                 155                 160

Ser Thr His Leu Gly Asp Gln Trp Gly Gly Gly Arg Pro Gly Arg Val
                165                 170                 175

His Gly Gly Val Pro Arg Leu Gly Leu Ala Ala Ala Ala Ala Val Arg
            180                 185                 190

Val Ala Arg Arg Ala Ala Ala Thr Asp Ala Arg Gly Arg Pro Gly Ala
        195                 200                 205

Ala Arg Gly Arg Pro Ala Gly Val Gly Gly Ala Ala Arg Arg Gly Arg
    210                 215                 220

Gly Gly Arg Glu Arg Thr Gly Thr Thr Thr Val Leu Ser Gly Leu Thr
225                 230                 235                 240

Gly Ser Arg Thr Ser Gly Thr Gly Arg Cys Arg Lys Pro Phe Arg Leu
```

245 250 255

Arg Gln Trp Trp Ala Gly Gly Ala Arg Gly Pro Pro Pro Pro Arg Gln
260 265 270

Ile Arg Ala Asp Val Arg Thr Arg
275 280

<210> 15
<211> 987
<212> DNA
<213> Rhodococcus erythropolis

<220>
<221> misc_feature
<223> DNA sequence encodes polypeptid with SEQ ID NO: 1

<400> 15

```
atggccgaag aaggaactag gtccgaagca gcggatgctg ccacacaagc gagacagcta      60
cccgattcgc ggaacatctt tgtctcgcac cgatttccgg aaaggcaggt cgatctcggt     120
gaagtggtga tgaacttcgc ggaggcgggc tctccggaca acccggcact gctcctcctc     180
cccgagcaga ccgggtcgtg gtggagttac gagccagtga tgggtcttct ggcagagaac     240
tttcatgtct ttgccgtcga tatccgtggg caaggtcgca gtacctggac gccacggcga     300
tacagcctgg acaacttcgg caatgatctg gtgcgtttca tcgctctggt catcaagcgc     360
cctgtcgtcg tggcagggaa ctcctcgggg gggctgctgg ccgcctggct ctcggcgtac     420
gcgatgcccg gccagatccg tgcagcattg tgtgaggacg caccgttctt tgcgtcggag     480
ttggtccccg catacggtca ctcggttctg caggcggcgg gtccggcatt cgagttgtac     540
cgggacttcc tcggggacca gtggtcgatt ggggactgga aagggttcgt tgaggcagcc     600
aaagcgtcgc cggcaaaggc tatgcaatta tttccgaccc cggatgaggc gccgcagaat     660
ctcaaggaat acgacccgga atgggggcgc gcattcttcg aagggactgt ggcactgcac     720
tgcccacacg acaggatgct ctcgcaagtc aagacaccaa ttctcatcac tcaccacgcg     780
cggacgatcg accccgagac gggcgagctg ttgggcgcgc tctccgacct tcaggcagag     840
catgcgcagg acatcattcg gtctgcgggc gttcgggtgg actatcagtc gcaccccgac     900
gcgcttcaca tgatgcatct gttcgatccc gctcgttacg cggagatctt gacatcctgg     960
tccgcaacac tgcctgcgaa cgactag                                         987
```

<210> 16
<211> 987
<212> DNA
<213> Artificial sequence

<220>
<223> ORF was codon optimized and thus differce from natural occuring DNA sequence.

<220>
<221> misc_feature
<223> Artificial DNA sequence encodes polypeptid with SEQ ID NO: 1

<400> 16

```
atggcagaag aaggcacccg tagcgaagca gcagatgcag caacccaggc acgtcagctg       60
ccggatagcc gtaacatttt tgttagccat cgttttccgg aacgtcaggt tgatctgggt      120
gaagttgtta tgaattttgc agaagcaggt agtccggata atccggcatt actgctgctg      180
ccggaacaga ccggtagttg gtggtcttat gaaccggtta tgggtctgct ggcagaaaac      240
tttcatgttt ttgcagttga tattcgtggt cagggtcgta gcacctggac accgcgtcgt      300
tatagcctgg ataattttgg taatgatctg gtgcgtttta ttgccctggt tattaaacgt      360
ccggttgttg ttgcaggtaa tagcagcggt ggcctgctgg ctgcatggct gagcgcctat      420
gcaatgcctg gtcagattcg tgcagcactg tgtgaagatg caccgttttt tgcaagcgaa      480
ctggttcctg cctatggtca tagcgttctg caggcagcag gtccggcatt tgaactgtat      540
cgtgattttc tgggtgatca gtggtcaatt ggtgattgga aaggttttgt tgaagcagca      600
aaagcaagtc cggctaaagc aatgcagctg tttccgacac cggatgaagc accgcagaat      660
ctgaaagaat atgatccgga atggggtcgt gcatttttg aaggcaccgt tgcactgcat       720
tgtccgcatg atcgtatgct gagccaggtt aaaaccccga ttctgattac ccatcatgca      780
cgtaccatcg atccggaaac cggtgaactg ctgggtgcac tgagtgatct gcaggccgaa      840
catgcacagg atattattcg tagtgccggt gttcgtgttg attatcagag ccatcctgat      900
gcactgcaca tgatgcacct gtttgatccg gcacgttatg cagaaattct gaccagttgg      960
agcgcaaccc tgcctgcaaa tgattaa                                           987
```

<210> 17
<211> 927
<212> DNA
<213> Artificial sequence

<220>
<223> ORF was codon optimized and thus differce from natural occuring DNA sequence.

<220>
<221> misc_feature
<223> Artificial DNA sequence encodes polypeptid with SEQ ID NO: 2

<400> 17

```
atggcagatc cggcacagcg tgatgtttat gttccgcatg catatccgga aaaacaggca      60
gatctgggtg aaattaccat gaattatgcc gaagccggtg aacctgatat gcctgcagtt     120
ctgctgattc cggaacagac cggtagttgg tggggttatg aagaagcaat gggtctgctg     180
gcagaaaact ttcatgttta tgcagttgat ctgcgtggtc agggtcgtag cagctgggca     240
ccgaaacgtt atagcctgga taattttggt aatgatctgg tgcgttttat tgccctggtt     300
gttaaacgtc cggttattgt tgcaggtaat agcagcggtg gtgttctggc agcatggctg     360
agcgcatata gcatgcctgg tcaggttcgt ggtgcactgt gtgaagatgc accgtttttt     420
gcaagcgaac tggttaccac ctgtggtcat agcattcgtc aggcagcagg tccgatgttt     480
gaactgtttc gtacctatct gggcgatcag tggtcagttg gtgattggac cggctattgt     540
cgtgcagcag atgcaagcag cagcccgatg gcacgttatt ttgttgcaga tgaaattccg     600
cagcacatgc gtgaatatga tccggaatgg gcacgtgcat tttgggaagg caccgttgca     660
ctgcattgtc cgcatgaaca gctgctgacc caggttaaaa caccggtgct gctgacacat     720
cacatgcgcg atattgatcc tgataccggt catctggttg gtgccctgag tgatgaacag     780
gcagcccgtg cacgtctgct gatggaaagt gccggtgtta aagttgatta tgcaagcgtt     840
ccggatgcac tgcacatgat gcaccagttt gatccgcctc gttatgttga aatctttacc     900
cagtgggcag caaccctggc agcataa                                         927
```

<210> 18
<211> 930
<212> DNA
<213> Artificial sequence

<220>
<223> ORF was codon optimized and thus differce from natural occuring DNA sequence.

<220>
<221> misc_feature
<223> Artificial DNA sequence encodes polypeptid with SEQ ID NO: 3

<400> 18

```
atggttacca gtccggcact gcgtgatgtt catgttccgc atgcatatcc ggaacagcag    60
gttgatctgg gtgaaattac catgaattat gccgaagccg gtgatccggg tcgtccggca   120
gttctgctga tcccggaaca gaccggtagt tggtggtctt atgaagaagc aatgggtctg   180
ctggcagaac attttcatgt ttatgcagtt gatctgcgtg gtcagggtcg tagcagctgg   240
accccgaaac gttatagcct ggataatttt ggtaatgatc tggtgcgttt tattgccctg   300
gttgttcgtc gtccggttgt tgttgcaggt aatagcagcg gtggtgttct ggcagcatgg   360
ctgagcgcat atagcatgcc tggtcagatt cgtggtgtgc tgtgtgaaga tccgcctttt   420
tttgcaagcg aactggttcc ggcacatggt catagcgttc gtcagggtgc aggtccggtt   480
tttgaactgt ttcgtaccta tctgggcgat cagtggtcag ttggtgattg ggaaggtttt   540
cgtagcgcag cagatgcaag cgcaagcccg atggcacgta gctttgttgc agataccatt   600
ccgcagcatc tgaaagaata tgatccggaa tgggcacgtg cattttatga aggcaccgtt   660
ggtctgaatt gtccgcatga acgtatgctg aatcgtgtta atacaccggt gctgctgacc   720
catcacatgc gtggcaccga tccggaaacc ggtaatctgc tgggtgcact gagtgatgaa   780
caggcagcac aggtgcgtcg tctgatggaa agtgccggtg ttaaagttga ttatgaaagc   840
gttccggatg caagccacat gatgcaccag agcgatccgg cacgttatgc agaaattctg   900
accccgtgga ccgcagcact ggcaccgtaa                                    930
```

<210> 19
<211> 930
<212> DNA
<213> Artificial sequence

<220>
<223> ORF was codon optimized and thus differce from natural occuring DNA sequence.

<220>
<221> misc_feature
<223> Artificial DNA sequence encodes polypeptid with SEQ ID NO: 4

<400> 19

atggttacca gtccggcact gcgtgatgtt catgttccgc atgcatatcc ggaacagcag 60
gttgatctgg gtgaaattac catgaattat gccgaagccg gtgatcctga tcgtccggca 120
gttctgctga tcccggaaca gaccggtagt tggtggtcat atgaagaagc aatgggtctg 180
ctggcagaac attttcatgt ttatgcagtt gatctgcgtg gtcagggtcg tagcagctgg 240
accccgaaac gttatagcct ggataatttt ggtaatgatc tggtgcgttt tattgccctg 300
gttgttaaac gtccggttgt tgttgcaggt aatagcagcg gtggtgttct ggcagcatgg 360
ctgagcgcat atagcatgcc tggtcagctg cgtggtgtgc tgtgtgaaga tccgcctttt 420
tttgcaagcg aactggttcc ggcacatggt catagcgttc gtcagggtgc aggtccggtt 480
tttgaactgt ttcgtaccta tctgggcgat cagtggtcag ttagcgattg ggaaggtttt 540
tgtcgtgcag ccggtgcaag cgcaagcccg atggcacgta gctttgttgc agatggtatt 600
ccgcagcatc tgaaagaata tgatccggaa tgggcacgtg catttcatga aggcaccgtt 660
ggtctgaatt gtccgcatga acgtatgctg ggtcgtgtta atacaccggt gctgctgacc 720
catcatatgc gtggcaccga tccggaaacc ggtaatctgc tgggtgcact gagtgatgaa 780
caggcagcac aggcacgtct gctgatggaa agtgccggtg ttcgtgttga ttatgaaagc 840
gttccggatg caagccatat gatgcaccag agcgatccgg cacgttatgc agaaatcttt 900
acccgttggg cagcagccct ggcaccgtaa 930

<210> 20
<211> 930
<212> DNA
<213> Artificial sequence

<220>
<223> ORF was codon optimized and thus differce from natural occuring DNA sequence.

<220>
<221> misc_feature
<223> Artificial DNA sequence encodes polypeptid with SEQ ID NO: 5

<400> 20

atggttacca gtccggcact gcgtgatgtt catgttccgc atgcatatcc ggaacagcag 60
gttgatctgg gtgaaattac catgaattat gccgaagccg gtgatccggg tcgtccggca 120
gttctgctga tcccggaaca gaccggtagt tggtggtctt atgaagaagc aatgggtctg 180

ctggcagaac attttcatgt ttatgcagtt gatctgcgtg gtcagggtcg tagcagctgg 240
accccgaaac gttatagcct ggataatttt ggtaatgatc tggtgcgttt tatggcactg 300
gttgttcgtc gtccggttgt tgttgcaggt aatagcagcg gtggtgttct ggcagcatgg 360
ctgagcgcat atagcatgcc tggtcagatt cgtggtgtgc tgtgtgaaga tccgcctttt 420
tttgcaagcg aactggttcc ggcacatggt catagcgttc gtcagggtgc aggtccggtt 480
tttgaactgt ttcgtaccta tctgggcgat cagtggtcag ttggtgattg ggaaggtttt 540
cgtagcgcag ccggtgcaag cgcaagcccg atggcacgta gctttgttgc agataccatt 600
ccgcagcatc tgaaagaata tgatccggaa tgggcacgtg cattttatga aggcaccgtt 660
ggtctgaatt gtccgcatga acgtatgctg aatcgtgtta atacaccggt gctgctgacc 720
catcacatgc gtggcaccga tccggaaacc ggtaatctgc tgggtgcact gagtgatgaa 780
caggcagcac aggcacgtcg tctgatggaa agtgccggtg ttaaagttga ttatgaaagc 840
gttccggatg caagccacat gatgcaccag agcgatccgg cacgttatgc agaaattctg 900
accccgtggg cagcagccct ggcaccgtaa 930

<210> 21
<211> 930
<212> DNA
<213> Artificial sequence

<220>
<223> ORF was codon optimized and thus differce from natural occuring DNA sequence.

<220>
<221> misc_feature
<223> Artificial DNA sequence encodes polypeptid with SEQ ID NO: 6

<400> 21

```
atggttacca gtccggcact gcgtgatgtt catgttccgc atgcatatcc ggaacagcag     60
gttgatctgg gtgaaattac catgaattat gccgaagccg gtgatcctga tcgtccggca    120
gttctgctga tcccggaaca gaccggtagt tggtggtctt atgaagaagc aatgggtctg    180
ctgagcgaac attttcatgt ttatgcagtt gatctgcgtg gtcagggtcg tagcagctgg    240
accccgaaac gttatagcct ggataatttt ggtaatgatc tggtgcgttt tattgccctg    300
gttgttaaac gtccggttgt tgttgcaggt aatagcagcg gtggtgttct ggcagcatgg    360
ctgagcgcat atagcatgcc tggtcagctg cgtggtgtgc tgtgtgaaga tccgcctttt    420
tttgcaagcg aactggttcc ggcacatggt catagcgttc gtcagggtgc aggtccggtt    480
tttgaactgt ttcgtaccta tctgggcgat cagtggtcag ttggtgattg ggaaggtttt    540
tgtcgtgcag ccggtgcaag cgcaagcccg atggcacgta gctttgttgc agatggtatt    600
ccgcagcatc tgcaagaata tgatccggaa tgggcacgtg tttttatga aggcaccgtt     660
ggtctgagct gtccgcatga acgtatgctg ggtcaggtta aaacaccggt gctgctgacc    720
catcacatgc gtggtatcga tccggaaacc ggtaatctgc tgggtgcact gagtgatgaa    780
caggccctgc gtgcacgtcg tctgatggat agtgccggtg ttaccgttga ttatgaaagc    840
gttccggatg caagccacat gatgcaccag agcgcaccgg cacgttatgt tgaaatcttt    900
acccgttggg cagcagccct ggcaccgtaa                                      930
```

<210> 22
<211> 903
<212> DNA
<213> Artificial sequence

<220>
<223> ORF was codon optimized and thus differce from natural occuring DNA sequence.

<220>
<221> misc_feature
<223> Artificial DNA sequence encodes polypeptid with SEQ ID NO: 7

<400> 22

```
atgccgcacg attatgaaga aaaactggtt gatctgggcg aaatcgatct gaattatgca      60
gaagcaggta gtccggataa accggcactg ctgctgattc cgagccagag cgaaagttgg     120
tggggctatg aagaagcaat gggtctgctg gccgaagatt atcatgtttt tgcagttgat     180
atgcgtggtc agggtcgtag cacctggaca ccgggtcgtt atagcctgga taattttggt     240
aatgatctgg tgcgctttat cgatctggtt attggtcgta ccgttattgt tagcggtaat     300
agcagcggtg gtgttgttgc agcatggctg gcagcattta gcctgcctgg tcaggttcgt     360
gcagcactgg cagaagatgc accgtttttt gcaagcgaac tggacccgaa agtgggtcat     420
accattcgtc aggcagcagg tcatattttt gttaactggc gtgattatct gggtgatcag     480
tggtcagttg gtgattatgc aggttttctg aaagcaatga aaagcagcga agttccgatg     540
ctgcgtcagg ttccgctgcc ggaaaccgca ccgcagaatc tgctggaata tgatccggaa     600
tgggcacgtg cattttatga aggcaccgtt gcacagacct gtccgcatga ttatatgctg     660
agccaggtta aagtgcctat gctggttacc catcatgcac gtatgattga tgaagcaacc     720
agcggtctgg ttggtgcaat gagcgatctg caggttcaga aagcagcaga aattattcgt     780
ggcaccggtg ttcaggttga tgttgttgat ctgccggaag caccgcatat tctgcatcag     840
ctggcaccga aagaatatgt ggaaattctg aataactggg tggaaaaact gcctccggtt     900
taa                                                                   903
```

<210> 23
<211> 924
<212> DNA
<213> Artificial sequence

<220>
<223> ORF was codon optimized and thus differce from natural occuring DNA sequence.

<220>
<221> misc_feature
<223> Artificial DNA sequence encodes polypeptid with SEQ ID NO: 8

<400> 23

```
atgatccaga acaataaaac cgcaccgtat aaatacaaag aaaaactggt tgatctgggc 60
gaaatcaaaa tgaactatat tgttgccggt gcagatgtta gtccggcact gctgctgatt 120
ccgggtcaga ccgaaagttg gtggggtttt gaagcagcaa ttgagaaact ggaaagcaac 180
tttcaggtgt ttgcaattga tctgcgtggt cagggtaaaa gcacccagac accgggtcgt 240
tatagcctga atctgatggg taatgatctg gttcgtttta ttagcctggt tattaaacgt 300
ccggttattg ttagcggtaa tagcagcggt ggtctgctgg cagcatggct gagcgcctat 360
gcaatgccga atcagattcg tgcaattcat tgtgaagatg caccgttttt taccgcagaa 420
aaagcaccgc tgtatggtca tgcaattcag caggcagcag gtccgatttt tagcctgatg 480
agcaaatttc tgggtgatca gtggtcaatt aacaattggg aaggtctgaa agcagcacag 540
gcaaaagata cccatccggc aaacaaaatg attagccagg ttgaacagcc tccgcagcat 600
ctgaaagaat atgatccgga atggggtcgt gcatttattg aaggcaaatt taacctgaac 660
agtccgcatc ataccctgct gagcgacatt aaaaccccga tgctgtatac ccatcacatg 720
cgttttgaag atccgcagac aggtctgctg attggtgcaa ccagcgattt tcaggcaagc 780
aaaatcaaag aaattgccct gaaaaccggc aatagcttcg aactgattga tgcaccggat 840
gcatttcata gtatgcatga agccgatccg cagcgttttg ttgatattct gaccagctgg 900
attgaacgtc tgaatctgca gtaa 924
```

<210> 24
<211> 966
<212> DNA
<213> Artificial sequence

<220>
<223> ORF was codon optimized and thus differce from natural occuring DNA sequence.

<220>
<221> misc_feature
<223> Artificial DNA sequence encodes polypeptid with SEQ ID NO: 9

<400> 24

```
atgggtatta gcgaagcagc agatcgtgca gatacctttg ttgcacataa atttgaagaa 60
cagctggttg atctgggtga aattcgtatg aattatgttg cagccggtga tccgaccagt 120
ccggcactgc tgctgattcc ggcacagggt gaaagttggt ggggttatga aatgcaatt 180
accctgctgg caaatgattt tcgtgttttt gcaattgatc tgcgtggtca gggtcgtagc 240
acctggacac cgggtcgtta taatctgaat acctggggta atgatgtgga acgctttatt 300
gatctggtta ttggtcgtcc gaccctggtt gcaggtaata gcagcggtgg tgttattgca 360
gcatggctgg cagcctatgc aaaaccgggt cagattcgtg gtgcaatgct ggaagatccg 420
cctctgtttg caagccaggc agcaccgcct tatggtccgg gtattatgca gaccctgggt 480
ccgatttttg ttctgtgggc aaaatggctg ggtccgcagt ggtcagttgg tgattgggat 540
```

ggtatggttg cagcggcacc gcgtgaactg ccggaatttc tgcatccggg tatcgcattt 600
ctgtttggtg atggcaccgg tgaaggtgca gcagcaaccc ctccgcagca tctgaaagaa 660
tatgatccgg aatgggcaca ggcatgggca accgatgttg caaatgcagg ttgtgatcat 720
gcaaccatgc tggcacagaa tcgtgttccg gttctgctga cccatcattt tcatctgacc 780
gatccggata caggccagct gatgggtgca atgaccgata ttcaggcaca gcaggcacgt 840
cgtctgctgg cagcaaccgg tcagccggtt acctttaccg cactggatgc accgcatacc 900
atgcatgatc ctgaacctga acgttatttt gaagttctga ccgaatgggc aagtgcactg 960
gattaa 966

<210> 25
<211> 960
<212> DNA
<213> Artificial sequence

<220>
<223> ORF was codon optimized and thus differce from natural occuring DNA sequence.

<220>
<221> misc_feature
<223> Artificial DNA sequence encodes polypeptid with SEQ ID NO: 10

<400> 25

atgggtcgtt atgccggtgt tttggtccg catgcaccgg aaagcaccta tgttggtcat 60
gcatatccgg aacaactgtt tgataccggt gaagttcgtc tgaattatgc agttgccggt 120
gatgcaagcg caagtccgct gctgctgatt ccgggtcaga ccgaaagttg gtggggttat 180
gaaccggcaa tgggtctgct ggcagaacat tttcatgttc atgcagttga tctgcgtggt 240
cagggtcgta gcacccgtac accgcgtcgt tataccctgg ataatattgg taatgatctg 300
gtgcgttttc tggatggtgt tattggtcgt ccggcatttg ttagcggtct gagcagcggt 360
ggtctgctga gcgcatggct gagcgccttt gcagaaccgg gtcaggttct ggcagcatgt 420
tatgaagatc cgcctttttt tagcagcgaa ctggacccgg tgattggtcc gggtctgatg 480
agcaccgttg gtccgctgtt tgcactgtat gttaaatatc tgggtgatca gtggtcaatt 540
ggtgattggg atggttttgt tgcaggcgca ccgcaagaac tggcaggttg gcaggcacat 600
gttgcactgg caggcggtac agcagaaccg cctcagcatc tgaaagaata tgatccggaa 660
tggggtcgtg catttgttgg tggcaccttt accaccggtt gtccgcatca ggttatgctg 720
agccaggtta aagttccggt tctgtttacc catcattttc gtatgctgga tgatgaaagc 780
ggtagcctga ttggtgcagc aaccgatgat caggcagcac gtgttgttga actggttgaa 840
aatagtggtg caccgctgac ctatcgtagc tttccgatga tgggtcatag tatgcatgca 900
caagatccgg cactgtttgc aggcaccctg gttgattggt ttaccgcagc acgtagctaa 960

<210> 26

<211> 960
<212> DNA
<213> Artificial sequence

<220>
<223> ORF was codon optimized and thus differce from natural occuring DNA sequence.

<220>
<221> misc_feature
<223> Artificial DNA sequence encodes polypeptid with SEQ ID NO: 11

<400> 26

```
atgggtcgtt atgccggtgt ttttggtccg catgcaccgg aagcaaccta tgttgaacat      60
ggttatccgg aacgtctgtt tgataccggt gaagtgcagc tgaattatgt tgttgccggt     120
gatgcagcag caccgcctct gctgctgatt ccgggtcaga gcgaaagttg gtggggttat     180
gaagcagcaa ttccgctgct ggcacgtcat tttcatgttc atgcagttga tctgcgtggt     240
cagggtcgta gcacccgtac accgggtcgc tataccctgg ataatgttgg taatgatctg     300
gtgcgttttc tggatggtgt tattggtcgt ccggcatttg ttagcggtct gagcagcggt     360
ggtctggcaa gcgcatggct gagcgcattt gcaaaaccgg gtcaggttgt tgcagcatgt     420
tgggaagatc cgcctttttt tagcagcgaa accgcaccga ttgttggtcc gcctattacc     480
gatagcattg gtccgctgtt tggtatgtgg gcacgttatc tgggtgatca gtggtcagtt     540
ggtgattggg atggttttgt tgccgcagtt ccgaccgaac tggcagattg gcaggcacat     600
gttgcactgg ttgttggcac cgcagatcct ccgcagaatc tgcgtgaata tgatccggaa     660
tggggtaaag catttattac cggcaccttt gcagcaagct gtccgcatca tgttatgctg     720
agcaaagtta aagttccggt tctgtatacc catcactttc gcatgattga tgaaggtagt     780
ggtggtctga ttggtgcatg tagcgatatt caggcaggtc gtgttaccca gctggcaaaa     840
tcaggtggtc gtagcgttac ctatcgtagc tttccgatga tggcacatag catgcatggt     900
caagatccgg cactgtttag cgaaaccctg gttgaatggt ttagccgttt taccggttaa     960
```

<210> 27
<211> 969
<212> DNA
<213> Artificial sequence

<220>
<223> ORF was codon optimized and thus differce from natural occuring DNA sequence.

<220>
<221> misc_feature
<223> Artificial DNA sequence encodes polypeptid with SEQ ID NO: 12

<400> 27

atgccgaaaa gcgaagcagc agatcgtgca gatagctttg ttagccatga tttcaaagaa 60
aacattgtgg atctgggcga aatccgcatg aattatgttg ttcagggcaa caaaaaaagt 120
ccggcactgc tgctgattcc ggcacagggt gaaagttggt ggggttatga agcagcaatt 180
ccgctgctgg caaaacattt tcaggttttt gcaattgatc tgcgtggtca gggtcgtacc 240

acctggacac cgggtcgtta taccctggat atttttggta atgatgtggt gcgctttatc 300
gatctggtta ttggtcgtga aaccctgatt gcaggtaata gcagcggtgg tctgattggt 360
gcatggctgg cagcatttgc aaaaccgggt caggttcgtg cagttatgct ggaagatccg 420
cctctgtttg caagcgaaat tcgtccgcct tatggtccgg gtatttggca gggtctgggt 480
ccgatgtttg cagcatgggc aaaatggctg ggtccgcagt ggtcaattgg tgattgggat 540
ggtatggtta aagcactgcc ggatgaactg ccggaagatc tgctgcctgg tattggtttt 600
atgctgggtg atggtgaaag tgatggtgca gcaccgaccc ctccgcagca tctgaaagaa 660
tatgatccgg aatggggtgc aagctgggca agcggttttg ccaataccgg ttgtgaacat 720
gaagcagtta ttagccaggt gcgtgttccg gttctgctga cccatcattt tcgtcagatt 780
aatgaagaaa ccggtcatct gatgggtgca ctgagcgatc tgcaggcagc acaggttcgt 840
catatcattg aagaagttgc aggtcaagag gttacctatg ttagcctgga tgcaccgcat 900
accatgcatg aaccgcagcc ggaacgttat accgatgttc tgctggattg ggttaaaaaa 960
ctgggttaa 969

<210> 28
<211> 987
<212> DNA
<213> Artificial sequence

<220>
<223> ORF was codon optimized and thus differce from natural occuring DNA sequence.

<220>
<221> misc_feature
<223> Artificial DNA sequence encodes polypeptid with SEQ ID NO: 13

<400> 28

atgaattatg caaccgcagg tagcagcgat aaaccggcac tgctgctggt tccgggtcag 60
agcgaaagtt ggtggggtta tgaaatggca atgtggctgc tgaaagatga ttatcaggtt 120
tttgcagttg atatgcgtgg tcagggtcag agtacctgga caccgggtcg ttatagcctg 180
gatacctttg gtaatgatct ggtgaaattc atcgatatcg tgattaaacg tccggttgtt 240
gttagcggtc tgagcagcgg tggtgttgtg agcgcatggc tgagcgcatt tgcaaaacct 300
ggtcagattc gtgcagcagt ttatgaagat ccgcctctgt ttgcaagcca gagcaaaccg 360
gcaattggtc agagtgttat gcagaccgtt gcaggtccgt tttttaacct gtggtataaa 420
tggctgggtg cacagtggac cattggtgat caggcaggta tggttgcagc aatgccgaaa 480
gaaattccgg catggattct gcagtatctg ggtaatacca ccagtggtcc gaccggtctg 540
gatctgacac tgaatgaata tgatccggaa tggggtcatg gttttgttag tggcaccgtt 600
gatgcaacct gtgatcatga agcaatgctg acccatgtta aagttccggt tctgtttacc 660
catcatagcc gtgcaattga tccgtatacc ggtaatctga ttggtagcgt tagcgatacc 720
caggttagct atgcacaggg tctgattacc accaatggca atcagagctt taccctgaaa 780
aactttccgc tggcaagcca tgatatgcat aattctgatc cggcaaccta tgttagcgca 840
attaccacct ggatggcaag cctgggtatt ggtagtgcag ttattccggg tccggttaaa 900
gttgcaagcg caagcgcaca ggttagcgca gcaagcaccg caccgcctag ctgtaccagc 960
accagcgcac cgagcaccgg tcattaa 987

<210> 29
<211> 843
<212> DNA
<213> Artificial sequence

<220>
<223> ORF was codon optimized and thus differce from natural occuring DNA sequence.

<220>
<221> misc_feature
<223> Artificial DNA sequence encodes polypeptid with SEQ ID NO: 14

<400> 29

```
atgaccgttg ttgatccgcc tgcaccgcgt gattttccgg aactgctggt tgatctgggt   60
gaagttgttc tgaatcatgc agaagcaggt agtccggatc gtccggcact ggttccggtg  120
ccggaacagg gtggtagttg gtggtcttat gaacgtgtta tgccgctgcc tgcacgcgat  180
tttcatgttt ttgcagttga tctgcgtggt cgtggtcgta gcacccgtac accgcgtcgt  240
tatagcctgg atgattttgg taatgatctg gttcgttttc tggccctggt tgttcgccgt  300
ccggcagttg ttgcaggtaa tagcagcggt ggtgttctgg cagcatggtc aagcgcctat  360
gcaatgcctg gtcaggttcg tgcagttctg ctggaagatc cgcctctgtt tagcagcgaa  420
ctgacaccgg tttgtggtcc gggtgttcgt caggcagcag gtccgctgtt tgaactgctg  480
agcacccatc tgggcgatca gtggggtggt ggtcgtccgg gtcgtgttca tggtggcgtt  540
ccgcgtctgg gtctggcagc cgcagcagca gttcgtgttg cacgtcgtgc agcagcaacc  600
gatgcacgtg gtcgccctgg tgcagcacgt ggacgtcctg ccggtgttgg tggtgcagct  660
cgtcgcggtc gcggtggtcg tgaacgcacc ggtacaacca ccgttctgag cggtctgacc  720
ggtagccgta ccagcggcac cggtcgttgt cgtaaaccgt ttcgtctgcg tcagtggtgg  780
gcaggcggtg cccgtggtcc tcctccgcct cgtcagattc gcgcagatgt tcgtacccgt  840
taa                                                                 843
```

<210> 30
<211> 7
<212> PRT
<213> Artificial sequence

<220>
<223> amino acid motif

<220>
<221> PEPTIDE
<222> (1)..(7)

<400> 30

```
Ala Gly Asn Ser Ser Gly Gly
1               5
```

<210> 31
<211> 7
<212> PRT
<213> Artificial sequence

<220>
<223> amino acid motif

<220>
<221> PEPTIDE
<222> (1)..(7)

<400> 31

Arg Thr Ile Asp Pro Glu Thr
1 5

<210> 32
<211> 7
<212> PRT
<213> Artificial sequence

<220>
<223> amino acid motif

<220>
<221> PEPTIDE
<222> (1)..(7)

<400> 32

Asp Ala Leu His Met Met His
1 5

<210> 33
<211> 7
<212> PRT
<213> Artificial sequence

<220>
<223> amino acid motif

<220>
<221> PEPTIDE
<222> (1)..(7)

<400> 33

Ala Gly Asp Ser Ser Gly Gly
1 5

<210> 34
<211> 7
<212> PRT
<213> Artificial sequence

<220>
<223> amino acid motif

<220>
<221> PEPTIDE
<222> (1)..(7)

<400> 34

Ala Gly Asp Ser Ser Leu Gly
1 5

<210> 35
<211> 7
<212> PRT
<213> Artificial sequence

<220>
<223> amino acid motif

<220>
<221> PEPTIDE
<222> (1)..(7)

<400> 35

```
Ala Gly Gln Ser Ser Gly Gly
1               5
```

<210> 36
<211> 7
<212> PRT
<213> Artificial sequence

<220>
<223> amino acid motif

<220>
<221> PEPTIDE
<222> (1)..(7)

<400> 36

```
Ala Gly His Ser Ser Gly Gly
1               5
```

<210> 37
<211> 7
<212> PRT
<213> Artificial sequence

<220>
<223> amino acid motif

<220>
<221> PEPTIDE
<222> (1)..(7)

<400> 37

```
Ala Gly Ser Ser Ser Gly Gly

1               5
```

<210> 38
<211> 7

<212> PRT
<213> Artificial sequence

<220>
<223> amino acid motif

<220>
<221> PEPTIDE
<222> (1)..(7)

<400> 38

```
Ser Gly Asn Ser Ser Gly Gly
1               5
```

<210> 39
<211> 7
<212> PRT
<213> Artificial sequence

<220>
<223> amino acid motif

<220>
<221> PEPTIDE
<222> (1)..(7)

<400> 39

```
Ser Gly Asp Ser Ser Gly Gly
1               5
```

<210> 40
<211> 7
<212> PRT
<213> Artificial sequence

<220>
<223> amino acid motif

<220>
<221> PEPTIDE
<222> (1)..(7)

<400> 40

```
Ser Gly Gln Ser Ser Gly Gly
1               5
```

<210> 41
<211> 7
<212> PRT
<213> Artificial sequence

<220>

<223> amino acid motif

<220>
<221> PEPTIDE
<222> (1)..(7)

<400> 41

```
Ser Gly His Ser Ser Gly Gly
1               5
```

<210> 42
<211> 7
<212> PRT
<213> Artificial sequence

<220>
<223> amino acid motif

<220>
<221> PEPTIDE
<222> (1)..(7)

<400> 42

```
Ser Gly Ser Ser Ser Gly Gly
1               5
```

<210> 43
<211> 7
<212> PRT
<213> Artificial sequence

<220>
<223> amino acid motif

<220>
<221> PEPTIDE
<222> (1)..(7)

<400> 43

```
Arg Thr Ile Asp Pro Glu Thr
1               5
```

<210> 44
<211> 7
<212> PRT
<213> Artificial sequence

<220>
<223> amino acid motif

<220>
<221> PEPTIDE

<222> (1)..(7)

<400> 44

Arg Asp Ile Asp Pro Asp Thr
1               5

<210> 45
<211> 7
<212> PRT
<213> Artificial sequence

<220>
<223> amino acid motif

<220>
<221> PEPTIDE
<222> (1)..(7)

<400> 45

Arg Gly Thr Asp Pro Glu Thr
1               5

<210> 46
<211> 7
<212> PRT
<213> Artificial sequence

<220>
<223> amino acid motif

<220>
<221> PEPTIDE
<222> (1)..(7)

<400> 46

Arg Gly Ile Asp Pro Glu Thr
1               5

<210> 47
<211> 7
<212> PRT
<213> Artificial sequence

<220>
<223> amino acid motif

<220>
<221> PEPTIDE
<222> (1)..(7)

<400> 47

Asp Ala Leu His Met Met His
1 5

<210> 48
<211> 7
<212> PRT
<213> Artificial sequence

<220>
<223> amino acid motif

<220>
<221> PEPTIDE
<222> (1)..(7)

<400> 48

Asp Ala Ser His Met Met His
1 5

<210> 49
<211> 11
<212> PRT
<213> Artificial sequence

<220>
<223> amino acid motif

<220>
<221> PEPTIDE
<222> (1)..(11)

<400> 49

Val Val Ala Gly Asn Ser Ser Gly Gly Leu Leu
1 5 10

<210> 50
<211> 11
<212> PRT
<213> Artificial sequence

<220>
<223> amino acid motif

<220>
<221> PEPTIDE
<222> (1)..(11)

<400> 50

Ile Val Ala Gly Asn Ser Ser Gly Gly Val Leu
1 5 10

<210> 51
<211> 11
<212> PRT
<213> Artificial sequence

<220>
<223> amino acid motif

<220>
<221> PEPTIDE
<222> (1)..(11)

<400> 51

```
His Ala Arg Thr Ile Asp Pro Glu Thr Gly Glu
1               5                   10
```

<210> 52
<211> 11
<212> PRT
<213> Artificial sequence

<220>
<223> amino acid motif

<220>
<221> PEPTIDE
<222> (1)..(11)

<400> 52

```
His Met Arg Asp Ile Asp Pro Asp Thr Gly His
1               5                   10
```

<210> 53
<211> 11
<212> PRT
<213> Artificial sequence

<220>
<223> amino acid motif

<220>
<221> PEPTIDE
<222> (1)..(11)

<400> 53

```
His Met Arg Gly Thr Asp Pro Glu Thr Gly Asn
1               5                   10
```

<210> 54
<211> 11
<212> PRT
<213> Artificial sequence

<220>
<223> amino acid motif

<220>
<221> PEPTIDE
<222> (1)..(11)

<400> 54

```
His Pro Asp Ala Leu His Met Met His Leu Phe
1               5                   10
```

<210> 55
<211> 11
<212> PRT
<213> Artificial sequence

<220>
<223> amino acid motif

<220>
<221> PEPTIDE
<222> (1)..(11)

<400> 55

```
Val Pro Asp Ala Leu His Met Met His Gln Phe
1               5                   10
```

<210> 56
<211> 11
<212> PRT
<213> Artificial sequence

<220>
<223> amino acid motif

<220>
<221> PEPTIDE
<222> (1)..(11)

<400> 56

```
Val Pro Asp Ala Ser His Met Met His Gln Ser
1               5                   10
```

<210> 57
<211> 21
<212> PRT
<213> Artificial sequence

<220>
<223> amino acid motif

<220>

<221> PEPTIDE
<222> (1)..(21)

<400> 57

Ile Lys Arg Pro Val Val Val Ala Gly Asn Ser Ser Gly Gly Leu Leu
1 5 10 15

Ala Ala Trp Leu Ser
20

<210> 58
<211> 21
<212> PRT
<213> Artificial sequence

<220>
<223> amino acid motif

<220>
<221> PEPTIDE
<222> (1)..(21)

<400> 58

Val Lys Arg Pro Val Ile Val Ala Gly Asn Ser Ser Gly Gly Val Leu
1 5 10 15

Ala Ala Trp Leu Ser
20

<210> 59
<211> 21
<212> PRT
<213> Artificial sequence

<220>
<223> amino acid motif

<220>
<221> PEPTIDE
<222> (1)..(21)

<400> 59

Val Arg Arg Pro Val Val Val Ala Gly Asn Ser Ser Gly Gly Val Leu
1 5 10 15

Ala Ala Trp Leu Ser
20

<210> 60
<211> 21
<212> PRT
<213> Artificial sequence

<220>
<223> amino acid motif

<220>
<221> PEPTIDE
<222> (1)..(21)

<400> 60

```
Val Lys Arg Pro Val Val Val Ala Gly Asn Ser Ser Gly Gly Val Leu
1               5                   10                  15

Ala Ala Trp Leu Ser
            20
```

<210> 61
<211> 21
<212> PRT
<213> Artificial sequence

<220>
<223> amino acid motif

<220>
<221> PEPTIDE
<222> (1)..(21)

<400> 61

```
Ile Leu Ile Thr His His Ala Arg Thr Ile Asp Pro Glu Thr Gly Glu
1               5                   10                  15

Leu Leu Gly Ala Leu

                20
```

<210> 62
<211> 21
<212> PRT
<213> Artificial sequence

<220>
<223> amino acid motif

<220>
<221> PEPTIDE
<222> (1)..(21)

<400> 62

Val Leu Leu Thr His His Met Arg Asp Ile Asp Pro Asp Thr Gly His
1               5                   10                  15

Leu Val Gly Ala Leu
            20

<210> 63
<211> 21
<212> PRT
<213> Artificial sequence

<220>
<223> amino acid motif

<220>
<221> PEPTIDE
<222> (1)..(21)

<400> 63

Val Leu Leu Thr His His Met Arg Gly Thr Asp Pro Glu Thr Gly Asn
1               5                   10                  15

Leu Leu Gly Ala Leu
            20

<210> 64
<211> 21
<212> PRT
<213> Artificial sequence

<220>
<223> amino acid motif

<220>
<221> PEPTIDE
<222> (1)..(21)

<400> 64

Val Leu Leu Thr His His Pro Asp Ala Leu His Met Met His Leu Phe
1               5                   10                  15

Leu Leu Gly Ala Leu
            20

<210> 65
<211> 21
<212> PRT
<213> Artificial sequence

<220>
<223> amino acid motif

<220>
<221> PEPTIDE
<222> (1)..(21)

<400> 65

```
Val Asp Tyr Gln Ser His Pro Asp Ala Leu His Met Met His Leu Phe
1               5                   10                  15

Asp Pro Ala Arg Tyr
            20
```

<210> 66
<211> 21
<212> PRT
<213> Artificial sequence

<220>
<223> amino acid motif

<220>
<221> PEPTIDE
<222> (1)..(21)

<400> 66

```
Val Asp Tyr Ala Ser Val Pro Asp Ala Leu His Met Met His Gln Phe
1               5                   10                  15

Asp Pro Pro Arg Tyr
            20
```

<210> 67
<211> 21
<212> PRT
<213> Artifical sequence

<220>
<221> PEPTIDE
<222> (1)..(21)

<400> 67

```
Val Asp Tyr Glu Ser Val Pro Asp Ala Ser His Met Met His Gln Ser
1               5                   10                  15

Ala Pro Ala Arg Tyr
            20
```

## Beschreibung

Die vorliegende Erfindung bezieht sich auf ein Polypeptid zur hydrolytischen Spaltung von Zearalenon und/oder von Zearalenon-Derivaten, ein isoliertes Polynukleotid, welche ein derartiges Polypeptid codiert, sowie einen Zusatzstoff enthaltend ein derartiges Polypeptid und eine Verwendung desselben, sowie auf ein Verfahren zur hydrolytischen Spaltung von Zearalenon und/oder von Zearalenon-Derivaten.

Mykotoxine sind von filamentösen Pilzen produzierte Sekundärmetabolite. Ein wichtiger Vertreter derselben ist das weltweit verbreitete Zearalenon (ZEN), früher bekannt als F-2 Toxin, das durch eine Vielzahl von *Fusarien*-Pilzen produziert wird. Diese Pilze befallen unter anderem Kulturpflanzen, wie verschiedene Getreidearten, wobei in der Regel der Pilzbefall vor der Ernte auftritt, wobei das Pilzwachstum bzw. die Mykotoxinproduktion vor und/oder bei nicht sachgemäßer Lagerung auch nach der Ernte erfolgen kann. Die Food and Agriculture Organization (FAO) schätzt, dass weltweit 25 % der agrarischen Produkte mit Mykotoxinen kontaminiert sind, was zu erheblichen wirtschaftlichen Einbußen führt. In einer aktuelleren weltweit durchgeführten Studie von I. Rodrigues und K. Naehrer, Toxins, 2012, 4, 663-675 wurden in dem Zeitraum von Jänner 2009 bis Dezember 2011 insgesamt 23.781 Proben analysiert, wobei 81 % positiv auf mindestens ein Mykotoxin und 45 % positiv auf ZEN getestet wurden. ZEN konnte in allen Regionen der Welt ebenso wie in allen getesteten Getreide- und Futtermittelklassen, wie beispielsweise Mais, Sojamehl, Weizen, Weizenkleie, DDGS (Trockenschlempe) sowie in Fertigfuttermischungen mit einer Häufigkeit von bis zu 100 % gefunden werden.

ZEN ist ein nicht steroides, östrogenes, makrozyklisches, über den Polyketid-Stoffwechselweg synthetisiertes Lacton der Strukturformel:

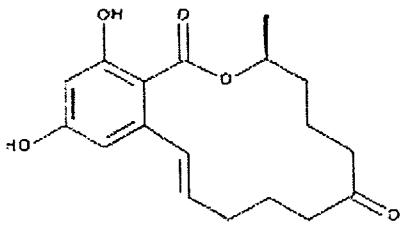

und den IUPAC Nomenklaturnamen (2E,11S)-15,17-dihydroxy-11-methyl-12-oxabicyclo[12.4.0]octadeca-1(18),2,14,16-tetraene-7,13-dion.

In der Natur kommt jedoch auch eine Vielzahl von ZEN-Derivaten vor, die durch enzymatische oder chemische Modifikationen von ZEN entstehen. Beispiele hierfür sind glykosidische oder sulfathältige ZEN-Konjugate, die durch Pilze, Pflanzen oder dem Säugetiermetabolismus gebildet werden, sowie ZEN-Metabolite, die unter anderem im menschlichen oder tierischen Organismus gebildet werden. Im Folgenden werden unter ZEN-Derivaten als in der Natur vorkommende oder durch chemische oder biochemische Synthese hergestellte ZEN-Konjugate oder ZEN-Metabolite, aber im speziellen α-Zearalenol (α-ZEL; (2E,7R,11S)-7,15,17-trihydroxy-11-methyl-12-oxabicyclo [12.4.0] octadeca-1(18),2,14,16-tetraen-13-on), β-Zearalenol (β-ZEL; (2E,7S,11S)-7,15,17-trihydroxy-11-methyl-12-oxabicyclo[12.4.0] octadeca-1(18),2,14,16-tetraen-13-on), α-Zearalanol (α-ZAL; (7R,11S)-7,15,17-trihydroxy-11-methyl-12-oxabicyclo[12.4.0]octadeca1(18),14,16-trien-13-on), β-Zearalanol (β-ZAL; (7S,11S)-7,15,17-trihydroxy-11-methyl-12-oxabicyclo [12.4.0] octadeca-1(14),15,17-trien-13-on), Zearalenon-14-Sulfat (Z14S; [(2E,11S)-15-hydroxy-11-methyl-7,13-dioxo-12-oxabicyclo[12.4.0]octadeca-1(18),2,14,16-tetraen-17-yl] hydrogensulfat), Zearalenon-14-glycosid (Z14G; (2E,11S)-15-hydroxy-11-methyl-17-[(3R,4S,5S,6R)-3,4,5-trihydroxy-6-(hydroxymethyl)tetrahydropyran-2-yl]oxy-12-oxabicyclo[12.4.0]octadeca-1(18)2,14,16-tetraene-7, 13-dion), sowie Zearalanon (ZAN; (11S)-15,17-dihydroxy-11-methyl-12-oxa-bicyclo [12.4.0] octadeca-1(18),14,16-triene-7,13-dion) verstanden.

ZEN ebenso wie ZEN-Derivate, vor allem α-ZEL, β-ZEL, Z14S, α-ZAL, β-ZAL, Z14G und ZAN können aufgrund ihrer hohen chemischen und physikalischen Stabilität auch in verarbeiteten Lebens- oder Futtermitteln, wie z.B. Brot oder Bier nachgewiesen werden.

ZEN bindet an den Östrogenrezeptor und kann hormonelle Störungen verursachen, wobei es unmittelbar nach der oralen Aufnahme absorbiert und von Säugetieren in die zwei stereoisomeren Metaboliten α-ZEL bzw. β-ZEL umgewandelt wird. Hierbei weisen z.B. α-ZEL, aber auch α-ZAL bzw. ZAN eine weitaus stärkere östrogene Wirkung auf als ZEN. Konjugierte ZEN-Derivate weisen mitunter eine geringere Östrogenität als ZEN auf, jedoch kann gegebenenfalls aus diesen ZEN-Derivaten im Verdauungstrakt wieder ZEN freigesetzt werden.

Obwohl ZEN eine relativ geringe akute Toxizität aufweist und einen oralen LD50-Wert von bis zu 20.000 mg/kg Körpergewicht besitzt, können bei längerer Einnahme subakute und/oder subchronische toxische Wirkungen, wie beispielsweise teratogene, karzinogene, östrogene und immunsupressive Wirkungen bei Tieren oder Menschen auftreten. Mit ZEN kontaminiertes Futtermittel führt zu Entwicklungsstörungen bei Säugetieren, wobei Schweine, und im speziellen Jungtiere äußerst sensitiv gegenüber ZEN sind. ZEN-Konzentrationen im Futtermittel von über 0,5 ppm führen zu

Entwicklungsstörungen, wobei z.B. Konzentrationen von über 1,5 ppm zu Hyperöstrogenität bei Schweinen führen können und Konzentrationen von 12 ppm ZEN für Fehlgeburten bei Rindern verantwortlich gemacht wurden. Da Zearalenon schnell über die Schleimhäute, insbesondere über die Magen- aber auch über die Mundschleimhaut aufgenommen wird, ist eine unverzügliche und vor allem quantitative Deaktivierung notwendig. Bereits 30 Minuten nach oraler Gabe von ZEN kann dieses im Blut nachgewiesen werden. Hierbei weist der Einsatz isolierter Enzyme gegenüber Mikroorganismen Vorteile, wie eine höhere spezifische Aktivität oder eine schnellere Wirkung auf. Aufgrund der aufgezeigten schädlichen Wirkung von ZEN gibt es in der Europäischen Union verbindliche ZEN-Obergrenzen in Lebensmitteln sowie Empfehlungen für ZEN-Obergrenzen in Futtermitteln (EC NO: 1881/2006).

Die primäre Strategie, um die ZEN-Kontamination von Lebensmitteln oder Futtermitteln zu reduzieren, ist die Einschränkung des Pilzwachstums, beispielsweise durch Einhaltung der "guten landwirtschaftlichen Praxis". Dazu zählt unter anderem, dass Saatgut frei von Schädlingen und Pilzbefall ist, oder dass landwirtschaftliche Abfallprodukte rechtzeitig vom Feld entfernt werden. Des Weiteren kann durch den Einsatz von Fungiziden das Pilzwachstum auf dem Feld reduziert werden. Nach der Ernte sollte das Erntegut bei einer Restfeuchtigkeit von unter 15 % und geringer Temperatur gelagert werden, um das Pilzwachstum zu verhindern. Ebenso sollte vor der Weiterverarbeitung durch Pilzbefall kontaminiertes Gut entfernt werden. Trotz dieser Liste von Maßnahmen berichteten I. Rodriges und K. Naehrer (2012), dass selbst in Regionen mit den höchsten landwirtschaftlichen Standards, wie den USA und Zentraleuropa in den Jahren 2009 bis 2011 29 % bzw. 39 % der getesteten Maisproben mit ZEN kontaminiert waren.

Weitere Möglichkeiten zur Entfernung von ZEN aus Futter- oder Lebensmitteln sind die Adsorption bzw. die Transformation des Mykotoxins. Hierfür ist es erforderlich, dass die Bindung des Mykotoxins zum Adsorbens stark und spezifisch über einen weiten pH-Bereich ist und während des gesamten Verdauungsprozesses im gastrointestinalen Bereich stabil bleibt. Obwohl einige nicht-biologische Adsorptionsmittel, wie z.B. Aktivkohle, Silikate oder synthetische Polymere, wie Cholestryamin, für Aflatoxine effizient eingesetzt werden können, ist ihr Einsatz für andere Mykotoxine beschränkt. Der wesentliche Nachteil der Adsorptionsmittel ist die nicht-spezifische Bindung von anderen Molekülen, die teilweise essentiell für die Ernährung sind. Biologische Adsorptionsmittel, wie z.B. Hefe oder Hefeextrakte sind in der Literatur ebenfalls beschrieben, haben jedoch eine ähnliche Limitierung wie nicht-biologische Adsorptionsmittel.

Auch die Detoxifikation von ZEN durch physikalische und chemische Behandlungen ist limitiert. Durch thermische Behandlung kann ZEN nicht effektiv deaktiviert werden, allerdings kann der ZEN-Gehalt durch Extrusion und Behandlung mit Oxidationsmitteln, z.B. für 16 h bei 80 °C mit 10 %iger Wasserstoffperoxidlösung um 83,9 % reduziert werden. Der Einsatz von Extrusionsverfahren und Oxidationsmitteln, wie Ozon oder Wasserstoffperoxid in der Futter- und Lebensmittelherstellung ist auf Grund der hohen Kosten, dem Qualitätsverlust, der teilweise geringen Effizienz und der geringen Spezifität begrenzt.

Auch die Biotransformation von ZEN mittels Mikroorganismen, wie z.B. *Trichosporon mycotoxinivorans*, *Gliocladium roseum* oder *Bacillus subtilis* Stämmen bzw. daraus isolierten Enzymen, wie Hydrolasen oder Peroxidasen ist z.B. in E. Vekiru et al., in Appl. and Environ. Microb., 2010, 76, 7, 2353-2359 beschrieben worden.

Aus der EP 0 938 575 B1 sind ZEN abbauende Eigenschaften von Bakterien der Gattung *Rhodococcus* und *Nocardia*, insbesondere *R. globerulus, R. erythropolis* und *N. globerula* bekannt geworden.

Der WO 02/076205 ist die ZEN abbauende Wirkung von aus *Gliocladium roseum* isolierten Enzymen, unter anderem der $\alpha/\beta$-Hydrolase, Zearalenonhydrolase 1 (ZHD1), entnehmbar, welche den Abbau von ZEN mittels einer katalytischen Triade katalysieren.

Aus der WO 2012/113827 sind rekombinante Zonasen, nämlich ZEN abbauende Enzyme zu entnehmen, die im Magen-Darm-Trakt stabil bleiben, insbesondere sind darin Mikroorganismen, wie *Thermobifidia fusca*, *Streptomyces exfoliatus*, *Acidovorans delafieldii* und *Streptomyces sp.* beschrieben.

Polypeptide oder Enzyme die in der Lage sind ZEN und/oder wenigstens ein ZEN Derivat zu hydrolysieren können auch als Zonasen bezeichnet werden.

Die im Nachfolgenden verwendeten Ausdrücke sind der Fachsprache entnommen und werden jeweils, außer es ist etwas anderes angeführt, in den herkömmlichen Bedeutungen verwendet. So bezieht sich der Ausdruck "Polynukleotid" auf jegliche Arten von genetischem Material aller Längen und Sequenzen, wie z.B. Einzelstrang und Doppelstrang DNS- und RNS- Moleküle, inklusive regulatorischer Elemente, Strukturelemente, Gruppen von Genen, Plasmiden, gesamte Genome und Fragmente davon. Die Bezeichnung "Polypeptid" umfasst Proteine, wie beispielsweise Enzyme, Antikörper sowie Polypeptide mit bis zu 500 Aminosäuren, wie beispielsweise Peptidinhibitoren, Domänen von Proteinen aber auch kurze Polypeptide mit geringen Sequenzlängen, z.B. weniger als 10 Aminosäuren, wie Rezeptoren, Liganden, Peptidhormone, Tags und dgl. Die Bezeichnung "Position" in einem Polynukleotid oder Polypeptid bezieht sich auf eine einzelne, spezifische Base oder Aminosäure in der Sequenz des Polynukleotids oder des Polypeptids.

Die vorliegende Erfindung zielt nun darauf ab, ein Polypeptid zur Verfügung zu stellen, mit dem es gelingt, ZEN und/oder ZEN-Derivate schnell und zuverlässig in hydrolysiertes ZEN und/oder in hydrolysierte ZEN-Derivate zu transformieren.

Zur Lösung dieser Aufgabe ist die vorliegende Erfindung im Wesentlichen dadurch gekennzeichnet, dass das Polypeptid eine Aminosäuresequenz aufweist, welche einen Grad an Sequenzidentität zu der Aminosäuresequenz mit

einer Sequenz ID-Nr. 1 von wenigstens 70 %, bevorzugt wenigstens 84 %, insbesondere bevorzugt wenigstens 92 % und am bevorzugtesten wenigstens 98 % aufweist, oder ein funktionelles Fragment davon enthält.

Die Bezeichnung "Sequenzidentität" entsprechend der vorliegenden Erfindung bezieht sich auf eine prozentuale Sequenzidentität. Für Aminosäuresequenzen und Nukleotidsequenzen kann die Sequenzidentität visuell bestimmt werden, bevorzugt aber mit einem Computerprogramm errechnet werden. Als Referenzsequenz wird die Aminosäuresequenz mit der SEQ ID-Nr. 1 definiert. Der Sequenzvergleich wird auch innerhalb von Sequenzabschnitten durchgeführt, wobei als Abschnitt eine durchgängige Sequenz der Referenzsequenz zu verstehen ist und bevorzugt eine konservierte Region der Sequenz umfasst.

Im vorliegenden Fall wurde die Sequenzidentität mit Hilfe des Programms NCBI BLAST (Basic Local Alignment Search Tool), insbesondere mit BLASTP für Polypeptide und BLASTN für Polynukleotide, welche auf der Homepage des "National Center for Biotechnology Information" (NCBI; http://www.ncbi.nlm.nih.gov/) zur Verfügung gestellt werden, durchgeführt. Damit ist es möglich zwei oder mehrere Sequenzen miteinander nach dem Algorithmus von Altschul et al., 1997 (Nucleic Acids Res., 25:3389-3402) zu vergleichen. Hierbei wurden die Programme in der Version vom 15. Mai 2013 verwendet. Als Programmeinstellungen wurden die Basiseinstellungen herangezogen, insbesondere aber für den Aminosäuresequenzvergleich: "max target sequence" = 100; "expected treshold" = 10; "word size" = 3; "matrix" = BLOSOM62; "gap costs" = "Existence: 11; Extention: 1"; "computational adjustment" = "Conditional compositional score matrix adjustment "; sowie für den Nukleotidsequenzvergleich Word Size: 11; Expect value: 10; Gap costs: Existence = 5 , Extension = 2; Filter = low complexity activated; Match/Mismatch Scores: 2,-3; Filter String: L; m.

Die Ausdrücke "Polypeptidvariante" oder "Variante" beziehen sich zum einen auf "allelische Varianten" des Polypeptids und zum anderen auf "Modifikation" des parentalen Polypeptids, wobei die enzymatische Funktion im Wesentlichen unverändert ist. Die Bezeichnung "allelische Variante" bezieht sich auf ein Polypeptid, das durch in der Natur zufällig vorkommende Mutation(en) der Nukleotidsequenz entstanden ist und eine Veränderung der Aminosäuresequenz bewirkt, wobei die enzymatische Funktion davon nicht beeinflusst ist. "Modifikationen" können beispielhaft C- oder N-terminale Fusionen mit Polypeptiden oder mutierte Polypeptide sein, wobei Mutationen durch Substitution, Insertion oder Deletion von wenigstens einer Aminosäure, insbesondere durch ortsspezifische Mutagenese bzw. zufällige Mutagenese, Rekombination und/oder irgendeiner anderen proteinengineering Methode erhalten werden können. Die Ausdrücke Substitution, Insertion und Deletion sind in der in der Gentechnik üblichen und dem Fachmann geläufigen Bedeutung verwendet.

Der hier verwendete Ausdruck "funktionelles Fragment" bezieht sich auf einen Teil bzw. eine Teilsequenz eines Polypeptids oder einen Teil bzw. eine Teilsequenz einer Variante davon, wobei die enzymatische Funktion im Wesentlichen beibehalten wird.

Durch die Wahl einer derartigen Aminosäuresequenz, eines funktionellen Fragments davon, wurde eine überraschend schnelle und vollständige Hydrolyse von ZEN und/oder von ZEN-Derivaten festgestellt, wobei besonders hohe Aktivitätswerte im Vergleich zu bis dato bekannten ZEN abbauenden Polypeptiden festgestellt werden konnten.

Gleichbleibend gute Ergebnisse konnten erzielt werden, wenn, wie dies einer Weiterbildung der Erfindung entspricht, die Polypeptidvariante wenigstens eine Aminosäuremodifikation gewählt aus der Gruppe Substitution, Deletion und Insertion von einer oder mehreren Aminosäuren aufweist.

Indem die Erfindung so weitergebildet ist, dass das Polypeptid eine spezifische Aktivität von wenigstens 0,01 U/mg, bevorzugt wenigstens 0,1 U/mg, insbesondere wenigstens 1 U/mg aufweist; und/oder einen $K_M$-Wert der hydrolytischen Spaltung von ZEN von höchstens 50 $\mu$M, bevorzugt höchstens 3,5 $\mu$M, insbesondere höchstens 0,5 $\mu$M aufweist; und/ oder einen $k_{cat}$-Wert der hydrolytischen Spaltung von ZEN von wenigstens 0,05 $s^{-1}$, bevorzugt wenigstens 0,6 $s^{-1}$, insbesondere wenigstens 5 $s^{-1}$ besitzt; und/oder einen $v_{max}$-Wert der hydrolytischen Spaltung von ZEN von wenigstens 0,00001 $\mu M^{-1}$ $s^{-1}$, bevorzugt wenigstens 0,0001 $\mu M^{-1}$ $s^{-1}$, insbesondere wenigstens 0,001 $\mu M^{-1}$ $s^{-1}$ besitzt, können ZEN und/oder ZEN-Derivate besonders rasch und vollständig hydrolysiert, insbesondere detoxifiziert werden.

Des Weiteren kann das Polypeptid so ausgewählt sein, dass es eine zur sauerstoffunabhängigen und cofaktorfreien hydrolytischen Spaltung der Estergruppierung des Zearalenons und/oder der ZEN-Derivate geeignete $\alpha/\beta$-Hydrolase ist, dass es eine die hydrolytische Spaltung katalysierende Aminosäuren-Triade, bestehend aus Serin, einer sauren Aminosäure, gewählt aus Glutaminsäure und Asparaginsäure, insbesondere Asparaginsäure sowie Histidin aufweist und dass die katalytische Triade S128, D264 und H303 ist, wobei die Positionierung relativ zur Sequenz mit der SEQ ID NO: 1 dargestellt ist.

Die Hydrolyse von ZEN und/oder von ZEN-Derivaten gelingt mit jedem der Polypeptide der Sequenz ID-Nrn. 1 bis 14 an der Estergruppe des Zearalenons oder seiner Derivate nach dem folgenden Reaktionsmechanismus:

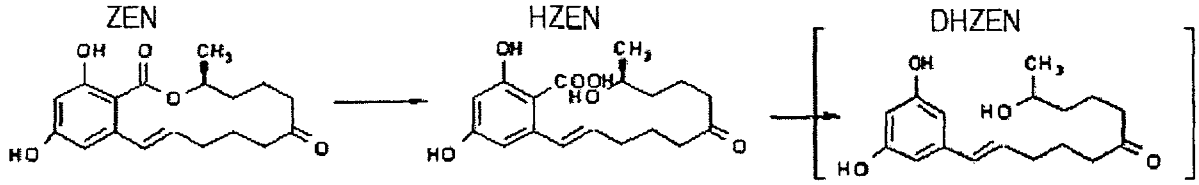

Die Hydrolyse von ZEN zu ungiftigem hydrolysiertem Zearalenon (HZEN) bzw. hydrolysierten ZEN-Derivaten erfolgt durch die erfindungsgemäßen Polypeptide, insbesondere die α/β-Hydroalsen. Die weitere Decarboxylierung von HZEN zu decarboxyliertem, hydrolysiertem ZEN (DHZEN) bzw. decarboxylierten, hydrolysierten ZEN-Derivaten erfolgt in der Regel spontan.

Insbesondere gelingt es mittels der oben genannten katalytischen Triade ZEN und/oder ZEN-Derivate vollständig zu hydrolysieren, wobei die Abbaureaktion eine gute pH-Stabilität, insbesondere pH-Werte im sauren Bereich aufweist.

Überraschenderweise hat sich herausgestellt, dass es gelingt, mit einem Polypeptid, das in einem aus 3 Aminosäuren vor und 3 Aminosäuren nach dem Serin der oben genannten katalytischen Triade bestehenden Sequenzabschnitt, wenigstens eine polare Aminosäure, gewählt aus Y, Q, N, T, K, R, E, D und wenigstens eine nicht polare Aminosäure, gewählt aus F, M, L, I, V, A, G, P enthält, gleichbleibend gute Ergebnisse zu erzielen und überdies wenigstens einen enzymkinetischen Parameter zu verbessern.

In einer bevorzugten Weiterbildung der Erfindung weist das Polypeptid wenigstens eine Mutation der Aminosäuresequenz in Bezug auf Sequenz ID-Nr. 1 an wenigstens einer der folgenden Positionen: 22, 23, 25, 26, 27, 29, 31, 32, 35, 37, 42, 43, 46, 51, 53, 54, 57, 60, 69, 72, 73, 78, 80, 84, 88, 95, 97, 99, 114, 118, 119, 123, 132, 141, 146, 148, 149, 154, 163, 164, 165, 169, 170, 172, 176, 180, 182, 183, 190, 191, 194, 196, 197, 198, 201, 204, 205, 206, 207, 208, 209, 210, 212, 213, 214, 216, 217, 220, 221, 222, 229, 231, 233, 238, 240, 244, 245, 246, 248, 249, 251, 254, 256, 260, 262, 263, 266, 269, 271, 277, 280, 281, 282, 283, 284, 285, 286, 287, 292, 296, 298, 302, 307, 308, 309, 311, 314, 317, 319, 321, 323, 325 und 326 auf. Diese Positionen ergeben sich aus den Sequenzunterschieden des Polypeptids mit der Sequenz ID-Nr. 1 und den zu dieser Sequenz einen hohen Grad an Identität aufweisenden und besonders aktiven Polypeptiden mit der Sequenz ID-Nr. 2 bis 6. Indem das Polypeptid mit der Sequenz ID-Nr. 1 an wenigstens einer dieser Positionen so verändert wird, dass die Aminosäurevarianten der Sequenz ID Nrn. 2 bis 6 an dieser Position übernommen werden, gelingt es zu zeigen, dass diese Positionen einen bedeutenden Einfluss auf die enzymkinetischen Parameter des Polypeptids besitzen und dass des Weiteren Kombinationen der Sequenz mit der Sequenz ID-Nr. 1 mit den einen hohen Grad an Sequenzidentität aufweisenden Sequenz ID-Nrn. 2 bis 6 zu höheren Aktivitäten führen.

Gemäß einer Weiterbildung der Erfindung weist das Polypeptid wenigstens eine Mutation gewählt aus der Gruppe: D22A, S23Q, S23L, N25D, I26V, F27Y, F27H, S29P, R31A, F32Y, R35K, R35Q, V37A, V42I, V43T, F46Y, S51E, S51D, D53G, N54M, N54R, L57V, L60I, S69G, P72E, V73A, A78S, N80H, F84Y, I88L, T95S, T97A, R99K, I114M, I118V, K119R, V123I, L132V, A141S, I146V, I146L, A148G, A149V, A154P, P163T, A164T, Y165C, Y165H, V169I, L170R, A172G, A176M, A176V, Y180F, D182T, F183Y, I190V, G191S, K194T, K194E, F196Y, V197C, V197R, E198R, E198S, K201D, K201G, P204S, P204A, A205S, K206P, A207M, M208A, Q209R, L210A, L210S, ΔP212, T213V, P214A, E216T, E216G, A217I, N220H, L221M, K222R, K222Q, G229A, A231V, F233W, F233Y, F233H, A238G, H240N, H240S, D244E, R245Q, M246L, S248T, S248N, S248G, Q249R, K251N, I254V, I256L, A260M, T262D, T262G, I263T, E266D, E269H, E269N, L271V, L277E, E280A, E280L, H281R, H281Q, A282V, Q283R, D284L, D284R, I285L, I286M, R287E, R287D, R292K, R292T, Q296A, Q296E, H298V, L302S, L307Q, F308S, D309A, A311P, A314V, L317F, S319Q, S319P, S319R, S321A, S321T, T323A, P325A, A326P in der Aminosäuresequenz in Bezug auf Sequenz ID-Nr. 1 auf. Mit einem derartigen Polypeptid gelingt es ZEN innerhalb kurzer Zeit vollständig zu hydrolysieren, insbesondere zu detoxifizieren, wobei die spezifische Aktivität des Polypeptides wenigstens 6,00 U/mg, bevorzugt wenigstens 7,00 U/mg, insbesondere wenigstens 8,00 U/mg beträgt. Die Einheit "U" oder auch "Unit" ist ein Maß für die absolute katalytische Aktivität und wird definiert durch die Hydrolyse von 1 μMol ZEN pro Minute bei 32 °C in 50 mM Tris-HCl Puffer (pH 8,2), wobei - als "katalytische Aktivität" die enzymatische Umsetzung eines Substrats unter definierten Reaktionsbedingungen verstanden wird und unter "spezifischer Aktivität" das Verhältnis der katalytischen Aktivität und der Polypeptid-Massenkonzentration (Masse pro Volumseinheit) verstanden wird.

Indem das Polypeptid so ausgebildet ist, dass wenigstens eines der folgenden Aminosäuremotive mit der Sequenz ID-Nr. 30 bis 48 enthalten ist, gelingt es Polypeptide zur Verfügung zu stellen, die eine spezifische Aktivität von wenigstens 7,00 U/mg, bevorzugt wenigstens 8,00 U/mg aufweisen. Überraschenderweise hat sich gezeigt, dass, wenn wenigstens eines der folgenden Aminosäuremotive mit der Sequenz ID-Nr. 49 bis 56 enthalten ist, die enzymatische Aktivität des Polypeptids, beispielsweise gegenüber einem Motiv enthaltend 7 Aminosäuren noch weiter gesteigert wird. Eine noch höhere spezifische Aktivität wird erreicht, wenn wenigstens eines der folgenden Aminosäuremotive mit der Sequenz mit der Sequenz ID-Nr. 57 bis 67 enthalten ist.

Gemäß einer Weiterbildung der Erfindung enthält das Polypeptid eine Aminosäuresequenz der Sequenz ID-Nr. 1 oder wenigstens ein funktionelles Fragment davon. Dieses Polypeptid zeigt nicht nur eine hohe spezifische Aktivität bei der Hydrolyse, insbesondere der Detoxifikation von ZEN und/oder von ZEN-Derivaten, sondern sind überdies in der Lage eine Aktivität in einem weiten pH-Wertbereich, wie z.B. pH 4 bis 9 und Temperaturbereich zwischen 15 und 65 °C aufrecht zu halten.

Gemäß einer Weiterbildung der Erfindung enthält das Polypeptid wenigstens eine konservative Aminosäuresubstitution an wenigstens einer Position enthält, und die konservative Aminosäuresubstitution ist aus Substitutionen von G zu A; oder A zu G, S; oder V zu I, L, A, T, S; oder I zu V, L, M; oder L zu I, M, V; oder M zu L, I, V; oder P zu A, S, N; oder F zu Y, W, H; oder Y zu F, W, H; oder W zu Y, F, H; oder R zu K, E, D; oder K zu R, E, D; oder H zu Q, N, S; oder D zu N, E, K, R, Q; oder E zu Q, D, K, R, N; oder S zu T, A; oder T zu S, V, A; oder C zu S, T, A; oder N zu D, Q, H, S; oder Q zu E, N, H, K, R gewählt, wobei sich die Bezeichnung konservative Aminosäuresubstitution auf die Substitution von Aminosäuren durch andere Aminosäuren, die vom Fachmann als konservativ angesehen werden, das heißt die ähnliche spezifische Eigenschaften besitzen, bezieht. Solche spezifische Eigenschaften sind beispielsweise deren Größe, Polarität, Hydrophobizität, Ladung oder der pKs-Wert der Aminosäure. Unter einer konservativen Mutation wird daher z.B. eine Substitution einer sauren Aminosäure gegen eine andere saure Aminosäure, eine basische Aminosäure gegen eine andere basische Aminosäure, oder eine polare gegen eine andere polare Aminosäure verstanden.

Mit derartigen konservativen Aminosäuresubstitutionen gelingt es Polypeptidvarianten herzustellen, deren spezifische Aktivität im Verglich zum parentalen Polypeptid in etwa gleich stark ist, bevorzugt jedoch um wenigstens 0,1 U/mg gesteigert werden kann.

Die vorliegende Erfindung zielt des Weiteren darauf ab, ein isoliertes Polynukleotid zur Verfügung zu stellen, mit dem es gelingt ein Polypeptid zur schnellen und zuverlässigen hydrolytischen Spaltung von ZEN und/oder von ZEN-Derivaten herzustellen.

Zur Lösung dieser Aufgabe ist die Erfindung dadurch gekennzeichnet, dass die Nukleotidsequenz für ein Polypeptid, das ein Zearalenon und/oder Zearalenon-Derivate hydrolysierende Eigenschaft besitzt, codiert und/oder einen Grad an Sequenzidentität zu wenigstens der aus den Nukleotidsequenzen der Sequenz ID-Nrn. 15 oder 16 gewählten Gruppe Pvon mindestens 70 % aufweist.

Zu exprimierende Nukleotidsequenzen, insbesondere deren Triplets (Codons) werden in der Regel je nach Wirtszelle verändert, so dass der Codon Bias je nach Wirtszelle optimiert wird. Das resultiert darin, dass auch Polynukleotide mit einem Grad an Sequenzidentität von weit unter 80 %, aber auch unter 70 % oder unter 60 % ein und dasselbe Polypeptid codieren können. Der Sequenzvergleich zur Bestimmung des Grads an Sequenzidentität muss auch innerhalb von Sequenzabschnitten durchgeführt werden, wobei ein Abschnitt als durchgängige Sequenz der Referenzsequenz zu verstehen ist. Die Länge der Sequenzabschnitte beträgt für Nukleotidsequenzen normalerweise 15 bis 600.

Mit Hilfe der genannten Nukleotidsequenzen oder Sequenzabschnitte gelingt es, Nukleinsäuresonden, die eine Länge von in der Regel wenigstens 15, 30, oder 40 Nukleotiden besitzen, zu generieren. Mit Hilfe solcher Sonden, die typischerweise zusätzlich markiert werden, z.B. mittels $^{3}H$, $^{32}P$, $^{35}S$, Biotin oder Avidin, können unter Anwendung von Standardmethoden Nukleotidsequenzen, die für Polypeptide mit ZEN und/oder ZEN-Derivate abbauender Wirkung codieren, identifiziert werden. Als Ausgangsmaterial für die Identifikation solcher Sequenzen können z.B. DNA, RNA oder cDNA von einzelnen Mikroorganismen, genomische DNA-Bibliotheken oder cDNA-Bibliotheken herangezogen werden.

Für Nukleotidsequenzen bzw. Nukleotidsonden mit einer Länge von wenigstens 100 Nukleotiden werden mittlere Stringenzbedingungen definiert als Vorhybridisierung und Hybridisierung bei 42°C in 5-fach NaCl versetzten Na-EDTA Puffer (SSPE, 0,9 M NaCl, 60 mM $NaH_2PO_4$, 6 mM EDTA) enthaltend 0,3 % Natriumdodecylsulfat (SDS), 200 μg/ml gescherte und denaturierte Lachsspermien-DNA und 35 % Formamid, gefolgt von Standard Southern Blot Bedingungen (J. Sambrook, E.F. Fritsch und T. Maniatis, 1989, Molecular Cloning, A Laboratory Manual, 2nd edition, Cold Spring Harbor, New York), wobei das Trägermaterial am Ende dreimal für 15 Minuten mit 2-fach Natriumchlorid-Zitratpuffer (SSC, 300 mM NaCl und 30 mM Trinatriumzitrat, 0,2 % SDS) bei 55°C gewaschen wird.

Für Nukleotidsequenzen bzw. Nukleotidsonden mit einer Länge von 15 Nukleotiden bis 100 Nukleotiden werden mittlere Stringenzbedingungen definiert als Vorhybridisierung und Hybridisierung im Puffer bestehend aus 0,9 M NaCl, 0,09 M Tris-HCl pH = 7,6, 6 mM EDTA, 0,5% NP-40, 1-fach Denhardts Lösung, 1 mM Natriumpyrophosphat, 1 mM Natriumdihydrogenphosphat, 0,1 mM ATP und 0,2 mg/ml Hefe RNA, wobei bei einer Temperatur die 5°C bis 10°C unter der errechneten Schmelztemperatur (Tm) liegt, vorhybridisiert und hybridisiert wird, wobei Tm durch Berechnung nach Bolton und McCarthy (1962, Proceedings of the National Academy of Sciences USA 48:1390) bestimmt wird. Im Anschluss wird der Versuch unter Standard Southern Blot Bedingungen weitergeführt (J. Sambrook, E.F. Fritsch und T. Maniatis, 1989, Molecular Cloning, A Laboratory Manual, 2nd edition, Cold Spring Harbor, New York). Das Trägermaterial wird am Ende einmal für 15 Minuten mit 6-fach SCC Puffer enthaltend 0,1% SDS und zweimal für 15 Minuten mit 6-fach SSC Puffer bei jeweils 5°C bis 10°C unter der errechneten Tm gewaschen.

Die vorliegende Erfindung zielt des Weiteren darauf ab, einen Zusatzstoff zur Verfügung zu stellen, mit dem eine schnelle und zuverlässige hydrolytische Spaltung von ZEN und/oder von ZEN-Derivaten in definierter oder komplexer Matrix, wie beispielsweise in Futtermitteln oder Lebensmitteln, gelingt.

Zur Lösung dieser Aufgabe ist ein zur hydrolytischen Spaltung von Zearalenon und/oder von Zearalenon-Derivaten befähigtes Polypeptid gemäß der Erfindung eingesetzt. Mit einem derartigen Zusatzstoff gelingt die biochemische Umwandlung von ZEN und/oder ZEN-Derivaten zu hydrolysiertem ZEN und/oder zu hydrolysierten ZEN-Derivaten. Dieser Zusatzstoff kann beispielsweise auch für die stereoselektive Hydrolyse von ZEN und/oder von ZEN-Derivaten in industriellen Prozessen herangezogen werden.

In einer bevorzugten Weiterbildung der Erfindung ist der Zusatzstoff so ausgebildet, dass zusätzlich wenigstens ein inerter Träger sowie gegebenenfalls weitere Bestandteile, wie Vitamine und/oder Mineralstoffe und/oder Enzyme und/oder weitere Komponenten zur Detoxifizierung von Mykotoxinen enthalten sind. Durch den Einsatz eines derartigen Zusatzstoffs kann beispielsweise in Futter- oder Lebensmitteln sichergestellt werden, dass die gegebenenfalls enthaltenen Mengen an ZEN und/oder an ZEN-Derivaten mit Sicherheit soweit hydrolysiert, insbesondere detoxifiziert werden, dass eine schädliche Wirkung auf den Organismus des dieses Futter- oder Lebensmittel aufnehmenden Subjekts ausbleibt.

Hierbei kann ein erfindungsgemäßes Polypeptid auch in einer Enzympräparation vorliegen, die neben wenigstens einem erfindungsgemäßen Polypeptid zusätzlich wenigstens ein Enzym enthält, das beispielsweise am Abbau von Proteinen beteiligt ist, wie z.B. Proteasen, oder das beim Metabolismus von Stärke oder Faser oder Fett oder Glycogen beteiligt ist, wie z.B. Amylase, Cellulase oder Glucanase ist, sowie beispielsweise Hydrolasen, lipolytische Enzyme, Mannosidasen, Oxidasen, Oxidoreductasen, Phytasen, Xylanasen und/oder Kombinationen davon.

Weitere Einsatzbereiche der Erfindung sind Enzympräparationen, die neben wenigstens einem erfindungsgemäßen Polypeptid zusätzlich wenigstens eine Komponente zur Detoxifizierung von Mykotoxinen enthalten, wie ein Mykotoxin-abbauendes Enzym, wie z.B. Aflatoxin Oxidase, Ergotamin Hydrolasen, Ergotamin Amidasen, Zearalenon Esterasen, Zearalenon Lactonasen, Ochratoxin Amidasen, Fumonisin Carboxylesterasen, Fumonisin Aminotransferasen, Aminopolyol Aminoxidasen, Deoxynivalenol Epoxidhydrolasen; und/oder wenigstens einen Mykotoxin-abbauenden Mikroorganismus, wie *Bacillus subtilis;* und/oder wenigstens eine Mykotoxin-bindende Komponente, beispielweise mikrobielle Zellwände oder anorganische Materialien, wie Bentonite enthalten.

Gemäß einer besonders bevorzugten Weiterbildung der Erfindung ist das Polypeptid im Zusatzstoff in einer Konzentration von höchstens 10.000 U/g, bevorzugt höchstens 1.000 U/g, bevorzugter höchstens 100 U/g und am bevorzugtesten höchstens 10 U/g darin enthalten, wodurch es gelingt, ZEN und/oder ZEN-Derivate schnell und insbesondere bereits vor deren Resorption durch den Körper, eines ein kontaminiertes Futter- oder Lebensmittel verzehrenden Subjekt, insbesondere Mensch oder Nutztier, in nicht bzw. weniger toxische Metabolite, insbesondere HZEN und DHZEN, umzuwandeln.

Gemäß einer Weiterbildung der Erfindung liegt das Polypeptid in verkapselter oder gecoateter Form vor, wobei für das Verkapseln oder Coaten Standardmethoden, wie z.B. in der WO 92/12645 beschrieben, herangezogen werden können. Durch das Verkapseln bzw. Coaten gelingt es, das Polypeptid ohne Veränderung, insbesondere ohne Abbau und Schädigung an seinen Einsatzort zu transportieren, so dass erst nach Auflösung der Schutzhülle, beispielsweise im Verdauungstrakt von Tieren, das Polypeptid zu wirken beginnt, wodurch ein noch gezielterer, rascher und vollständiger Abbau von ZEN und/oder von ZEN-Derivaten auch im sauren, proteasereichen und anaeroben Milieu erzielt werden kann. Des Weiteren gelingt es durch die Verkapselung oder das Coaten auch die Temperaturstabilität der Polypeptide im Zusatzstoff zu erhöhen.

Die vorliegende Erfindung zielt des Weiteren auf eine Verwendung des Zusatzstoffes zur hydrolytischen Spaltung von Zearalenon und/oder von Zearalenon-Derivaten in Futtermitteln, insbesondere für Schweine, Geflügel und Aquakultur; in Nahrungsmitteln; oder in Trockenschlempe ab. Durch die erfindungsgemäße Verwendung des Zusatzstoffes gelingt es das im Nahrungs- oder Futtermittel bzw. in der Trockenschlempe enthaltene ZEN und/oder ZEN-Derivat zu hydrolysieren bzw. zu detoxifizieren, wobei eine derartige Entgiftung bereits bei Polypeptidkonzentrationen von etwa 1 U/g kontaminiertem Futter- oder Nahrungsmittel gelingt.

Die vorliegende Erfindung zielt des Weiteren darauf ab, ein Verfahren zur Verfügung zu stellen, mit dem eine schnelle und zuverlässige hydrolytische Spaltung von ZEN und/oder von ZEN-Derivaten mittels Hydrolyse durch ein Polypeptid ermöglicht wird.

Zur Lösung dieser Aufgabe ist das Verfahren so geführt, dass Zearalenon und/oder wenigstens ein Zearalenon Derivat durch ein Polypeptid gemäß der Erfindung und/oder ein Zusatzstoff gemäß der Erfindung hydrolysiert wird.

Gemäß einer bevorzugten Weiterentwicklung ist das Verfahren so geführt, dass darin das Polypeptid oder der Zusatzstoff mit einem mit Zearalenon und/oder mit wenigstens einem Zearalenon-Derivat kontaminierten Futter- oder Nahrungsmittel versetzt wird, dass das kontaminierte Futter- oder Nahrungsmittelmit Feuchtigkeit in Kontakt gebracht wird, und dass das Polypeptid oder der Zusatzstoff das im kontaminierten Futter- oder Nahrungsmittel enthaltende Zearalenon und/oder wenigstens ein Zearalenon-Derivat im Verdauungstrakt hydrolysiert. Bei feuchten Futter- oder Nahrungsmitteln, wie Maische oder Breien, wird die Hydrolyse des Zearalenons und/oder des wenigstens einen Zearalenon-Derivats vor der oralen Aufnahme im feuchten Futter- oder Nahrungsmittel stattfinden. Durch dieses Verfahren kann sichergestellt werden, dass die schädigenden Wirkungen von Zearalenon und von Zearalenon-Derivaten auf Mensch und Tier weitestgehend eliminiert werden. Als Feuchtigkeit wird hierbei die Anwesenheit von Wasser oder

wasserhältigen Flüssigkeiten verstanden, wobei darunter auch z.B. Speichel oder andere im Verdauungstrakt vorhandenen Flüssigkeiten fallen. Als Verdauungstrakt wird die Mundhöhle, die Pharynx (Rachen), die Speiseröhre und der Magen-Darm-Trakt oder Äquivalente davon definiert, wobei unterschiedliche Bezeichnungen bei Tieren vorkommen können bzw. einzelne Bestandteile nicht im Verdauungstrakt von Tieren vorhanden sein können.

Das erfinderische Verfahren kann auch so geführt werden, dass das Futter- oder Nahrungsmittel vor der oralen Aufnahme pelletiert wird.

Gemäß einer Weiterbildung der Erfindung ist das Verfahren so geführt, dass dadurch wenigstens 70 %, bevorzugt wenigstens 80 %, insbesondere wenigstens 90 % des Zearalenons und/oder wenigstens eines Zearalenon-Derivats hydrolysiert werden.

Die Erfindung wird nachfolgend anhand von Ausführungsbeispielen sowie Zeichnungen näher erläutert. In diesen zeigt:

Fig. 1 den zeitlichen Abbau von ZEN und die Zunahme der Metabolite HZEN und DHZEN durch das Polypeptid mit der SEQ ID NO: 1, wobei in Fig. 1A das Polypeptid nicht getagt ist, in Fig. 1B das Polypeptid einen C-terminalen 6xHis Tag und in Fig. 1C einen N-terminalen 6xHis Tag besitzt,

Fig. 2 die Michaelis-Menten Kinetik des Polypeptids mit der SEQ ID NO: 1.

Beispiel 1: Modifikation, Klonierung und Expression von Polynukleotiden, die für Polypeptide mit ZEN und/oder ZEN-Derivate hydrolysierender Wirkung codieren

Aminosäuresubstitutionen, -insertionen oder -deletionen wurden durch Mutation der Nukleotidsequenzen mittels PCR unter Verwendung des "Quick-change Site-directed Mutagenesis Kits" (Stratagene) laut Anleitung durchgeführt. Alternativ dazu wurden auch komplette Nukleotidsequenzen bezogen (GeneArt). Die mittels PCR-Mutagenese generierten bzw. von Gene Art bezogenen Nukleotidsequenzen enthielten auf Aminosäureebene optional zusätzlich einen C- oder N-terminalen 6xHis-Tag und wurden mittels Standardmethoden in Expressionsvektoren zur Expression in *E. coli* oder *P. pastoris* integriert, in *E. coli* oder *P. pastoris* transformiert, sowie in *E. coli* oder *P. pastoris* exprimiert (J.M. Cregg, Pichia Protocols, second Edition, ISBN-10: 1588294293, 2007; J. Sambrook et al. 2012, Molecular Cloning, A Laboratory Manual 4th Edition, Cold Spring Harbor), wobei für diese Aufgabe auch jede andere geeignete Wirtszelle herangezogen werden kann.

Die Bezeichnung "Expressionsvektor" bezieht sich auf ein DNS Konstrukt das in der Lage ist *in vivo* oder *in vitro* ein Gen zu exprimieren. Im Besonderen werden darunter DNS Konstrukte verstanden, die geeignet sind, die das Polypeptid codierende Nukleotidsequenz in die Wirtszelle zu transferieren, um dort ins Genom zu integrieren oder frei im extrachromosomalen Raum vorzuliegen und die das Polypeptid codierende Nukleotidsequenz intrazellulär zu exprimieren und gegebenenfalls das Polypeptid aus der Zelle auszuschleusen.

Die Bezeichnung "Wirtszelle" bezieht sich auf alle Zellen, die entweder eine zu exprimierende Nukleotidsequenz oder einen Expressionsvektor enthalten und in der Lage sind, ein erfindungsgemäßes Enzym bzw. Polypeptid herzustellen. Im Besonderen werden darunter prokaryotische und/oder eukaryotische Zellen verstanden, bevorzugt *P. pastoris, E. coli, Bacillus subtilis, Streptomyces, Hansenula, Trichoderma, Lactobacillus, Aspergillus,* Pflanzenzellen und/oder Sporen von *Bacillus*, *Trichoderma* oder *Aspergillus.*

Das lösliche Zelllysat im Falle von *E. coli* beziehungsweise der Kulturüberstand im Falle von *P. pastoris* wurden zur Bestimmung der katalytischen Eigenschaften der Polypeptide herangezogen. Zur Bestimmung des $K_M$-Werts, von $v_{max}$, $k_{cat}$ und der spezifischen Aktivität wurden die Polypeptide mittels Standardmethoden chromatographisch über Nickel-Sepharosesäulen selektiv angereichert. Die Bestimmung der Proteinkonzentration erfolgte mittels Standardmethoden, entweder mit der BCA Methode (Pierce BCA Protein Assay KitProd # 23225), bevorzugt jedoch photometrisch mit den spezifischen Extinktionskoeffizienten für die jeweiligen Proteine, die mit dem Programm "ProtParam", online verfügbar auf http://web.expasy.org/protparam (Gasteiger E. et al.; Protein Identification and Analysis Tools on the ExPASy Server; (ln) John M. Walker (ed): The Proteomics Protocols Handbook, Humana Press, 2005, pp. 571-607) berechnet wurden.

Beispiel 2: Bestimmung der Sequenzidentität sowie der konservierten Regionen

Die Bestimmung der prozentuellen Sequenzidentität über die gesamte Polypeptidlänge der Polypeptide mit den Aminosäuresequenzen mit den Sequenz ID-Nrn. 1 bis 15 relativ zueinander (Tabelle 1) wurde mit Hilfe des Programms BLAST (Basic Local Alignment Search Tool), insbesondere mit BLASTP, welches auf der Homepage des "National Center for Biotechnology Information" (NCBI; http://www.ncbi.nlm.nih.gov/) benutzt werden kann, durchgeführt. Damit ist es möglich zwei oder mehrere Sequenzen miteinander nach dem Algorithmus von Altschul et al., 1997 (Nucleic Acids Res. (1997) 25:3389-3402) zu vergleichen. Als Programmeinstellungen wurden die Basiseinstellungen herangezogen, insbesondere aber: "max target sequence" = 100; "expected treshold" = 10; "word size" = 3; "matrix" = BLOSOM62;

"gap costs" = "Existence: 11; Extention: 1"; "computational adjustment" = "Conditional compositional score matrix adjustment ".

Zur Bestimmung der konservierten Regionen der Polypeptide mit den Sequenz ID-Nrn. 1 bis 6, wurden diese Sequenzen, die eine Sequenzidentität von wenigstens 70 % zueinander besitzen, mit Hilfe der Software COBALT (J.S. Papadopoulos und R. Agarwala, 2007, COBALT: constraint-based alignment tool for multiple protein sequences, Bioinformatics 23:1073-79.) unter Heranziehung der Standardparameter, insbesondere der Parameter ("Gap penalties": -11, -1; "End-Gap Penalties": -5, -1; "Use RPS BLAST": on; "Blast E-value": 0,003; "Find Conserved columns and Recompute": on; "use query clusters": on; "word size": 4; "may cluster distance": 0,8; "Alphabet": regulär; "Homology conversation setting": 3 bits) abgeglichen. Das Resultat dieser Analyse stellt die konservierten Aminosäuren dar. Als konservierte Regionen wurden die folgenden Bereiche von wenigstens 5 aufeinanderfolgenden konservierten Aminosäuren definiert, nämlich in Bezug auf die Sequenz ID-Nr. 1 Region A von Position +24 bis Position +50, B von +52 bis +77, C von +79 bis +87, D von +89 bis +145, E von +150 bis +171, F von +177 bis +193, G von +223 bis +228, H von +230 bis + 237, I von +239 bis +247, J von +249 bis +255, K von +257 bis +261, L von +263 bis +270, M von +272 bis +279, N von +297 bis +301 und O von +303 bis +313.

Die Bestimmung der prozentualen Sequenzidentität der konservierten Regionen der einzelnen Sequenzen relativ zur konservierten Region der Sequenz mit der SEQ ID NO: 1 (Tabelle 1 Spalte 3 und Spalte 4) wurde, wie oben beschrieben, mittels dem Programm COBALT durchgeführt.

Tabelle 1: Prozentuale Sequenzidentität über die gesamte Polypeptidlänge und von den zwei ausgewählten konservierten Regionen D und G.

| | Sequenzidentität relativ zur SEQ ID-Nr. 1 | | |
|---|---|---|---|
| Polypeptid | gesamtes Polypeptid | konservierte Region D | konservierte Region G |
| SEQ ID-Nr. 1 | 100 % | 100 % | 100 % |
| SEQ ID-Nr. 2 | 70% | 87,7 % | 100 % |
| SEQ ID-Nr. 3 | 71 % | 89,5 % | 100 % |
| SEQ ID-Nr. 4 | 71 % | 91,2 % | 100 % |
| SEQ ID-Nr. 5 | 71 % | 87,7 % | 100 % |
| SEQ ID-Nr. 6 | 71 % | 91,2% | 100 % |
| SEQ ID-Nr. 7 | 64 % | 78,9 % | 100 % |
| SEQ ID-Nr. 8 | 57 % | 82,5 % | 100 % |
| SEQ ID-Nr. 9 | 50 % | 73,7 % | 100 % |
| SEQ ID-Nr. 10 | 55 % | 75,4 % | 100 % |
| SEQ ID-Nr. 11 | 53 % | 71,9 % | 100 % |
| SEQ ID-Nr. 12 | 50 % | 71,9 % | 100 % |
| SEQ ID-Nr. 13 | 55 % | 77,2 % | 100 % |
| SEQ ID-Nr. 14 | 73 % | 84,2 % | 0 % |

Beispiel 3: Hydrolyse von ZEN durch Polypeptide in Zelllysaten

Zur Bestimmung ihrer Fähigkeit, ZEN in die nicht bzw. weniger toxischen Metabolite HZEN und DHZEN abzubauen, wurde das Polypeptid mit der SEQ ID NO:1, codiert durch die Nukleotidsequenz mit der SEQ ID NO:15, als solches und mit einem C- bzw. N- terminalen 6xHis tag in *E. coli,* wie in Beispiel 1 beschrieben, hergestellt. Jeweils 100 ml einer *E. coli* Kultur mit einer optischen Dichte (OD600 nm) von 2,0 - 2,5 wurden durch Zentrifugation bei 4 °C geerntet und in 20 ml Brunner Mineralmedium (DSMZ microorganisms medium number 462, 2012) resuspendiert. Die Zellsuspension wurde durch 3-malige Behandlung mit einer Frensh Press bei 20.000 psi lysiert. Das so erhaltene Zelllysat wurde in einer 1:10, einer 1:100 oder einer 1:1.000 Verdünnung, die in Brunner Mineralmedium inklusive 0,1 mg/ml BSA (Rinderserum Albumin) hergestellt wurde, eingesetzt. Für einen ZEN-Abbauversuch wurden 9,9 ml Brunner Mineralmedium inklusive 0,1 mg/ml BSA, 0,1 ml verdünntes Zelllysat und 31 μl ZEN-Substratstammlösung eingesetzt. In Summe wurden die Zelllysate somit 1:1.000, 1:10.000 bzw. 1:100.000 verdünnt. Als ZEN-Substratstammlösung diente eine 2,08 mM ZEN-Lösung (40 Vol.-% ACN + 60 Vol.-% $H_2O$). Zur Herstellung dieser Lösung wurde ZEN in kristalliner Form

(Biopure Standard von Romer Labs, Art. Nr. 001109, Reinheit mind. 98 %) entsprechend eingewogen und aufgelöst. Jeder Abbauansatz wurde in 25 ml Glasfläschchen durchgeführt und bei 25 °C unter Schütteln mit 100 Upm für insgesamt 120 h inkubiert. Zu den Zeitpunkten 0; 0,5; 1; 2; 5; 24; 47; 72 und 120 h wurde jeweils eine Probe von 1 ml entnommen, die Polypeptide wurden für 10 min bei 99 °C hitzeinaktiviert und bei -20 °C gelagert. Nach dem Auftauen der Proben wurden die nicht löslichen Bestandteile durch Zentrifugation abgetrennt. ZEN, HZEN und DHZEN wurden mittels LC/MS/MS analysiert. Dazu wurden die Metabolite mittels einer Phenomenex Luna C18(2) Säule mit den Dimensionen 250 mm x 3 mm und einer Partikelgröße von 5 μm chromatographisch getrennt. Als Laufmittel diente ein Acetonitril-Wassergemisch mit einer Ameisensäurekonzentration von 1 ml/l. Das UV-Signal bei 270 nm wurde aufgezeichnet. Als Ionisierungsquelle diente Elektrospray Ionisation (ESI). ZEN, HZEN und DHZEN wurden mittels QTrap/LC/MS/MS (triple quadrupole, Applied Biosystems) im "enhanced Modus" quantifiziert.

In Fig. 1 ist der zeitliche Abbau von ZEN und die Zunahme von HZEN sowie DHZEN beispielhaft für eine 1:10.000 verdünnte Zelllysatlösung für nicht getaggtes (Fig. 1A) als auch für C-terminal 6xHis getaggtes (Fig. 1B) und N-terminal 6xHis getaggtes (Fig. 1C) Polypeptid mit der SEQ ID NO: 1 zu sehen. Daraus ist klar ersichtlich, dass erstens die Umsetzung von ZEN unmittelbar und vollständig erfolgt, da bereits in der 1. Probe (0 h), die unmittelbar nach dem Versuchsstart gezogen wurden, nahezu kein ZEN mehr detektiert werden konnte und es 2. durch das Anfügen eines Tags, C- oder N- terminal, zu keinen nennenswerten Aktivitätsverlusten kam.

Beispiel 4: Hydrolyse von ZEN-Derivaten durch Polypeptide in Zelllysaten

Zur Bestimmung der Fähigkeit der Polypeptide neben ZEN auch ZEN-Derivate in nicht bzw. weniger toxische Metabolite zu transformieren, wurden die Polypeptide mit der Sequenz ID-Nr. 1 bis 14, wie in Beispiel 3 beschrieben, mit C-terminalem His-Tag, hergestellt und als Zelllysate im Abbauversuch eingesetzt. Zur Herstellung der Polypeptide mit den Sequenz ID-Nrn. 1 bis 14 wurden die jeweiligen synthetischen Nukleotidsequenzen mit den Sequenz ID-Nrn. 16 bis 29 herangezogen.

Die Abbauversuche wurden, wie in Beispiel 3 beschrieben, durchgeführt, wobei jedes Polypeptid mit jedem ZEN-Derivat, α-ZEL, β-ZEL, α-ZAL, β-ZAL, Z14G, Z14S und ZAN, getestet wurde. Die Zelllysate wurden in einer Gesamtverdünnung von 1:10.000 eingesetzt. Als Substratstammlösung wurde anstatt einer 2,08 mM ZEN-Lösung (40 Vol.-% ACN + 60 Vol-.% $H_2O$), äquimolare, d.h. 2,08 mM Lösungen der ZEN-Derivate eingesetzt. α-ZEL, β-ZEL, α-ZAL, β-ZAL und ZAN wurden von Sigma bezogen und als Standards für die Analyse eingesetzt. Z14G und Z14S wurden mit einer Reinheit von wenigstens 90 %, nach den Verfahren wie in P. Krenn et al., 2007 (Mykotoxin Research, 23, 4, 180-184 und M. Sulyok et al., Anal. Bioanal. Chem., 289, 1505-1523) beschrieben, hergestellt und als Standards für die Analyse eingesetzt. Ein weiterer Unterschied zu Beispiel 3 ist, dass lediglich eine Probe, nach 24 h, genommen wurde. Die Reduktion der Konzentration der ZEN-Derivate während des Abbauversuches wurde mittels LC/MS/MS quantifiziert. α-ZEL, β-ZEL, Z14G und Z14S wurden nach der Methode von M. Sulyok et al. (2010, Food Chemistry, 119, 408-416); α-ZAL, β-ZAL und ZAN wurden nach der Methode von P. Songsermaskul et al. (2011, J. of Animal Physiol. and Animal Nutr., 97, 155-161) gemessen. Überraschenderweise hat sich herausgestellt, dass nach 24 h Inkubation, nur noch 0 bis maximal 13 % der Ausgangsmengen der ZEN-Derivate vorhanden waren.

Beispiel 5: Spezifische Aktivität sowie enzymkinetische Parameter der Polypeptide sowie von Varianten davon

Die Bestimmung der spezifischen Aktivität der Polypeptide sowie von Varianten davon erfolgte photometrisch, wobei alle eingesetzten Polypeptide einen C-terminalen 6xHis Tag aufwiesen. Die Herstellung, Anreicherung und Reinigung der Polypeptide bzw. von Varianten davon erfolgte wie in Beispiel 1 beschrieben. Der Abbau von ZEN zu HZEN wurde über die Abnahme der Adsorption bei der Wellenlänge von 315 nm gemessen. Die molaren Extinktionskoeffizienten [ε] von ZEN und HZEN wurden experimentell bestimmt und betragen 0,0078895 L $\mu mol^{-1}$ $cm^{-1}$ und 0,0030857 L $\mu mol^{-1}$ $cm^{-1}$. Die Extinktionskoeffizienten sind stark pH-abhängig und daher muss die Aktivitätsmessung immer bei exakt demselben pH-Wert und vorzugsweise auch in derselben Matrix durchgeführt werden. Die Messungen wurden in einer 50 mM Tris-HCl pH = 8,2 Pufferlösung in Quarzküvetten in einem Wellenlängenbereich von 200 bis 2500 nm in einem UV-VIS Photometer (Hitachi U-2001) bei 32 °C durchgeführt.

Als ZEN-Substratstammlösung diente eine 2,08 mM ZEN-Lösung (40 vol-% ACN + 60 vol-% $H_2O$). Zur Herstellung dieser Lösung wurde ZEN in kristalliner Form (Biopure Standard von Romer Labs, Art. Nr. 001109, Reinheit mind. 98 %) entsprechend eingewogen und aufgelöst. Die ZEN-Substratverdünnungen (0,79 μM, 1,57 μM, 2,36 μM, 3,14 μM, 4,71 μM, 6,28 μM, 7,85 μM, 9,42 μM, 10,99 μM, 12,56 μM, 14,13 μM, 15,71 μM, 17,28 μM, 18,85 μM) wurden mit 50 mM Tris-HCl pH = 8,2 hergestellt. Die Polypeptidlösungen wurden mit 50 mM Tris-HCl Puffer pH = 8,2 auf eine Endkonzentration von ca. 70 ng/ml verdünnt. Die ZEN-Substratverdünnungen wurden im Wasserbad auf 32 °C vorgewärmt.

100 μl der jeweiligen ZEN-Substratverdünnung wurden mit 0,2 μl Polypeptidlösung versetzt und die Absorption wurde für 5 min gemessen, wobei jede Kombination aus Polypeptidlösung - ZEN-Substratverdünnung mindestens zweifach gemessen wurde.

Unter Berücksichtigung der Extinktionskoeffizienten von ZEN und HZEN wurde über die Steigung der Absorption die Reaktionsgeschwindigkeit für jede Substratkonzentration errechnet.

Die Bezeichnungen "$K_M$ - Wert" oder "Michaelis-Menten Konstante" beziehen sich auf einen Parameter zu Beschreibung der enzymatischen Affinität, der die Einheiten [μM] oder [mM] besitzt und mit Hilfe des linearen Hanes Plots nach H. Bisswang (2002, Enzyme Kinetics, ISBN 3-527-30343-X, Seite 19) berechnet wird, wobei hierfür bevorzugt die Funktion "Enzymkinetik, Single Substrat" des Programms SigmaPlot 12.0 verwendet wird. Die Bezeichnungen "katalytische Konstante der Enzymreaktion" oder der "$k_{cat}$-Wert" beziehen sich auf einen Parameter zur Beschreibung der Umsatzrate eines Polypeptids bzw. Enzyms, der in $[s]^{-1}$ angegeben und bevorzugt mit Hilfe der Funktion "Enzymkinetik, Single Substrat" des Programms SigmaPlot 12.0 berechnet wird. Die "maximale Enzymgeschwindigkeit" oder der "$v_{max}$-Wert" wird in den Einheiten [μM/s] oder [mM/s] angegeben und analog zum $K_M$-Wert mit Hilfe des linearen Hanes Plot ermittelt, wobei hierfür bevorzugt die Funktion "Enzymkinetik, Single Substrat" des Programms SigmaPlot 12.0 verwendet wird.

Mittels $v_{max}$ und der eingesetzten Enzymkonzentration wurde die spezifische Aktivität gemäß der Formel

$$\text{spezifische Aktivität [U/mg]} = \frac{\text{vmax [μM/s] x 60 [s/min]}}{\text{Enzymkonzentration [mg/l]}}$$

berechnet, wobei ein Unit als Hydrolyse von 1 μmol ZEN pro Minute bei 32 °C in 50 mM Tris-HCl-Pufferlösung, pH = 8,2 definiert ist.

Nachfolgend sind die Rohdaten für die Bestimmung der Enzymparameter $K_M$, $v_{max}$, $k_{cat}$ sowie der spezifischen Aktivität beispielhaft für das Polypeptid mit der Sequenz ID-Nr. 1 angeführt. Tabelle 2 zeigt die Reaktionsgeschwindigkeiten bei den jeweiligen ZEN-Substratkonzentrationen, Fig. 2 die zugehörigen Michaelis-Menton Graphiken und in Tabelle 3 sind die entsprechenden enzymkinetischen Parameter angegeben. Die eingesetzte Enzymlösung hatte eine Konzentration von 68 ng/l.

Tabelle 2: Reaktionsgeschwindigkeiten des Polypeptids mit der Sequenz ID-Nr. 1 bei unterschiedlichen ZEN-Konzentrationen.

| ZEN-Substratverdünnung [μM] | Messung 1 Reaktionsgeschwindigkeit [μM/s] | Messung 2 Reaktionsgeschwindigkeit [μM/s] |
|---|---|---|
| 0,79 | 0,0073 | 0,0071 |
| 1,57 | 0,0087 | 0,0082 |
| 2,36 | 0,0095 | 0,0080 |
| 3,14 | 0,0101 | 0,0073 |
| 4,71 | 0,0103 | 0,0087 |
| 6,28 | 0,0096 | 0,0088 |
| 7,85 | 0,0084 | 0,0088 |
| 9,42 | 0,0111 | 0,0087 |
| 10,99 | 0,0093 | 0,0081 |
| 12,56 | 0,0100 | 0,0086 |
| 14,13 | 0,0089 | 0,0101 |
| 15,71 | 0,0089 | 0,0090 |
| 17,28 | 0,0100 | 0,0074 |
| 18,85 | 0,0100 | 0,0085 |

Tabelle 3: Enzymkinetische Parameter des Polypeptids mit der Sequenz ID-Nr. 1.

| | $V_{max}$ [μM / s] | | $K_M$ [μM] | | $k_{cat}$ [$s^{-1}$] | | sp. Aktivität [U/mg] | |
|---|---|---|---|---|---|---|---|---|
| Messung | 1 | 2 | 1 | 2 | 1 | 2 | 1 | 2 |
| Wert | 0,00993 | 0,008756 | 0,2172 | 0,1898 | 5,44 | 4,79 | 8,76 | 7,73 |
| Mittelwert | 0,009343 | | 0,2035 | | 5,12 | | 8,25 | |

Die spezifische Aktivität der untersuchten Polypeptide beträgt für Sequenz ID-Nr. 1 8,25 U/mg, für Sequenz ID-Nr. 2 10,56 U/mg, für Sequenz ID-Nr. 8,36 U/mg, für Sequenz ID-Nr. 4 8,33 U/mg, für Sequenz ID-Nr. 5 8,56 U/mg, für Sequenz ID-Nr. 6 9,95 U/mg, für Sequenz ID-Nr. 7 3,83 U/mg, für Sequenz ID-Nr. 8 2,57 U/mg, für Sequenz ID-Nr. 9 4,87 U/mg, für Sequenz ID-Nr. 10 5,12 U/mg, für Sequenz ID-Nr. 11 3,88 U/mg, für Sequenz ID-Nr. 12 2,78 U/mg, für Sequenz ID-Nr. 13 6,43 U/mg, für Sequenz ID-Nr. 14 3,33 U/mg.

Die spezifischen Aktivitäten der untersuchten Polypeptidvarianten sind in Tabelle 4 und Tabelle 5 aufgelistet.

Tabelle 4: Spezifische Aktivität von Varianten des Polypeptids mit der SEQ ID NO: 1, konservierte Bereich(e) in dem die Mutation(en) liegen und Sequenzidentitäten der Varianten zur parentalen Sequenz mit der Sequenz ID-Nr. 1. Die Position der Mutation(en) ist relativ zur Aminosäuresequenz mit der Sequenz ID-Nr. 1 angegeben. Die Sequenzidentität wurde mittels BLAST, wie in Beispiel 2 beschrieben ermittelt.

| Variante | Mutation(en) | Mutation(en) im Bereich | Identität zu Sequenz ID-Nr. 1 | sp. Aktivität [U/mg] |
|---|---|---|---|---|
| ZH1-A-001 | N25D | A | 99,7 % | 8,10 |
| ZH1-A-002 | F27Y | A | 99,7 % | 7,93 |
| ZH1-A-003 | F27H | A | 99,7 % | 7,78 |
| ZH1-A-004 | R35K | A | 99,7 % | 8,98 |
| ZH1-A-005 | R35Q | A | 99,7 % | 8,56 |
| ZH1-A-006 | N25D/S29P/V42I/V43T | A | 98,8 % | 7,84 |
| ZH1-A-007 | I26V/R31A/F32Y/F46Y | A | 98,8 % | 8,61 |
| ZH1-A-S02 | N25D/I26V/F27Y/S29P/R31 A/F32Y/R35K/V37A/V42I/V 43T/F46Y | A | 96,6 % | 8,73 |
| ZH1-A-S03 | N25D/I26V/F27H/S29P/R31 A/F32Y/R35Q/V42I/V43T/F 46Y | A | 97,0 % | 8,52 |
| ZH1-B-001 | D53G | B | 99,7 % | 8,10 |
| ZH1-B-002 | N54M | B | 99,7 % | 8,41 |
| ZH1-B-003 | N54R | B | 99,7 % | 8,33 |
| ZH1-B-004 | S69G | B | 99,7 % | 8,06 |
| ZH1-B-005 | P72E | B | 99,7 % | 8,65 |
| ZH1-B-006 | P72R | B | 99,7 % | 8,78 |
| ZH1-B-S02 | N54M/L57V/L60I/S69G/P72 E/V73A | B | 98,2 % | 8,51 |
| ZH1-B-S03 | D53G/N54R/L57V/L60I/P72 E/V73A | B | 98,2 % | 8,56 |
| ZH1-B-S04 | N54R/L57V/L60I/P72E/V73 A | B | 98,5 % | 8,96 |
| ZH1-B-S14 | N54R/L58V/L59P/L60V/T64 G/P72R/G75P/L77P | B | 97,6 % | 8,68 |
| ZH1-C-001 | N80H | C | 99,7 % | 8,24 |
| ZH1-C-002 | N80D | C | 99,7 % | 8,48 |
| ZH1-C-003 | F84Y | C | 99,7 % | 8,65 |
| ZH1-C-S06 | N80H/F84Y | C | 99,4 % | 8,88 |

(fortgesetzt)

| Variante | Mutation(en) | Mutation(en) im Bereich | Identität zu Sequenz ID-Nr. 1 | sp. Aktivität [U/mg] |
|---|---|---|---|---|
| ZH1-C-S10 | N80H/F84H | C | 99,4 % | 8,32 |
| ZH1-C-S14 | E79R/N80D | C | 99,4 % | 8,45 |
| ZH1-D-001 | T95S | D | 99,7 % | 8,53 |
| ZH1-D-002 | R99K | D | 99,7 % | 8,25 |
| ZH1-D-003 | V123I | D | 99,7 % | 8,17 |
| ZH1-D-004 | A125G | D | 99,7 % | 8,36 |
| ZH1-D-005 | G126A | D | 99,7 % | 8,41 |
| ZH1-D-006 | G130A | D | 99,7 % | 8,69 |
| ZH1-D-007 | G130V | D | 99,7 % | 8,54 |
| ZH1-D-008 | G131A | D | 99,7 % | 8,71 |
| ZH1-D-009 | N127D | D | 99,7 % | 8,29 |
| ZH1-D-010 | N127Q | D | 99,7 % | 8,34 |
| ZH1-D-011 | A141S | D | 99,7 % | 8,67 |
| ZH1-D-012 | F106W | D | 99,7 % | 7,84 |
| ZH1-D-013 | I118V | D | 99,7 % | 8,37 |
| ZH1-D-014 | I118V/V123L | D | 99,4 % | 8,55 |
| ZH1-D-015 | I118V/K119R/L132V | D | 99,1 % | 8,86 |
| ZH1-D-016 | W96Q/F106W/L116G/V122 A | D | 98,8 % | 8,65 |
| ZH1-D-017 | Q91R/N105D/K119G/A141 S/M142K | D | 98,5 % | 8,46 |
| ZH1-D-S02 | T95S/T97A/R99K/I118V/V1 23I/L132V/A141S | D | 97,7 % | 8,66 |
| ZH1-D-S03 | T95S/R99K/1118V/K119R/L 132V/A141S | D | 98,2 % | 9,32 |
| ZH1-D-S04 | T95S/R99K/I118V/L132V/A 141S | D | 98,5 % | 9,15 |
| ZH1-D-S05 | T95S/R99K/I114M/I118V/K 119R/L132V/A141S | D | 97,7 % | 8,84 |
| ZH1-D-S07 | R99G/A115D/K119G/P121 T/V123I/A125S/L132V/L133 V/S138A/Y140F/A141S/M1 42L | D | 96,3 % | 8,79 |
| ZH1-D-S08 | R93K/W96Q/R99G/D104N/ N105L/F106M/A115S/V123I /A125S/G144N | D | 97,0 % | 8,86 |
| ZH1-D-S09 | R99G/S102N/D104N/N105 T/F106W/L110V/V111E/A1 15D/K119G/V122T/V123L/L 132V/L133I/S138A/M142K | D | 95,4 % | 8,99 |
| ZH1-D-S10 | W96R/S102T/F106I/I114L/ | D | 96,0 % | 9,12 |
| | A115S/L116G/K119G/V122 A/V123F/A125S/A134S/Y14 0F/M142E | | | |
| ZH1-D-S11 | W96R/R99G/S102T/F106V/ I114L/A115D/L116G/K119G /V122A/V123F/A125S/N127 L/L133A/A134S/Y140F/M14 2K | D | 95,1 % | 8,54 |

(fortgesetzt)

| Variante | Mutation(en) | Mutation(en) im Bereich | Identität zu Sequenz ID-Nr. 1 | sp. Aktivität [U/mg] |
|---|---|---|---|---|
| ZH1-D-S12 | S94T/R99G/S102T/N105I/L 110V/A115D/K119G/P121E /V122T/V123L/V124I/L133I/ A134G/S138A/Y140F/M142 K | D | 95,1 % | 8,69 |
| ZH1-D-S13 | R93Q/R99G/N105T/R112K/ A115D/L116I/A125S/N127L /L132V/L133V/A134S/Y140 F/M142K | D | 96,0 % | 8,47 |
| ZH1-D-S14 | Q91R/W96R/N105D/I114L/I 118V/K119R/V122A/L132V/ L137S | D | 97,3 % | 8,55 |
| ZH1-E-001 | Y165C | E | 99,7 % | 8,46 |
| ZH1-E-002 | Y165H | E | 99,7 % | 8,33 |
| ZH1-E-003 | P163T | E | 99,7 % | 7,95 |
| ZH1-E-004 | A154P/Y165C | E | 99,4 % | 8,13 |
| ZH1-E-S02 | P163T/A164T/Y165C/V169I /L170R | E | 98,5 % | 8,83 |
| ZH1-E-S05 | A154P/Y165H/L170R | E | 99,1 % | 9,65 |
| ZH1-F-001 | Y180F | F | 99,7 % | 8,35 |
| ZH1-F-002 | D182T | F | 99,7 % | 8,41 |
| ZH1-F-003 | D182K | F | 99,7 % | 8,19 |
| ZH1-F-004 | Y180F/R181V/I190V | F | 99,1 % | 8,56 |
| ZH1-F-S04 | Y180F/D182T/F183Y/I190V /G191S | F | 98,5 % | 8,56 |
| ZH1-F-S06 | Y180F/D182T/F183Y/I190V | F | 98,8 % | 8,64 |
| ZH1-F-S10 | E178A/R181V/D182K/F183 | F | 98,8 % | 7,55 |
| | Y | | | |
| ZH1-H-001 | T236K | H | 99,7 % | 8,09 |
| ZH1-H-002 | V237F | H | 99,7 % | 8,11 |
| ZH1-H-003 | E234G | H | 99,7 % | 8,54 |
| ZH1-H-S02 | F233W | H | 99,7 % | 8,37 |
| ZH1-H-S03 | F233Y | H | 99,7 % | 8,64 |
| ZH1-H-S04 | F233H | H | 99,7 % | 8,36 |
| ZH1-H-S06 | A231V/F233Y | H | 99,4 % | 8,54 |
| ZH1-H-S09 | F232W/F233A/E234T/G235 D/L239A | H | 98,5 % | 8,83 |
| ZH1-I-001 | H240N | I | 99,7 % | 8,54 |
| ZH1-I-002 | H240S | I | 99,7 % | 8,79 |
| ZH1-I-003 | D244E/R245Y | I | 99,4 % | 8,42 |
| ZH1-I-S02 | D244E/R245Q/M246L | I | 99,1 % | 8,36 |
| ZH1-I-S03 | H240N/D244E | I | 99,4 % | 9,26 |
| ZH1-I-S06 | H240S/D244E | I | 99,4 % | 9,02 |
| ZH1-I-S07 | L239Q/H240T/R245Y | I | 99,1 % | 8,41 |

(fortgesetzt)

| Variante | Mutation(en) | Mutation(en) im Bereich | Identität zu Sequenz ID-Nr. 1 | sp. Aktivität [U/mg] |
|---|---|---|---|---|
| ZH1-J-001 | Q249R | J | 99,7 % | 8,36 |
| ZH1-J-002 | T252V | J | 99,7 % | 7,94 |
| ZH1-J-S02 | I254V | J | 99,7 % | 8,55 |
| ZH1-J-S03 | Q249R/K251 N/I254V | J | 99,1 % | 9,03 |
| ZH1-J-S07 | T252V/I254M | J | 99,4 % | 7,81 |
| ZH1-J-S10 | T252V/I254V | J | 99,4 % | 7,97 |
| ZH1-K-S05 | A260M | K | 99,7 % | 8,64 |
| ZH1-K-S11 | A260F | K | 99,7 % | 8,82 |
| ZH1-K-S13 | A260S | K | 99,7 % | 9,01 |
| ZH1-L-001 | E266Y | L | 99,7 % | 8,46 |
| ZH1-L-002 | E266D | L | 99,7 % | 8,31 |
| ZH1-L-003 | T262G | L | 99,7 % | 8,32 |
| ZH1-L-004 | T262D/E266D/ | L | 99,4 % | 8,56 |
| ZH1-L-005 | T262G/I263T/ | L | 99,4 % | 8,68 |
| ZH1-L-S02 | E266D/E269H | L | 99,4 % | 8,59 |
| ZH1-L-S04 | I263T/E269N | L | 99,4 % | 8,73 |
| ZH1-L-S06 | E269N | L | 99,7 % | 8,69 |
| ZH1-L-S13 | E266Y/E269N | L | 99,4 % | 8,33 |
| ZH1-M-001 | L274M | M | 99,7 % | 8,29 |
| ZH1-M-002 | L274C | M | 99,7 % | 8,37 |
| ZH1-M-S02 | L277E | M | 99,7 % | 8,96 |
| ZH1-M-S07 | L274M/A279V | M | 99,4 % | 8,23 |
| ZH1-M-S08 | L274T/L277F | M | 99,4 % | 8,63 |
| ZH1-M-S11 | L274C/L277I | M | 99,4 % | 8,51 |
| ZH1-N-001 | H297L | N | 99,7 % | 8,27 |
| ZH1-N-002 | H298V/L302S | N | 99,4 % | 9,03 |
| ZH1-N-S02 | H298V | N | 99,7 % | 8,94 |
| ZH1-N-S09 | H298L/P299D | N | 99,4 % | 8,37 |
| ZH1-O-001 | L307Q | O | 99,7 % | 8,62 |
| ZH1-O-002 | F308S | O | 99,7 % | 8,57 |
| ZH1-O-S02 | L307Q/A311P | O | 99,4 % | 8,34 |
| ZH1-O-S03 | L307Q/F308S | O | 99,4 % | 8,74 |
| ZH1-O-S06 | L307Q/F308S/D309A | O | 99,1 % | 9,18 |
| ZH1-B/H-001 | D53G/N54R/L57V/L60I/P72 E/V73A/F233V/E234G/V23 7F | B+H | 97,3 % | 9,26 |
| ZH1-C/D-001 | N80H/F84Y/T95S/R99K/I11 8V/K119R/L132V/A141S | C+D | 97,6 % | 9,31 |

(fortgesetzt)

| Variante | Mutation(en) | Mutation(en) im Bereich | Identität zu Sequenz ID-Nr. 1 | sp. Aktivität [U/mg] |
|---|---|---|---|---|
| ZH1-D/K-001 | T95S/T97A/R99K/I118V/V1 23I/L132V/A141S/A260M | D+K | 97,6 % | 9,66 |
| ZH1-D/M-001 | T95S/T97A/R99K/I118V/V1 23I/L132V/A141S/L277E | D+M | 97,6% | 10,63 |
| ZH1-K/N-001 | A260M/H298V | K+N | 99,4 % | 8,94 |
| ZH1-K/L-001 | A260M/T262D/E266D/E269 H | K+L | 98,8 % | 9,03 |
| ZH1-K/L-002 | A260M/T262G/I263T/E269 N | K+L | 98,8 % | 8,84 |
| ZH1-N/O-001 | Q296A/H298V/L307Q/A311 P | N+O | 98,8 % | 9,26 |
| ZH1-N/O-002 | Q296E/H298V/L302S/L307 Q/F308S | N+O | 98,5 % | 9,46 |
| ZH1-C/D/J-001 | N80H/F84Y/T95S/R99K/I11 8V/L132V/A141S/Q249R/K 251 N/I254V | C+D+J | 97,0 % | 9,97 |
| ZH1-B/D/K-001 | D53G/N54R/L57V/L60I/P72 E/V73A/T95S/R99K/I114M/I 118V/K119R/L132V/A141S/ A260M | B+D+K | 95,7 % | 10,78 |
| ZH-J/K/L-001 | I254V/I256L/A260M/T262G/ I263T/E269N | J+K+L | 98,2 % | 9,11 |
| ZH1-J/K/LM-001 | I254V/I256L/A260M/T262D/ E266D/E269H/L271V | J+K+L +M | 97,7 % | 9,14 |
| ZH1-B/C/D/J-002 | E79R/N80D/D53G/N54R/L5 7V/L60I/P72E/V73A/W96R/ R99G/S102T/F106V/I114L/ A115D/L116G/K119G/V122 A/V123F/A125S/N127L/L13 3A/A134S/Y140F/M142K/T 252V/I254V | B+C+D+J | 92,1 % | 11,31 |
| ZH1-DEL-001 | ΔP212 | - | 99,7 % | 8,56 |
| ZH1-DEL-002 | ΔG5/ΔT6/ΔR7/ΔS8/ΔE9/ΔA 10/ΔA11/ΔD12/ΔA13/ΔA14/ ΔT15/ΔQ16/ΔA17/ΔR18/ΔQ 19 | - | 95,4 % | 8,37 |
| ZH1-DEL-003 | ΔN327/ΔD328 | - | 99,4 % | 8,27 |
| ZH1-A/B/C-001 | N25D/I26V/F27Y/S29P/R31 A/F32Y/R35K/V37A/V42I/V 43T/F46Y/ N54R/L58V/L59P/L60V/T64 G/P72R/G75P/L77P/R99G/ S102N/D104N/N105T/F106 W/L110V/V111E/A115D/K1 19G/V122T/V123L/L132V/L 133I/S138A/M142K | A+B+C | 89,6 % | 9,54 |

(fortgesetzt)

| Variante | Mutation(en) | Mutation(en) im Bereich | Identität zu Sequenz ID-Nr. 1 | sp. Aktivität [U/mg] |
|---|---|---|---|---|
| ZH1-DEL/B/C/D/J-001 | ΔG5/ΔT6/ΔR7/ΔS8/ΔE9/ΔA10/ΔA11/ΔD12/ΔA13/ΔA14/ΔT15/ΔQ16/ΔA17/ΔR18/ΔQ19/ΔP212/ΔN327/ΔD328/E79R/N80D/D53G/N54R/L57V/L60I/P72E/V73A/W96R/R99G/S102T/F106V/I114L/A115D/L116G/K119G/V122A/V123F/A125S/N127L/L133A/A134S/Y140F/M142K/T2 52V/I254V | B+C+D+J | 86,6 % | 11,52 |
| ZH1-DEL/A/B/C/D/J-001 | ΔG5/ΔT6/ΔR7/ΔS8/ΔE9/ΔA10/ΔA11/ΔD12/ΔA13/ΔA14/ΔT15/ΔQ16/ΔA17/ΔR18/ΔQ19/ΔP212/ΔN327/ΔD328/N25D/I26V/F27Y/S29P/R31A/F32Y/R35K/V37A/V42I/V43T/F46Y/E79R/N80D/D53G/N54R/L57V/L60I/P72E/V73A/W96R/R99G/S102T/F106V/I114L/A115D/L116G/K119G/V122A/V123F/A125S/N127L/L133A/A134S/Y140F/ M142K/T252V/I254V | A+B+C+D+J | 83,3 % | 10,92 |
| ZH1-001 | L302S | - | 99,7 % | 8,31 |

Tabelle 5: Spezifische Aktivitäten von Varianten des Polypeptids mit der Sequenz ID-Nr. 2. Die Position der Mutation (en) ist relativ zur Aminosäuresequenz mit der Sequenz ID-Nr. 2 angegeben. Die Sequenzidentitäten wurde mittels BLAST, wie in Beispiel 2 beschrieben ermittelt.

| Variante | Mutation(en) | Identität zu Sequenz ID-Nr. 2 | sp. Aktivität [U/mg] |
|---|---|---|---|
| ZH2-001 | D3D(GTRSEAADAATQARQL) | 93,6 % | 10,15 |
| ZH2-002 | D8N/V9I/Y10F | 99,0 % | 10,42 |
| ZH2-003 | M37N/E55P/A56V/V101I/S124A/F194FP/T146P/T147A/C148Y | 97,0 % | 10,58 |
| ZH2-004 | S187P/S188A/P189K/M190A/A 191M/R192Q/Y193L | 97,7 % | 10,43 |
| ZH2-005 | A262E/R263H/R265Q/L266D/L2 67I/M268I/E269R | 97,7 % | 10,68 |
| ZH2-006 | D3D(GTRSEAADAATQARQL)/M37N/E55P/A56V/V101I/S124A/F194FP/T146P/T147A/C148Y/S187P/S188A/P189K/M190A/A191M/R192Q/Y193L/A262E/R263H/R265Q/L266D/L267I/M268I /E269R | 86,1 % | 10,71 |

Beispiel 6: Abbau von ZEN und ZEN-Derivaten in kontaminiertem Mais

Zur Bestimmung der Fähigkeit der Polypeptide, natürlich vorkommendes ZEN und ZEN-Derivate in einer komplexen Matrix und bei niedrigem pH-Wert abzubauen, wurde kontaminierter Mais mit unterschiedlichen Konzentrationen von jeweils einem der Polypeptide mit der Sequenz ID-Nr. 1 bis 6 versetzt und der Abbau von ZEN und ZEN -Derivaten verfolgt.

Der kontaminierte Mais wurde gemahlen und im Abbauversuch eingesetzt, wobei ein Ansatz aus 1 g gemahlenem, kontaminiertem Mais, 8,9 ml 100 mM Acetatpuffer pH = 4,0 und 0,1 ml Polypeptidlösung bestand. Es wurden angereicherte und gereinigte Polypeptidlösungen, wie in Beispiel 5 beschrieben, hergestellt, wobei diese auf eine Konzentration von 10 mU/ml, 100 mU/ml bzw. 1.000 mU/ml verdünnt wurden. Im Ansatz wurden somit absolut 1 mU (= 1 mU/g Mais), 10 mU (= 10 mU/g Mais) bzw. 100 mU (= 100 mU/g Mais) eingesetzt. Jeder Abbauansatz wurde in 25 ml Glasfläschchen durchgeführt und bei 37 °C unter schütteln mit 100 Upm inkubiert. Vor der Enzymzugabe bzw. nach 1 stündiger Inkubation wurde jeweils eine Probe von 1 ml entnommen, das Polypeptid für 10 min bei 99 °C hitzeinaktiviert und die Probe bei -20 °C gelagert. Nach dem Auftauen der Probe wurden die nicht löslichen Bestandteile durch Zentrifugieren abgetrennt. Konzentrationen an ZEN sowie an ZEN-Derivaten wurden mittels LC/MS/MS, wie in M. Sulyok et al. (2007, Anal. Bioanal. Chem., 289, 1505-1523) beschrieben, gemessen. Der Gehalt an ZEN und ZEN-Derivaten in diesem Mais betrug für ZEN 238 ppb, für α-ZEL 15 ppb, für β-ZEL 23 ppb, für Z14G 32 ppb und für Z14S 81 ppb. In Tabelle 6 ist die prozentuale Abnahme des Gehalts an ZEN und an ZEN-Derivaten im Abbauversuch dargestellt.

Tabelle 6: Reduktion an ZEN und ZEN-Derivaten in Prozent bezogen auf den Startgehalt des Abbauversuches von unterschiedlichen Polypeptiden und Polypeptidmengen.

| Polypeptid | Menge im Ansatz | ZEN | α-ZEL | β-ZEL | Z14G | Z14S |
|---|---|---|---|---|---|---|
| Sequenz ID-Nr. 1 | 0,1 mU | 83 % | ≥ 80 % | 70 % | 78 % | 80 % |
| | 1 mU | 96 % | ≥ 80 % | 76% | ≥ 80 % | 92 % |
| | 10 mU | 97 % | ≥ 80 % | ≥ 85 % | ≥ 80 % | 94 % |
| Sequenz ID-Nr. 2 | 0,1 mU | 87 % | ≥ 80 % | 73 % | ≥ 80 % | 84 % |
| | 1 mU | 97 % | ≥ 80 % | 78 % | ≥ 80 % | 90 % |
| | 10 mU | 99 % | ≥ 80 % | ≥ 85 % | ≥ 80 % | 96 % |
| Sequenz ID-Nr. 3 | 0,1 mU | 79 % | 79 % | 67 % | 73 % | 75 % |
| | 1 mU | 85 % | ≥ 80 % | 72 % | 79 % | 82 % |
| | 10 mU | 92 % | ≥ 80 % | 78 % | ≥ 80 % | 88 % |
| Sequenz ID-Nr. 4 | 0,1 mU | 82 % | 78 % | 65 % | 76 % | 80 % |
| | 1 mU | 89 % | ≥ 80 % | 73 % | ≥ 80 % | 86 % |
| | 10 mU | 93 % | ≥ 80 % | 82 % | ≥ 80 % | 91 % |
| Sequenz ID-Nr. 5 | 0,1 mU | 79 % | 76 % | 66 % | 78 % | 80 % |
| | 1 mU | 83 % | ≥ 80 % | 73 % | ≥ 80 % | 81 % |
| | 10 mU | 91 % | ≥ 80 % | 79 % | ≥ 80 % | 86 % |
| Sequenz ID-Nr. 6 | 0,1 mU | 93% | ≥ 80 % | 75 % | ≥ 80 % | 90 % |
| | 1 mU | 95 % | ≥ 80 % | 82 % | ≥ 80 % | 92 % |
| | 10 mU | 98 % | ≥ 80 % | ≥ 85 % | ≥ 80 % | 96 % |

Beispiel 7: Polypeptid enthaltende Zusatzstoffe zur hydrolytischen Spaltung von ZEN und/oder ZEN-Derivaten

Zur Herstellung von Zusatzstoffen zur hydrolytischen Spaltung von ZEN wurden Fermentationsüberstände von mittels *P. pastoris* exprimierten Polypeptiden mit den Sequenzen Sequenz ID-Nr. 1, Sequenz ID-Nr. 2, Sequenz ID-Nr. 6 und Sequenz ID-Nr. 13 mittels Mikrofiltration und Ultrafiltration (Ausschlussgrenze: 10 kDa) unter Standardbedingungen gereinigt und bis zu einer Trockensubstanzkonzentration von etwa 9 Gew.-% konzentriert. Im Anschluss wurden diese polypeptidhältigen Lösungen mit einem Sprühtrockner, (Mini B290 von Büchi) ebenfalls unter Standardbedingungen zu trockenen Pulvern weiterverarbeitet. Diese vier Pulver werden in weiterer Folge als Zusatzstoffe Z1, Z2, Z6 und Z13 bezeichnet. Z1, Z2, Z6 bzw. Z13 wurden des Weiteren mit Bentonit mit einer durchschnittlichen Korngröße ca. 1 μm, in einem Verhältnis von 1 Gew.-% Zusatzstoff Z1, Z2, Z6 bzw. Z13 und 99 Gew.-% Bentonit in einem Überkopfschüttler,

gemischt. Die so enthaltenen Zusatzstoffe werden als Zusatzstoff Z1.B, Z2.B, Z6.B und Z13.B bezeichnet. Des Weiteren wurden Z1, Z2, Z6 und Z13 mit Bentonit und einem Vitamin-Spurenelemente Konzentrat in einem Verhältnis von 0,1 Gew.-% Zusatzstoff Z1, Z2, Z6 bzw. Z13, 0,9 Gew.-% Vitamin-Spurenelemente Konzentrat und 99 Gew.-% Bentonit in einem Überkopfschüttler, gemischt. Die so enthaltenen Zusatzstoffe werden als Zusatzstoff Z1.BVS, Z2.BVS, Z6.BVS und Z13.BVS bezeichnet. 100 g der Zusatzstoffe Z1.BVS, Z2.BVS, Z6.BVS und Z13.BVS enthielten 200 mg Eisensulfat, 50 mg Kupfersulfat, 130 mg Zinnoxid, 130 mg Manganoxid, 2,55 mg Kalzimcarbonat, 160 mg Vitamin E, 6,5 mg Vitamin K3, 6,5 mg Vitamin B1, 14 mg Vitamin B2, 15 mg Vitamin B6, 0,15 mg Vitamin B12, 150 mg Nicotinsäure, 30 mg Pantothensäure und 5,3 mg Folsäure.

Die Zusatzstoffe wurden in einem 50 mMTris-HCl Puffer pH = 8,2 für 30 Minuten extrahiert und im selben Puffer so weiterverdünnt, dass die Endkonzentration an Polypeptid bei ca. 70 ng/ml lag.

Im Anschluss wurde die Zearalenon abbauende Wirkung dieser Lösungen, wie in Beispiel 5 beschrieben, bestimmt. Die entsprechenden Aktivitäten waren für Z1 8.230 U/g, für Z2 9.310 U/g, für Z6 9.214 U/g, für Z1.B 83 U/g, für Z2.B 92 U/g, für Z2.C 90 U/g, für Z13.B 57 U/g, für Z1.BVS 8 U/g, für Z2.BVS 9 U/g, für Z6.BVS 9 U/g und für Z13.BVS 6 U/g.

Die Fähigkeit zum Abbau der ZEN-Derivate α-ZEL, β-ZEL, α-ZAL, β-ZAL, Z14G, Z14S und ZAN durch die Zusatzstoffe Z1, Z2, Z6, Z13, Z1.B, Z2.B, Z6.B, Z13.B, Z1.BVS, Z2.BVS, Z6.BVS und Z13.BVS, wurde, wie in Beispiel 4 beschrieben, durchgeführt, jedoch wurden anstatt 100 μl eines Zelllysats 100 μl einer Polypeptidlösung mit einer Polypeptidkonzentration von ca. 70 ng/ml eingesetzt. Nach 6 stündiger Inkubation lagen nur noch maximal 15 % der Ausgangsmenge als nicht hydrolysiertes ZEN-Derivat vor.

**Patentansprüche**

1. Polypeptid zur hydrolytischen Spaltung von Zearalenon und/oder von Zearalenon Derivaten, **dadurch gekennzeichnet, dass** das Polypeptid eine Aminosäuresequenz aufweist, welche einen Grad an Sequenzidentität zu der Aminosäuresequenz mit einer Sequenz ID-Nr. 1 von wenigstens 70 %, bevorzugt wenigstens 84 %, insbesondere

bevorzugt wenigstens 92 % und am bevorzugtesten wenigstens 98 % aufweist, oder ein funktionelles Fragment davon enthält.

2. Polypeptid nach Anspruch 1, **dadurch gekennzeichnet, dass** die Variante eine Aminosäuremodifikation gewählt aus der Gruppe Substitution, Deletion und Insertion von einer oder mehreren Aminosäuren aufweist.

3. Polypeptid nach einem der Ansprüche 1 oder 2, **dadurch gekennzeichnet, dass** das Enzym eine spezifische Aktivität von wenigstens 0,01 U/mg, bevorzugt wenigstens 0,1 U/mg, insbesondere wenigstens 1 U/mg aufweist, und/oder einen $K_M$-Wert der hydrolytischen Spaltung von ZEN von höchstens 50 $\mu$M, bevorzugt höchstens 3,5 $\mu$M, insbesondere höchstens 0,5 $\mu$M aufweist, und/oder einen kcat-Wert der hydrolytischen Spaltung von ZEN von wenigstens 0,05 $s^{-1}$, bevorzugt wenigstens 0,6 $s^{-1}$, insbesondere wenigstens 5 $s^{-1}$ besitzt, und/oder einen $v_{max}$-Wert der hydrolytischen Spaltung von ZEN von wenigstens 0,00001 $\mu M^{-1}$ $s^{-1}$, bevorzugt wenigstens 0,0001 $\mu M^{-1}$ $s^{-1}$, insbesondere wenigstens 0,001 $\mu M^{-1}$ $s^{-1}$ besitzt.

4. Polypeptid nach einem der Ansprüche 1 bis 3, **dadurch gekennzeichnet, dass** es wenigstens eine Mutation der Aminosäuresequenz in Bezug auf Sequenz ID-Nr. 1 an wenigstens einer der folgenden Positionen: 22, 23, 25, 26, 27, 29, 31, 32, 35, 37, 42, 43, 46, 51, 53, 54, 57, 60, 69, 72, 73, 78, 80, 84, 88, 95, 97, 99, 114, 118, 119, 123, 132, 141, 146, 148, 149, 154, 163, 164, 165, 169, 170, 172, 176, 180, 182, 183, 190, 191, 194, 196, 197, 198, 201, 204, 205, 206, 207, 208, 209, 210, 212, 213, 214, 216, 217, 220, 221, 222, 229, 231, 233, 238, 240, 244, 245, 246, 248, 249, 251, 254, 256, 260, 262, 263, 266, 269, 271, 277, 280, 281, 282, 283, 284, 285, 286, 287, 292, 296, 298, 302, 307, 308, 309, 311, 314, 317, 319, 321, 323, 325 und 326 aufweist.

5. Polypeptid nach Anspruch 4, **dadurch gekennzeichnet, dass** das Polypeptid wenigstens eine Mutation gewählt aus der Gruppe: D22A, S23Q, S23L, N25D, I26V, F27Y, F27H, S29P, R31A, F32Y, R35K, R35Q, V37A, V42I, V43T, F46Y, S51E, S51D, D53G, N54M, N54R, L57V, L60I, S69G, P72E, V73A, A78S, N80H, F84Y, I88L, T95S, T97A, R99K, I114M, I118V, K119R, V123I, L132V, A141S, I146V, I146L, A148G, A149V, A154P, P163T, A164T, Y165C, Y165H, V169I, L170R, A172G, A176M, A176V, Y180F, D182T, F183Y, I190V, G191S, K194T, K194E, F196Y, V197C, V197R, E198R, E198S, K201D, K201G, P204S, P204A, A205S, K206P, A207M, M208A, Q209R, L210A, L210S, ΔP212, T213V, P214A, E216T, E216G, A217I, N220H, L221M, K222R, K222Q, G229A, A231V, F233W, F233Y, F233H, A238G, H240N, H240S, D244E, R245Q, M246L, S248T, S248N, S248G, Q249R, K251N, I254V, I256L, A260M, T262D, T262G, I263T, E266D, E269H, E269N, L271V, L277E, E280A, E280L, H281R, H281Q, A282V, Q283R, D284L, D284R, I285L, I286M, R287E, R287D, R292K, R292T, Q296A, Q296E, H298V, L302S, L307Q, F308S, D309A, A311P, A314V, L317F, S319Q, S319P, S319R, S321A, S321T, T323A, P325A, A326P in der Aminosäuresequenz in Bezug auf Sequenz ID-Nr. 1 aufweist.

6. Polypeptid nach einem der Ansprüche 1 bis 5, **dadurch gekennzeichnet, dass** wenigstens eines der folgenden Aminosäuremotive gewählt aus der Gruppe der Sequenz ID-Nrn. 30 bis 67 enthalten ist.

7. Polypeptid nach einem der Ansprüche 1 bis 6, **dadurch gekennzeichnet, dass** wenigstens eine Aminosäuresequenz der Sequenz ID-Nr. 1 oder wenigstens ein funktionelles Fragment davon enthalten ist.

8. Polypeptid nach Anspruch 7, **dadurch gekennzeichnet, dass** das Polypeptid wenigstens eine konservative Aminosäuresubstitution an wenigstens einer Position enthält, und die konservative Aminosäuresubstitution ist aus Substitutionen von G zu A; oder A zu G, S; oder V zu I, L, A, T, S; oder I zu V, L, M; oder L zu I, M, V; oder M zu L, I, V; oder P zu A, S, N; oder F zu Y, W, H; oder Y zu F, W, H; oder W zu Y, F, H; oder R zu K, E, D; oder K zu R, E, D; oder H zu Q, N, S; oder D zu N, E, K, R, Q; oder E zu Q, D, K, R, N; oder S zu T, A; oder T zu S, V, A; oder C zu S, T, A; oder N zu D, Q, H, S; oder Q zu E, N, H, K, R gewählt.

9. Isoliertes Polynukleotid, **dadurch gekennzeichnet, dass** die Nukleotidsequenz für ein Polypeptid, das eine Zearalenon und/oder Zearalenon Derivate hydrolysierende Eigenschaft besitzt, codiert und/oder einen Grad an Sequenzidentität zu wenigstens der aus den Nukleotidsequenzen der Sequenz ID-Nrn. 15 oder 16 gewählten Gruppe von mindestens 70 % aufweist.

10. Zusatzstoff **dadurch gekennzeichnet, dass** wenigstens ein zur hydrolytischen Spaltung von Zearalenon und/oder von Zearalenon Derivaten befähigtes Polypeptid nach einem der Ansprüche 1 bis 8 enthalten ist.

11. Zusatzstoff nach Anspruch 10, **dadurch gekennzeichnet, dass** zusätzlich wenigstens ein inerter Träger sowie gegebenenfalls weitere Bestandteile, wie Vitamine und/oder Mineralstoffe und/oder Enzyme und/oder weitere Komponenten zur Detoxifizierung von Mykotoxinen enthalten sind.

**12.** Zusatzstoff nach einem der Ansprüche 10 oder 11, **dadurch gekennzeichnet, dass** das Polypeptid nach einem der Ansprüche 1 bis 8 im Zusatzstoff in einer Konzentration von höchstens 10.000 U/g, bevorzugt höchstens 1.000 U/g, bevorzugter höchstens 100 U/g und am bevorzugtesten höchstens 10 U/g im enthalten ist.

**13.** Zusatzstoff nach einem der Ansprüche 10, 11 oder 13, **dadurch gekennzeichnet, dass** das Polypeptid nach einem der Ansprüche 1 bis 8 in verkapselter oder gecoateter Form vorliegt.

**14.** Verfahren zur hydrolytischen Spaltung von Zearalenon und/oder von Zearalenon-Derivaten, **dadurch gekennzeichnet, dass** Zearalenon und/oder wenigstens ein Zearalenon-Derivat durch ein Polypeptid nach einem der Ansprüche 1 bis 8 und/oder ein Zusatzstoff nach einem der Ansprüche 10 bis 13 hydrolysiert wird.

**15.** Verfahren nach Anspruch 14, **dadurch gekennzeichnet, dass** das Polypeptid oder der Zusatzstoff mit einem Futter- oder Nahrungsmittel, dass mit Zearalenon und/oder mit wenigstens einem Zearalenon-Derivat kontaminiert ist, versetzt wird, dass ein Tier oder ein Mensch das kontaminierte Futter- oder Nahrungsmittel aufnimmt und dass das Polypeptid oder der Zusatzstoff das im kontaminierten Futter- oder Nahrungsmittel enthaltende Zearalenon und/oder wenigstens ein Zearalenon Derivat im Verdauungstrakt hydrolysiert, wobei bei feuchten Futter- oder Nahrungsmittels die Hydrolyse des Zearalenons und/oder des wenigstens einen Zearalenon-Derivats bereits vor der oralen Aufnahme im feuchten Futter- oder Nahrungsmittel stattfinden kann.

**16.** Verfahren nach einem der Ansprüche 14 bis 15, **dadurch gekennzeichnet, dass** dadurch wenigstens 70 %, bevorzugt wenigstens 80 %, insbesondere wenigstens 90 % des Zearalenons und/oder wenigstens eines Zearalenon-Derivats hydrolysiert wird.

## Claims

**1.** A polypeptide for the hydrolytic cleavage of zearalenone and/or zearalenone derivatives, **characterized in that** the polypeptide comprises an amino acid sequence having a degree of sequence identity with the amino acid sequence SEQ ID No: 1 of at least 70%, preferably at least 84%, particularly preferably at least 92%, and most preferably at least 98%, or contains a functional fragment thereof.

**2.** A polypeptide according to claim 1, **characterized in that** the variant comprises an amino acid modification selected from the group consisting of substitution, deletion and insertion of one or several amino acids.

**3.** A polypeptide according to any one of claim 1 or 2, **characterized in that** the enzyme exhibits a specific activity of at least 0.01 U/mg, preferably at least 0.1 U/mg, in particular at least 1 U/mg, and/or has a $K_M$ value of the hydrolytic cleavage of ZEN of at most 50 $\mu$M, preferably at most 3.5 $\mu$M, in particular at most 0.5 $\mu$M, and/or has a kcat value of the hydrolytic cleavage of ZEN of at least 0.05 $s^{-1}$, preferably at least 0.6 $s^{-1}$, in particular at least 5 $s^{-1}$, and/or has a $v_{max}$ value of the hydrolytic cleavage of ZEN of at least 0.00001 $\mu M^{-1} s^{-1}$, preferably at least 0.0001 $\mu M^{-1} s^{-1}$, in particular at least 0.001 $\mu M^{-1} s^{-1}$.

**4.** A polypeptide according to any one of claims 1 to 3, **characterized in that** it comprises at least one mutation of the amino acid sequence in respect to SEQ ID No: 1 in at least one of the following positions: 22, 23, 25, 26, 27, 29, 31, 32, 35, 37, 42, 43, 46, 51, 53, 54, 57, 60, 69, 72, 73, 78, 80, 84, 88, 95, 97, 99, 114, 118, 119, 123, 132, 141, 146, 148, 149, 154, 163, 164, 165, 169, 170, 172, 176, 180, 182, 183, 190, 191, 194, 196, 197, 198, 201, 204, 205, 206, 207, 208, 209, 210, 212, 213, 214, 216, 217, 220, 221, 222, 229, 231, 233, 238, 240, 244, 245, 246, 248, 249, 251, 254, 256, 260, 262, 263, 266, 269, 271, 277, 280, 281, 282, 283, 284, 285, 286, 287, 292, 296, 298, 302, 307, 308, 309, 311, 314, 317, 319, 321, 323, 325 und 326.

**5.** A polypeptide according to claim 4, **characterized in that** the polypeptide comprises at least one mutation selected from the group consisting of D22A, S23Q, S23L, N25D, I26V, F27Y, F27H, S29P, R31A, F32Y, R35K, R35Q, V37A, V42I, V43T, F46Y, S51E, S51D, D53G, N54M, N54R, L57V, L60I, S69G, P72E, V73A, A78S, N80H, F84Y, I88L, T95S, T97A, R99K, I114M, I118V, K119R, V123I, L132V, A141S, I146V, I146L, A148G, A149V, A154P, P163T, A164T, Y165C, Y165H, V169I, L170R, A172G, A176M, A176V, Y180F, D182T, F183Y, I190V, G191S, K194T, K194E, F196Y, V197C, V197R, E198R, E198S, K201D, K201G, P204S, P204A, A205S, K206P, A207M, M208A, Q209R, L210A, L210S, ΔP212, T213V, P214A, E216T, E216G, A217I, N220H, L221M, K222R, K222Q, G229A,

A231V, F233W, F233Y, F233H, A238G, H240N, H240S, D244E, R245Q, M246L, S248T, S248N, S248G, Q249R, K251N, I254V, I256L, A260M, T262D, T262G, I263T, E266D, E269H, E269N, L271V, L277E, E280A, E280L, H281R, H281Q, A282V, Q283R, D284L, D284R, I285L, I286M, R287E, R287D, R292K, R292T, Q296A, Q296E, H298V, L302S, L307Q, F308S, D309A, A311P, A314V, L317F, S319Q, S319P, S319R, S321A, S321T, T323A, P325A, A326P.

**6.** A polypeptide according to any one of claims 1 to 5, **characterized in that** at least one of the following amino acid motifs selected from the group consisting of SEQ ID Nos: 30 to 67 is contained.

**7.** A polypeptide according to any one of claims 1 to 6, **characterized in that** at least one amino acid sequence of SEQ ID No: 1 or at least one functional fragment thereof is contained.

**8.** A polypeptide according to claim 7, **characterized in that** the polypeptide comprises at least one conservative amino acid substitution in at least one position, and the conservative amino acid substitution is selected from substitutions of G for A; or A for G, S; or V for I, L, A, T, S; or I for V, L, M; or L for I, M, V; or M for L, I, V; or P for A, S, N; or F for Y, W, H; or Y for F, W, H; or W for Y, F, H; or R for K, E, D; or K for R, E, D; or H for Q, N, S; or D for N, E, K, R, Q; or E for Q, D, K, R, N; or S for T, A; or T for S, V, A; or C for S, T, A; or N for D, Q, H, S; or Q for E, N, H, K, R.

**9.** An isolated polynucleotide, **characterized in that** the nucleotide sequence encodes for a polypeptide having a zearalenone and/or zearalenone derivative-hydrolyzing property, and/or has a degree of sequence identity of at least 70% with at least a group selected from the nucleotide sequences of SEQ ID No: 15 or 16.

**10.** An additive, **characterized in that** at least one polypeptide capable of the hydrolytic cleavage of zearalenone and/or zearalenone derivatives according to any one of claims 1 to 8 is contained.

**11.** An additive according to claim 10, **characterized in that** at least one inert carrier and optionally further components such as vitamins and/or minerals and/or enzymes and/or further components for detoxifying mycotoxins are additionally contained.

**12.** An additive according to any one of claim 10 or 11, **characterized in that** the polypeptide according to any one of claims 1 to 8 is contained in the additive at a concentration of at most 10.000 U/g, preferably at most 1.000 U/g, more preferably at most 100 U/g and most preferably at most 10 U/g.

**13.** An additive according to any one of claim 10, 11 or 12, **characterized in that** the polypeptide according to any one of claims 1 to 8 is present in encapsulated or coated form.

**14.** A method for the hydrolytic cleavage of zearalenone and/or zearalenone derivatives, **characterized in that** zearalenone and/or at least one zearalenone derivative is hydrolyzed by a polypeptide according to any one of claims 1 to 8 and/or an additive according to any one of claims 10 to 13.

**15.** A method according to claim 14, **characterized in that** the polypeptide, or the additive, is mixed with a feed or food contaminated with zearalenone and/or at least one zearalenone derivative, that an animal or a human ingests the contaminated feed or food, and that the polypeptide, or the additive, hydrolyzes in the digestive tract the zearalenone and/or at least one zearalenone derivative contained in the contaminated feed or food, wherein, when the feed or food is wet, the hydrolysis of the zearalenone and/or the at least one zearalenone derivative may already take place in the wet feed or food before the oral ingestion.

**16.** A method according to any one of claims 14 to 15, **characterized in that** thereby at least 70%, preferably at least 80%, in particular at least 90%, of the zearalenone and/or the at least one zearalenone derivative is hydrolyzed.

## Revendications

**1.** Polypeptide pour le clivage hydrolytique de la zéaralénone et/ou de dérivés de zéaralénone, **caractérisé en ce que** le polypeptide présente une séquence d'acide aminé qui présente un degré d'identité de séquence avec la séquence d'acide aminé ayant une séquence ID n° 1 d'au moins 70 %, de préférence d'au moins 84 %, de manière particulièrement préférée, d'au moins 92 % et de manière préférée entre toutes, d'au moins 98 %, ou contient un fragment fonctionnel de celle-ci.

2. Polypeptide selon la revendication 1, **caractérisé en ce que** la variante présente une modification d'acide aminé choisie dans le groupe comprenant substitution, délétion et insertion d'un ou plusieurs acides aminés.

3. Polypeptide selon l'une des revendications 1 ou 2, **caractérisé en ce que** l'enzyme présente une activité spécifique d'au moins 0,01 U/mg, de préférence d'au moins 0,1 U/mg, en particulier d'au moins 1 U/mg et/ou une valeur $K_M$ du clivage hydrolytique de ZEN au plus de 50 $\mu$M, de préférence au plus de 3,5 $\mu$M, en particulier au plus de 0,5 $\mu$M, et/ou possède une valeur $k_{cat}$ du clivage hydrolytique de ZEN d'au moins 0,05 $s^{-1}$, de préférence d'au moins 0,6 $s^{-1}$, en particulier d'au moins 5 $s^{-1}$, et/ou une valeur $V_{max}$ du clivage hydrolytique de ZEN d'au moins 0,00001 $\mu M^{-1} s^{-1}$, de préférence d'au moins 0,00001 $\mu M^{-1} s^{-1}$, en particulier d'au moins 0,001 $\mu M^{-1} s^{-1}$.

4. Polypeptide selon l'une des revendications 1 à 3, **caractérisé en ce qu'**il présente au moins une mutation de la séquence d'acide aminé par rapport à séquence ID n° 1 sur au moins l'une des positions suivantes : 22, 23, 25, 26, 27, 29, 31, 32, 35, 37, 42, 43, 46, 51, 53, 54, 57, 60, 69, 72, 73, 78, 80, 84, 88, 95, 97, 99, 114, 118, 119, 123, 132, 141, 146, 148, 149, 154, 163, 164, 165, 169, 170, 172, 176, 180, 182, 183, 190, 191, 194, 196, 197, 198, 201, 204, 205, 206, 207, 208, 209, 210, 212, 213, 214, 216, 217, 220, 221, 222, 229, 231, 233, 238, 240, 244, 245, 246, 248, 249, 251, 254, 256, 260, 262, 263, 266, 269, 271, 277, 280, 281, 282, 283, 284, 285, 286, 287, 292, 296, 298, 302, 307, 308, 309, 311, 314, 317, 319, 321, 323, 325 et 326.

5. Polypeptide selon la revendication 4, **caractérisé en ce que** le polypeptide présente au moins une mutation choisie dans le groupe : D22A, S23Q, S23L, N25D, I26V, F27Y, F27H, S29P, R31A, F32Y, R35K, R35Q, V37A, V42I, V43T, F46Y, S51E, S51D, D53G, N54M, N54R, L57V, L60I, S69G, P72E, V73A, A78S, N80H, F84Y, I88L, T95S, T97A, R99K, I114M, I118V, K119R, V123I, L132V, A141S, I146V, L146L, A148G, A149V, A154P, P163T, A164T, Y165C, Y165H, V169I, L170R, A172G, A176M, A176V, Y180F, D182T, F183Y, I190V, G191S, K194T, K194E, F196Y, V197C, V197R, E198R, E198S, K201D, K201G, P204S, P204A, A205S, K206P, A207M, M208A, Q209R, L210A, L210S, ΔP212, T213V, P214A, E216T, E216G, A217I, N220H, L221M, K222R, K222Q, G229A, A231V, F233W, F233Y, F233H, A238G, H240N, H240S, D244E, R245Q, M246L, S248T, S248N, S248G, Q249R, K251N, I254V, I256L, A260M, T262D, T262G, I263T, E266D, E269H, E269N, L271V, L277E, E280A, E280L, H281R, H281Q, A282V, Q283R, D284L, D284R, I285L, I286M, R287E, R287D, R292K, R292T, Q296A, Q296E, H298V, L302S, L307Q, F308S, D309A, A311P, A314V, L317F, S319Q, S319P, S319R, S321A, S321T, T323A, P325A, A326P dans la séquence d'acide aminé par rapport à séquence ID n° 1.

6. Polypeptide selon l'une des revendications 1 à 5, **caractérisé en ce qu'**est contenu au moins l'un des motifs d'acides aminés suivants choisi dans le groupe de SEQ ID N° 30 à 67.

7. Polypeptide selon l'une des revendications 1 à 6, **caractérisé en ce qu'**est contenue au moins une séquence d'acide aminé de séquence ID n° 1 ou au moins un fragment fonctionnel de celui-ci.

8. Polypeptide selon la revendication 7, **caractérisé en ce que** le polypeptide contient au moins une substitution conservative d'acide aminé sur au moins une position, et est la substitution conservative d'acide aminé parmi les substitutions de G pour A ; ou de A pour G, S ; ou de V pour I, L, A, T, S ; ou de I pour V, L, M ; ou de L pour I, M, V ; ou de M pour L, I, V ; ou de P pour A, S, N ; ou de F pour Y, W, H ou de Y pour F, W, H ; ou de W pour Y, F, H ; ou de R pour K, E, D ; ou de K pour R, E, D ; ou de H pour Q, N, S ; ou de D pour N, E, K, R, Q ; ou de E pour Q, D, K, R, N ; ou de S pour T, A ; ou de T pour S, V, A ; ou de C pour S, T, A ; ou de N pour D, Q, H, S ; ou de Q pour E, N, H, K, R.

9. Polynucléotide isolé, **caractérisé en ce que** la séquence nucléotidique code pour un polypeptide qui possède une propriété d'hydrolyse d'une zéaralénone et/ou d'un dérivé de zéaralénone, et/ou présente un degré d'identité de séquence avec au moins le groupe choisi parmi les séquences nucléotidiques de séquence ID n° 15 ou 16 d'au moins 70 %.

10. Additif **caractérisé en ce qu'**est contenu au moins un polypeptide capable d'entraîner un clivage hydrolytique de la zéaralénone et/ou de dérivés de zéaralénone selon l'une des revendications 1 à 8.

11. Additif selon la revendication 10, **caractérisé en ce qu'**en outre sont contenus au moins un véhicule inerte ainsi qu'éventuellement d'autres composants tels que vitamines et/ou sels minéraux et/ou enzymes et/ou d'autres composants pour la détoxification de mycotoxines.

12. Additif selon l'une des revendications 10 ou 11, **caractérisé en ce que** le polypeptide selon l'une des revendications

1 à 8 est contenu dans l'additif en une concentration au plus de 10 000 U/g, de préférence au plus de 1000 U/g, de manière davantage préférée au plus de 100 U/g et de manière préférée entre toutes, au plus de 10 U/g

**13.** Additif selon l'une des revendications 10, 11 ou 13, **caractérisé en ce que** le polypeptide selon l'une des revendications 1 à 8 se présente sous une forme encapsulée ou revêtue.

**14.** Procédé de clivage hydrolytique de zéaralénone et/ou de dérivés de zéaralénone, **caractérisé en ce que** la zéaralénone et/ou au moins un dérivé de zéaralénone est hydrolysé par un polypeptide selon l'une des revendications 1 à 8 et/ou un additif selon l'une des revendications 10 à 13.

**15.** Procédé selon la revendication 14, **caractérisé en ce que** le polypeptide ou l'additif est mélangé avec un aliment pour animaux ou un produit alimentaire qui est contaminé avec de la zéaralénone et/ou avec au moins un dérivé de zéaralénone, **en ce qu'**un animal ou un être humain absorbe l'aliment pour animaux ou le produit alimentaire contaminé et **en ce que** le polypeptide ou l'additif hydrolyse dans les voies digestives, la zéaralénone et/ou au moins un dérivé de zéaralénone contenu(e) dans l'aliment pour animaux ou le produit alimentaire contaminé, dans lequel dans l'aliment pour animaux ou le produit alimentaire humide, l'hydrolyse de la zéaralénone et/ou d'au moins un dérivé de zéaralénone peut déjà avoir lieu avant l'absorption orale dans l'aliment pour animaux ou le produit alimentaire.

**16.** Procédé selon l'une des revendications 14 à 15, **caractérisé en ce qu'**il provoque une hydrolyse d'au moins 70 %, de préférence d'au moins 80 %, en particulier d'au moins 90 % de la zéaralénone et/ou d'au moins un dérivé de zéaralénone.

Fig. 1

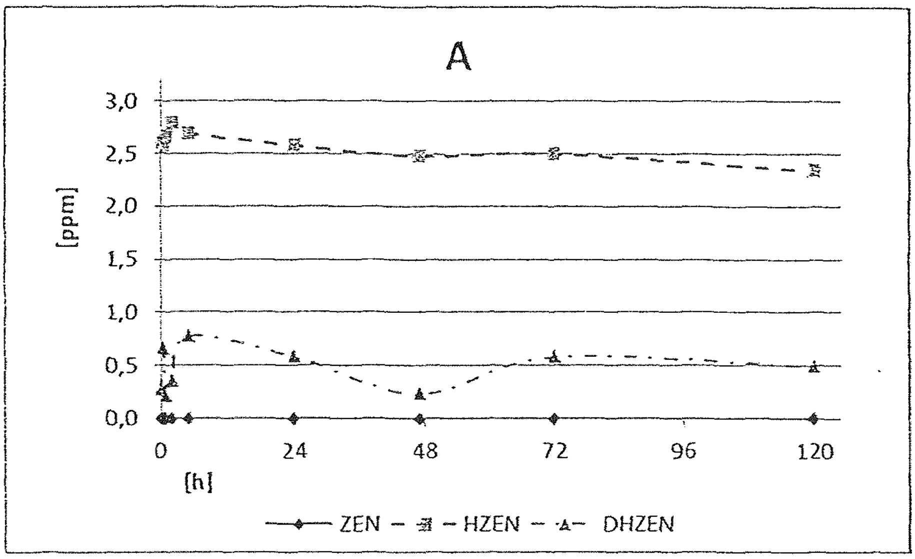


Fig. 1A

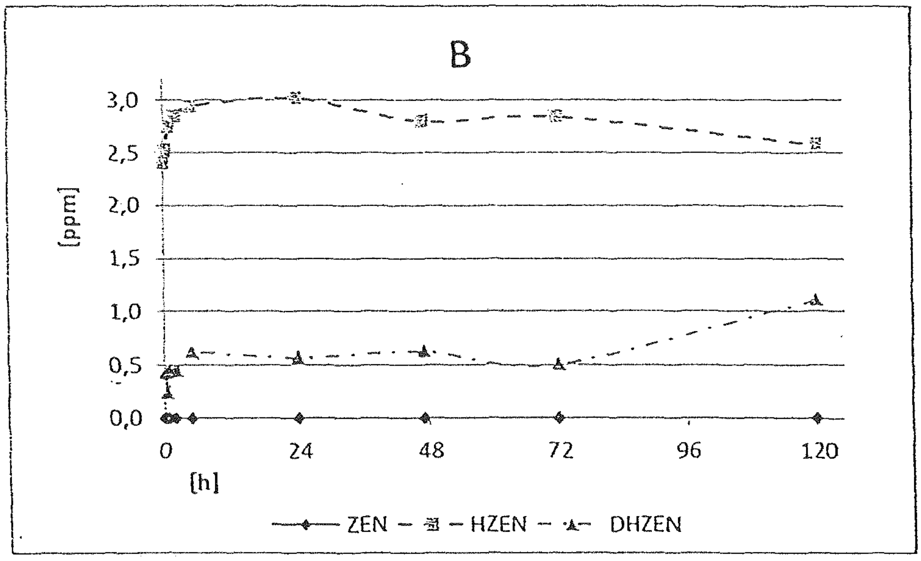


Fig. 1B

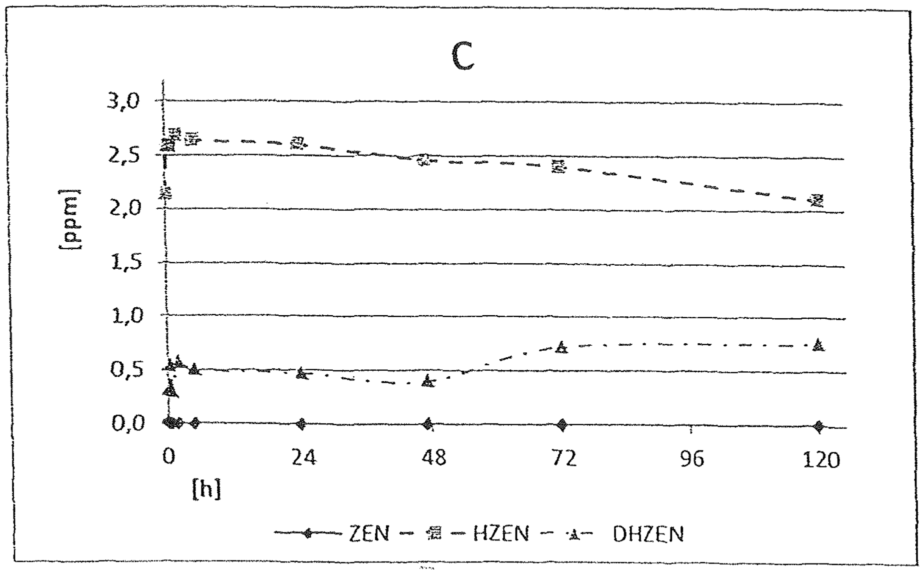


Fig. 1C

Fig. 2

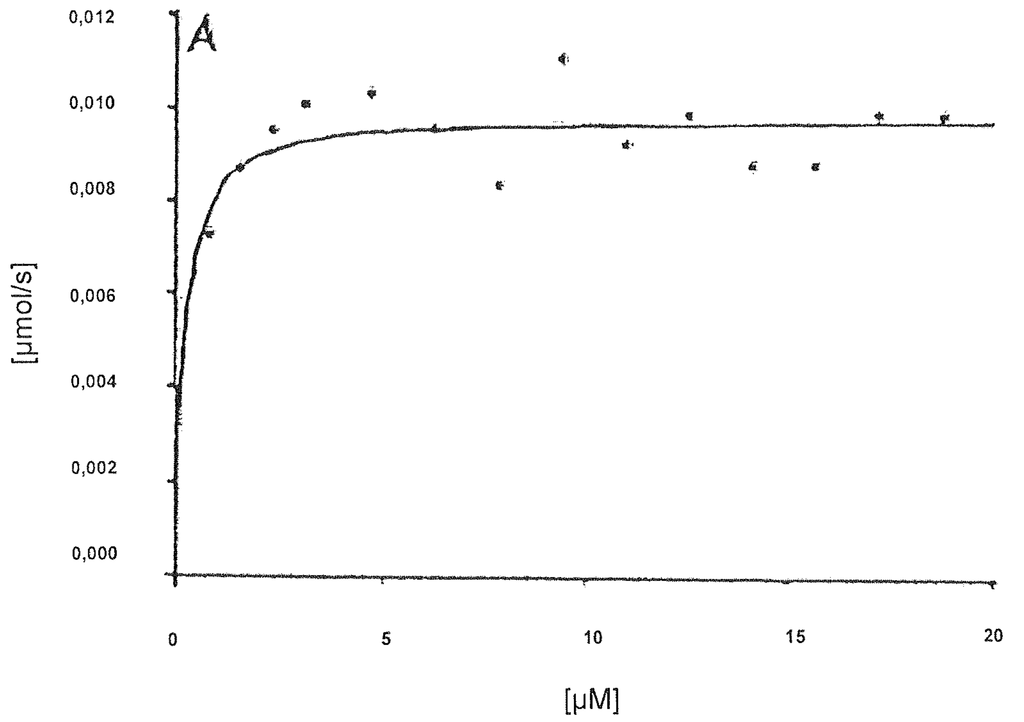


Fig. 2A

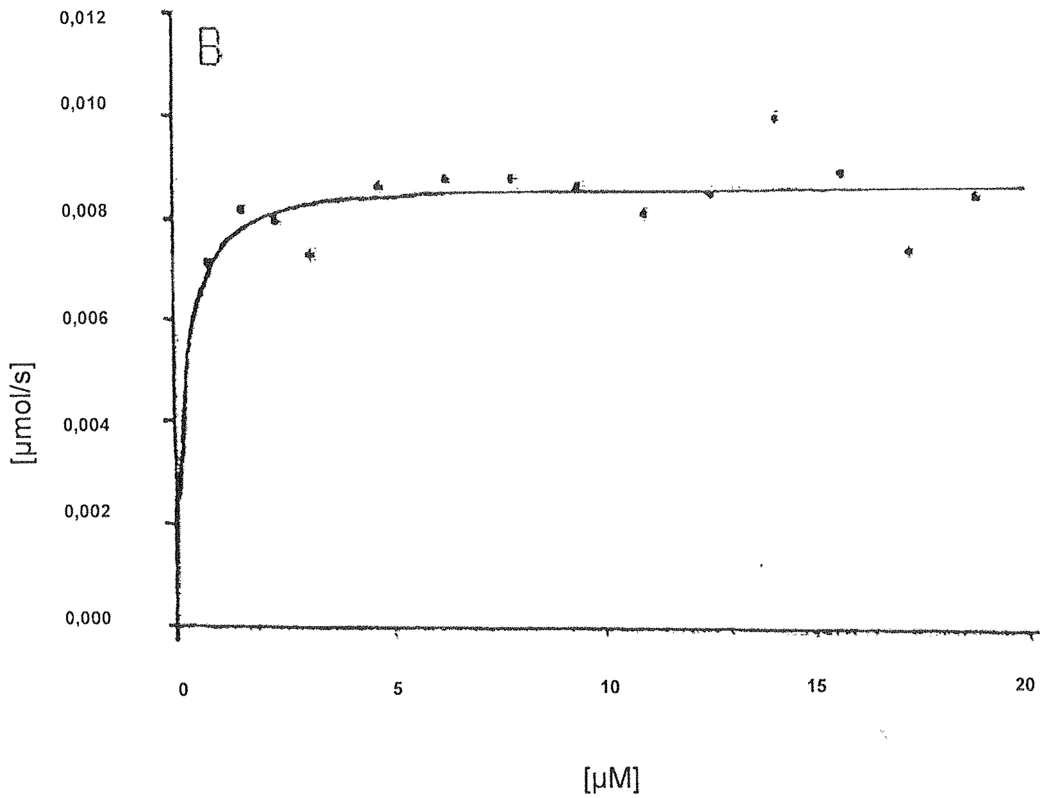


Fig. 2B

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0938575 B1
- WO 02076205 A
- WO 2012113827 A
- WO 9212645 A


### In der Beschreibung aufgeführte Nicht-Patentliteratur


- **I. RODRIGUES ; K. NAEHRER.** *Toxins,* 2012, vol. 4, 663-675
- **E. VEKIRU et al.** *Appl. and Environ. Microb.,* 2010, vol. 76 (7), 2353-2359
- **ALTSCHUL et al.** *Nucleic Acids Res.,* 1997, vol. 25, 3389-3402
- **J. SAMBROOK ; E.F. FRITSCH ; T. MANIATIS.** Molecular Cloning. A Laboratory Manual, 1989
- **BOLTON ; MCCARTHY.** *Proceedings of the National Academy of Sciences USA,* 1962, vol. 48, 1390
- **J. SAMBROOK ; E.F. FRITSCH ; T. MANIATIS.** Molecular Cloning, A Laboratory Manua. Cold Spring Harbor, 1989
- **J.M. CREGG.** Pichia Protocols. 2007
- **J. SAMBROOK et al.** Molecular Cloning, A Laboratory Manual. Cold Spring Harbor, 2012
- Protein Identification and Analysis Tools on the ExPASy Server. **GASTEIGER E. et al.** The Proteomics Protocols Handbook. Humana Press, 2005, 571-607
- **J.S. PAPADOPOULOS ; R. AGARWALA.** COBALT: constraint-based alignment tool for multiple protein sequences. *Bioinformatics,* 2007, vol. 23, 1073-79
- **P. KRENN et al.** *Mykotoxin Research,* 2007, vol. 23 (4), 180-184
- **M. SULYOK et al.** *Anal. Bioanal. Chem.,* vol. 289, 1505-1523
- **M. SULYOK et al.** *Food Chemistry,* 2010, vol. 119, 408-416
- **P. SONGSERMASKUL et al.** *J. of Animal Physiol. and Animal Nutr.,* 2011, vol. 97, 155-161
- **H. BISSWANG.** *Enzyme Kinetics,* 2002, ISBN 3-527-30343-X, 19
- **M. SULYOK et al.** *Anal. Bioanal. Chem.,* 2007, vol. 289, 1505-1523